# EUROPEAN PATENT APPLICATION

(11) **EP 3 730 516 A1**
(43) Date of publication of application: **28.10.2020**
(21) Application number: 20179064.9
(22) Date of filing: 19.05.2014
(51) Int. Cl.: C07K 16/18, C07K 16/28, A61K 38/43, A61K 39/00, A61P 25/28, C07K 16/26, C12N 9/14, A61P 43/00

(54) **METHODS AND COMPOSITIONS FOR INCREASING ENZYME ACTIVITY IN THE CNS**

(30) Priority: 22.07.2013 US 201361857140 P
(62) Divisional of application: 14733790.1
(71) Applicant: Armagen, Inc., Agoura Hills, CA 91301 (US)
(72) Inventor: PARDRIDGE, William M., Pacific Palisades, CA (US); BOADO, Ruben J., Agoura Hills, CA 91301 (US)
(74) Representative: Mewburn Ellis LLP

(57) **Abstract**

Provided herein are methods and compositions for treating a subject suffering from an enzyme deficiency in the central nervous system (CNS). The bifunctional fusion antibodies provided herein comprise an antibody to an endogenous blood brain barrier (BBB) receptor and an enzyme deficient in mucopolysaccharidosis III (MPS-III). The fusion antibodies provided herein comprise N-sulfoglucosamine sulfohydrolase (SGSH), alpha-N-acetylgulcosaminidase (NAGLU), heparin-alpha-glucosaminide N-acetyltransferase (HGSNAT), or N-acetylglucosamine-6-sulfatase (GNS). The methods of treating an enzyme deficiency in the CNS comprise systemic administration of a fusion antibody provided herein.

## Description

### CROSS REFERENCE TO RELATED APPLICATIONS

This application claims the benefit of U.S. Provisional Patent Application serial No. 61/857,140 filed July 22, 2013, the contents of which are incorporated herein by reference in their entirety

### SEQUENCE LISTING

This application contains a Sequence Listing which has been submitted in ASCII format via EFS-Web and is hereby incorporated by reference in its entirety. Said ASCII copy, created on May 14, 2014, is named 28570_713_601_SL.txt and is 84 kilobytes in size.

### BACKGROUND OF THE INVENTION

Mucopolysaccharidosis (MPS) III, also called MPS-III or Sanfilippo syndrome, is an inherited metabolic disease that mainly affects the central nervous system (CNS). MPS III is caused by defects in enzymes needed to break down long chains of sugar molecules called glycosaminoglycans. There are four main types of MPS-III. Type A (MPS-IIIA) is caused by a defect in the lysosomal enzyme N-sulfoglucosamine sulfohydrolase (SGSH), also called sulfamidase or N-heparan sulfatase, which functions to degrade heparan sulfate glycosaminoglycans (GAGs). SGSH causes the hydrolysis of N-linked sulfate groups from the non-reducing terminal glucosaminide residues of heparan sulfate. Type B (MPS-IIIB) is caused by a defect in alpha-N-acetylgulcosaminidase (NAGLU). Type C (MPS-IIIC) is caused by a defect in heparin-alpha-glucosaminide N-acetyltransferase (HGSNAT). Type D (MPS-IIID) is caused by a defect in N-acetylglucosamine-6-sulfatase (GNS). An insufficient level of these enzymes causes a pathological buildup of glycosaminoglycans in, e.g., peripheral tissues, and the CNS. However, the clinical features of MPS-III are almost exclusively neurological. Symptoms begin in early life including behavioral disturbances progressing to dementia and developmental regression, followed by death in the second or third decade. Typically, treatment of a lysosomal storage disorder such as MPS-III would include intravenous enzyme replacement therapy with recombinant enzymes that are deficient. However, systemically administered recombinant enzymes do not cross the blood brain barrier (BBB), and therefore would have little impact on the effects of the disease in the CNS.

### SUMMARY OF THE INVENTION

Described herein are methods and compositions for treating a subject suffering from a deficiency of sulfamidase, i.e, N-sulfoglucosamine sulfohydrolase ("SGSH"). In certain embodiments, the methods provided herein comprise delivery of SGSH to the CNS by systemically administering a therapeutically effective amount of a bifunctional fusion antibody or protein. In certain embodiments, the bifunctional fusion antibody comprises the amino acid sequences of an antibody to an endogenous blood brain barrier (BBB) receptor and SGSH. In some embodiments, the bifunctional fusion antibody is a human insulin antibody (HIR Ab) genetically fused to SGSH ("HIR Ab-SGSH fusion antibody"). In certain embodiments, the HIR Ab-SGSH fusion antibody binds to the extracellular domain of the insulin receptor and is transported across the blood brain barrier ("BBB") into the CNS, while retaining SGSH enzyme activity. In certain embodiments, the HIR Ab binds to the endogenous insulin receptor on the BBB, and acts as a molecular Trojan horse to ferry the SGSH into the brain. In certain embodiments, therapeutically effective systemic dose of a HIR Ab-SGSH fusion antibody for systemic administration is based, in part, on the specific CNS uptake characteristics of the fusion antibody from peripheral blood as described herein.

In one aspect provided herein is a method for treating an SGSH deficiency in the central nervous system of a subject in need thereof, comprising systemically administering to the subject a therapeutically effective dose of a fusion antibody having SGSH activity. In some embodiments, the fusion antibody comprises the amino acid sequence of an immunoglobulin heavy chain, the amino acid sequence of an SGSH, and the amino acid sequence of an immunoglobulin light chain. In some embodiments, the fusion antibody binds to an extracellular domain of an endogenous BBB receptor (e.g., the human insulin receptor) and catalyzes hydrolysis of the N-linked sulfate group from the non-reducing terminal glucosaminide residues of heparan sulfate. In some embodiments, the amino acid sequence of the SGSH is covalently linked to the carboxy terminus of the amino acid sequence of the immunoglobulin heavy chain.

In some embodiments, the fusion antibody is post-translationally modified by a sulfatase modifying factor type 1 (SUMF1). In some embodiments, the post-translational modification comprises a cysteine to formylglycine conversion. In some embodiments, the fusion antibody comprises formylglycine.

In some embodiments, the SGSH retains at least 20% of its activity compared to its activity as a separate entity. In some embodiments, the SGSH and the immunoglobulin each retains at least 20% of its activity compared to its activity as a separate entity.

In some embodiments, at least about 10 ug of SGSH enzyme are delivered to the brain. In some embodiments at least about 20 ug of SGSH enzyme are delivered to the brain. In some embodiments at least about 30 ug of SGSH enzyme are delivered to the brain. In some embodiments at least about 40 ug of SGSH enzyme are delivered to the brain. In some embodiments at least about 50 ug of SGSH enzyme are delivered to the brain. In some embodiments at least about 100 ug of SGSH enzyme are delivered to the brain. In some embodiments at least about 200 ug of SGSH enzyme are delivered to the brain. In some embodiments at least about 300 ug of SGSH enzyme are delivered to the brain. In some embodiments at least about 400 ug of SGSH enzyme are delivered to the brain. In some embodiments at least about 500 ug of SGSH enzyme are delivered to the brain. In some embodiments at least about 1000 ug of SGSH enzyme are delivered to the brain. In some embodiments at least about 5 ug of SGSH enzyme are delivered to the brain. In some embodiments at least about 1 ug of SGSH enzyme are delivered to the brain. In some embodiments at least about 0.5 ug of SGSH enzyme are delivered to the brain. In some embodiments at least about 0.1 ug of SGSH enzyme are delivered to the brain.

In some embodiments, at least about 200 ug of SGSH enzyme are delivered to the brain, normalized per 50 kg body weight. In some embodiments, at least about 250 ug of SGSH enzyme are delivered to the brain, normalized per 50 kg body weight. In some embodiments, at least about 300 ug of SGSH enzyme are delivered to the brain, normalized per 50 kg body weight. In some embodiments, at least about 400 ug of SGSH enzyme are delivered to the brain, normalized per 50 kg body weight. In some embodiments, at least about 500 ug of SGSH enzyme are delivered to the brain, normalized per 50 kg body weight. In some embodiments, at least about 1000 ug of SGSH enzyme are delivered to the brain, normalized per 50 kg body weight. In some embodiments, at least about 2000 ug of SGSH enzyme are delivered to the brain, normalized per 50 kg body weight. In some embodiments, at least about 150 ug of SGSH enzyme are delivered to the brain, normalized per 50 kg body weight. In some embodiments, at least about 100 ug of SGSH enzyme are delivered to the brain, normalized per 50 kg body weight. In some embodiments, at least about 50 ug of SGSH enzyme are delivered to the brain, normalized per 50 kg body weight. In some embodiments, at least about 10 ug of SGSH enzyme are delivered to the brain, normalized per 50 kg body weight.

In some embodiments, the therapeutically effective dose of the fusion antibody comprises at least about 0.5 mg/Kg of body weight. In some embodiments, the therapeutically effective dose of the fusion antibody comprises at least about 0.6 mg/Kg of body weight. In some embodiments, the therapeutically effective dose of the fusion antibody comprises at least about 0.7 mg/Kg of body weight. In some embodiments, the therapeutically effective dose of the fusion antibody comprises at least about 0.8 mg/Kg of body weight. In some embodiments, the therapeutically effective dose of the fusion antibody comprises at least about 0.9 mg/Kg of body weight. In some embodiments, the therapeutically effective dose of the fusion antibody comprises at least about 1 mg/Kg of body weight. In some embodiments, the therapeutically effective dose of the fusion antibody comprises at least about 2 mg/Kg of body weight. In some embodiments, the therapeutically effective dose of the fusion antibody comprises at least about 5 mg/Kg of body weight. In some embodiments, the therapeutically effective dose of the fusion antibody comprises at least about 0.4 mg/Kg of body weight. In some embodiments, the therapeutically effective dose of the fusion antibody comprises at least about 0.3 mg/Kg of body weight. In some embodiments, the therapeutically effective dose of the fusion antibody comprises at least about 0.2 mg/Kg of body weight. In some embodiments, the therapeutically effective dose of the fusion antibody comprises at least about 0.1 mg/Kg of body weight.

In some embodiments, the therapeutically effective dose of the fusion antibody comprises at least about 1000 units/Kg of body weight. In some embodiments, the therapeutically effective dose of the fusion antibody comprises at least about 1500 units/Kg of body weight. In some embodiments, the therapeutically effective dose of the fusion antibody comprises at least about 2000 units/Kg of body weight. In some embodiments, the therapeutically effective dose of the fusion antibody comprises at least about 3000 units/Kg of body weight. In some embodiments, the therapeutically effective dose of the fusion antibody comprises at least about 4000 units/Kg of body weight. In some embodiments, the therapeutically effective dose of the fusion antibody comprises at least about 5000 units/Kg of body weight. In some embodiments, the therapeutically effective dose of the fusion antibody comprises at least about 10,000 units/Kg of body weight. In some embodiments, the therapeutically effective dose of the fusion antibody comprises at least about 15,000 units/Kg of body weight. In some embodiments, the therapeutically effective dose of the fusion antibody comprises at least about 20,000 units/Kg of body weight. In some embodiments, the therapeutically effective dose of the fusion antibody comprises at least about 25,000 units/Kg of body weight. In some embodiments, the therapeutically effective dose of the fusion antibody comprises at least about 900 units/Kg of body weight. In some embodiments, the therapeutically effective dose of the fusion antibody comprises at least about 800 units/Kg of body weight. In some embodiments, the therapeutically effective dose of the fusion antibody comprises at least about 700 units/Kg of body weight. In some embodiments, the therapeutically effective dose of the fusion antibody comprises at least about 600 units/Kg of body weight. In some embodiments, the therapeutically effective dose of the fusion antibody comprises at least about 500 units/Kg of body weight. In some embodiments, the therapeutically effective dose of the fusion antibody comprises at least about 400 units/Kg of body weight. In some embodiments, the therapeutically effective dose of the fusion antibody comprises at least about 300 units/Kg of body weight. In some embodiments, the therapeutically effective dose of the fusion antibody comprises at least about 200 units/Kg of body weight. In some embodiments, the therapeutically effective dose of the fusion antibody comprises at least about 100 units/Kg of body weight.

In some embodiments, the SGSH specific activity of the fusion antibody is at least 1000 units/mg protein. In some embodiments, the SGSH specific activity of the fusion antibody is at least 1500 units/mg. In some embodiments, the SGSH specific activity of the fusion antibody is at least 2000 units/mg. In some embodiments, the SGSH specific activity of the fusion antibody is at least 3000 units/mg. In some embodiments, the SGSH specific activity of the fusion antibody is at least 4000 units/mg. In some embodiments, the SGSH specific activity of the fusion antibody is at least 5000 units/mg. In some embodiments, the SGSH specific activity of the fusion antibody is at least 10,000 units/mg. In some embodiments, the SGSH specific activity of the fusion antibody is at least 12,000 units/mg. In some embodiments, the SGSH specific activity of the fusion antibody is at least 15,000 units/mg.

In some embodiments, systemic administration is parenteral, intravenous, subcutaneous, intra-muscular, trans-nasal, intra-arterial, transdermal, or respiratory.

In some embodiments, the fusion antibody is a chimeric antibody. In some embodiments, the fusion antibody is a humanized antibody.

In some embodiments, the immunoglobulin heavy chain is an immunoglobulin heavy chain of IgG. In some embodiments, the immunoglobulin heavy chain is an immunoglobulin heavy chain of IgG1 class.

In some embodiments, the immunoglobulin heavy chain of the fusion antibody comprises a CDR1 corresponding to the amino acid sequence of SEQ ID NO:1 with up to 4 single amino acid mutations, a CDR2 corresponding to the amino acid sequence of SEQ ID NO:2 with up to 6 single amino acid mutations, or a CDR3 corresponding to the amino acid sequence of SEQ ID NO:3 with up to 3 single amino acid mutations, wherein the single amino acid mutations are substitutions, deletions, or insertions.

In other embodiments, the immunoglobulin heavy chain of the fusion antibody comprises a CDR1 corresponding to the amino acid sequence of SEQ ID NO:1 with up to 3 single amino acid mutations, a CDR2 corresponding to the amino acid sequence of SEQ ID NO:2 with up to 6 single amino acid mutations, and a CDR3 corresponding to the amino acid sequence of SEQ ID NO:3 with up to 3 single amino acid mutations.

In other embodiments, the immunoglobulin heavy chain of the fusion antibody comprises a CDR1 corresponding to the amino acid sequence of SEQ ID NO:1 with up to 3 single amino acid mutations, a CDR2 corresponding to the amino acid sequence of SEQ ID NO:2 with up to 6 single amino acid mutations, and a CDR3 corresponding to the amino acid sequence of SEQ ID NO:3 with a single amino acid mutation.

In other embodiments, the immunoglobulin heavy chain of the fusion antibody comprises a CDR1 corresponding to the amino acid sequence of SEQ ID NO:1 with a single amino acid mutations, a CDR2 corresponding to the amino acid sequence of SEQ ID NO:2 with a single amino acid mutations, and a CDR3 corresponding to the amino acid sequence of SEQ ID NO:3 with a single amino acid mutation.

In other embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, or a CDR3 corresponding to the amino acid sequence of SEQ ID NO:3.

In further embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, and a CDR3 corresponding to the amino acid sequence of SEQ ID NO:3.

In some embodiments, the immunoglobulin light chain is an immunoglobulin light chain of kappa or lambda class.

In some embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 5 single amino acid mutations, or a CDR3 corresponding to the amino acid sequence of SEQ ID NO:6 with up to 5 single amino acid mutations, wherein the single amino acid mutations are substitutions, deletions, or insertions.

In other embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 5 single amino acid mutations, and a CDR3 corresponding to the amino acid sequence of SEQ ID NO:6 with up to 5 single amino acid mutations.

In other embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 3 single amino acid mutations, and a CDR3 corresponding to the amino acid sequence of SEQ ID NO:6 with up to 3 single amino acid mutations.

In other embodiments, the immunoglobulin light chain of the fusion antibody comprises a CDR1 corresponding to the amino acid sequence of SEQ ID NO:4 with a single amino acid mutations, a CDR2 corresponding to the amino acid sequence of SEQ ID NO:5 with a single amino acid mutations, and a CDR3 corresponding to the amino acid sequence of SEQ ID NO:6 with a single amino acid mutations.

In other embodiments, the immunoglobulin light chain of the fusion antibody comprises a CDR1 corresponding to the amino acid sequence of SEQ ID NO:4, a CDR2 corresponding to the amino acid sequence of SEQ ID NO:5, or a CDR3 corresponding to the amino acid sequence of SEQ ID NO:6.

In further embodiments, the immunoglobulin light chain of the fusion antibody comprises a CDR1 corresponding to the amino acid sequence of SEQ ID NO:4, a CDR2 corresponding to the amino acid sequence of SEQ ID NO:5, and a CDR3 corresponding to the amino acid sequence of SEQ ID NO:6.

In some embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, and a CDR3 corresponding to the amino acid sequence of SEQ ID NO:3; and the immunoglobulin light chain comprises a CDR1 corresponding to the amino acid sequence of SEQ ID NO:4, a CDR2 corresponding to the amino acid sequence of SEQ ID NO:5, and a CDR3 corresponding to the amino acid sequence of SEQ ID NO:6.

In some embodiments, the immunoglobulin heavy chain of the fusion antibody is at least 90% identical to SEQ ID NO:7 and the amino acid sequence of the light chain immunoglobulin is at least 90% identical to SEQ ID NO:8.

In some embodiments, the immunoglobulin heavy chain of the fusion antibody is at least 95% identical to SEQ ID NO:7 and the amino acid sequence of the light chain immunoglobulin is at least 95% identical to SEQ ID NO:8.

In some embodiments, the immunoglobulin heavy chain of the fusion antibody comprises SEQ ID NO:7 and the amino acid sequence of the light chain immunoglobulin comprises SEQ ID NO:8

In some embodiments, the SGSH comprises an amino acid sequence at least 90% identical to SEQ ID NO:9. In some embodiments, the SGSH comprises an amino acid sequence at least 95% identical to SEQ ID NO:9. In some embodiments, the SGSH comprises an amino acid sequence of SEQ ID NO:9.

In other embodiments, the amino acid sequence of the immunoglobulin heavy chain of the fusion antibody at least 90% identical to SEQ ID NO:7; the amino acid sequence of the light chain immunoglobulin is at least 90% identical to SEQ ID NO:8; and the amino acid sequence of the SGSH is at least 95% identical to SEQ ID NO:9 or comprises SEQ ID NO:9.

In other embodiments, the amino acid sequence of the immunoglobulin heavy chain of the fusion antibody comprises SEQ ID NO:8, the amino acid sequence of the immunoglobulin light chain comprises SEQ ID NO:8, and the amino acid sequence of the SGSH comprises SEQ ID NO:9

In some embodiments, the fusion antibody provided herein crosses the BBB by binding an endogenous BBB receptor-mediated transport system. In some embodiments, the fusion antibody crosses the BBB via an endogenous BBB receptor selected from the group consisting of the insulin receptor, transferrin receptor, leptin receptor, lipoprotein receptor, and the insulin-like growth factor (IGF) receptor. In some embodiments, the fusion antibody crosses the BBB by binding an insulin receptor.

In some embodiments, the systemic administration is parenteral, intravenous, subcutaneous, intra-muscular, trans-nasal, intra-arterial, transdermal, or respiratory.

In some embodiments, the SGSH deficiency in the central nervous system is mucopolysaccharidosis Type IIIA (MPS-IIIA) or Sanfilippo syndrome type A.

In some aspects, provided herein is a method for treating an N-sulfoglucosamine sulfohydrolase (SGSH) deficiency in the central nervous system of a subject in need thereof, comprising systemically administering to the subject a therapeutically effective dose of a fusion antibody having SGSH activity, wherein the fusion antibody comprises: (a) a fusion protein comprising the amino acid sequences of an immunoglobulin light chain and a SGSH, and (b) an immunoglobulin heavy chain; wherein the fusion antibody crosses the blood brain barrier (BBB). In some embodiments, the amino acid sequence of the SGSH is covalently linked to the carboxy terminus of the amino acid sequence of the immunoglobulin light chain.

In some aspects, provided herein is a method for treating an SGSH deficiency in the central nervous system of a subject in need thereof, comprising systemically administering to the subject a therapeutically effective dose of a fusion antibody having SGSH activity, wherein the fusion antibody comprises: (a) a fusion protein comprising an amino acid sequence that is at least 90% identical to SEQ ID NO:10, and (b) an immunoglobulin light chain. In some embodiments, the fusion antibody binds to an extracellular domain of an endogenous BBB receptor. In some embodiments, the endogenous BBB receptor is the human insulin receptor. In some embodiments, the fusion antibody catalyzes hydrolysis of N-linked sulfate from heparan sulfate. In some embodiments, the fusion protein comprises an amino acid sequence that is at least 95% identical to SEQ ID NO: 10. In some embodiments, the fusion protein comprises the amino acid sequence of SEQ ID NO: 10.

In some aspects, provided herein is a fusion antibody having SGSH activity, the fusion antibody comprising (a) a fusion protein comprising an amino acid sequence that is at least 90% identical to SEQ ID NO:10, and (b) an immunoglobulin light chain. In some embodiments, the fusion antibody binds to an extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate. In some embodiments, the fusion protein comprises an amino acid sequence that is at least 95% identical to SEQ ID NO: 10. In some embodiments, the fusion protein comprises the amino acid sequence of SEQ ID NO: 10.

In some aspects, provided herein is a fusion antibody having SGSH activity, the fusion antibody comprising (a) a fusion protein comprising the amino acid sequence of an immunoglobulin heavy chain and an SGSH, and (b) an immunoglobulin light chain. In some embodiments, the amino acid sequence of the SGSH is covalently linked to the carboxy terminus of the amino acid sequence of the immunoglobulin heavy chain. In some embodiments, provided herein is a fusion antibody having SGSH activity, the fusion antibody comprising (a) a fusion protein comprising the amino acid sequence of an immunoglobulin light chain and an SGSH, and (b) an immunoglobulin heavy chain. In some embodiments, the amino acid sequence of the SGSH is covalently linked to the carboxy terminus of the amino acid sequence of the immunoglobulin light chain. In some embodiments, the fusion antibody binds to the extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate.

In some embodiments, the fusion protein provided herein further comprises a linker between the amino acid sequence of the SGSH and the carboxy terminus of the amino acid sequence of the immunoglobulin heavy chain.

In some embodiments, provided herein is a pharmaceutical composition comprising a therapeutically effective amount of a fusion antibody described herein and a pharmaceutically acceptable excipient.

In some embodiments, provided herein is an isolated polynucleotide encoding the fusion antibody described herein. In some embodiments, the isolated polynucleotide comprises the nucleic acid sequence of SEQ ID NO:14. In some embodiments, provided herein is a vector comprising an isolated polynucleotide provided herein. In some embodiments, provided herein is a vector comprising the nucleic acid sequence of SEQ ID NO:14. In some embodiments, provided herein is a host cell comprising a vector described herein. In some embodiments, the host cell is a Chinese Hamster Ovary (CHO) cell. In some aspects, provided herein is a method for treating an SGSH deficiency in the central nervous system of a subject in need thereof, comprising systemically administering to the subject a therapeutically effective dose of a fusion antibody having SGSH activity, wherein the fusion antibody comprises (a) a fusion protein comprising the amino acid sequence of an immunoglobulin heavy chain and an SGSH, and (b) an immunoglobulin light chain. In some embodiments, the amino acid sequence of the SGSH is covalently linked to the carboxy terminus of the amino acid sequence of the immunoglobulin heavy chain. In some embodiments, provided herein is a method for treating an SGSH deficiency in the central nervous system of a subject in need thereof, comprising systemically administering to the subject a therapeutically effective dose of a fusion antibody having SGSH activity, wherein the fusion antibody comprises (a) a fusion protein comprising the amino acid sequence of an immunoglobulin light chain and an SGSH, and (b) an immunoglobulin heavy chain. In some embodiments, the amino acid sequence of the SGSH is covalently linked to the carboxy terminus of the amino acid sequence of the immunoglobulin light chain. In some embodiments, the fusion antibody binds to the extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate.

In certain embodiments, provided herein are methods and compositions for treating a subject suffering from an enzyme deficiency in the CNS. In certain embodiments, the methods provided herein comprise delivery of an enzyme deficient in mucopolysaccharidosis III (MPS-III) to the CNS by systemically administering a therapeutically effective amount of a bifunctional fusion antibody or protein. In certain embodiments, the bifunctional fusion antibody comprises the amino acid sequences of an antibody to an endogenous blood brain barrier (BBB) receptor and an enzyme deficient in MPS-III. In some embodiments, the bifunctional fusion antibody is a human insulin antibody (HIR Ab) genetically fused to the enzyme. In certain embodiments, the fusion antibody binds to the extracellular domain of the insulin receptor and is transported across the BBB into the CNS, while retaining enzyme activity. In certain embodiments, the fusion antibody binds to the endogenous insulin receptor on the BBB, and acts as a molecular Trojan horse to ferry the enzyme into the brain. In certain embodiments, therapeutically effective systemic dose of a fusion antibody for systemic administration is based, in part, on the specific CNS uptake characteristics of the fusion antibody from peripheral blood as described herein.

In one aspect provided herein is a method for treating an enzyme deficiency in the central nervous system of a subject in need thereof, comprising systemically administering to the subject a therapeutically effective dose of a fusion antibody comprising the amino acid sequence of an immunoglobulin heavy chain, the amino acid sequence of an enzyme deficient in MPS-III, and the amino acid sequence of an immunoglobulin light chain. In some embodiments, the fusion antibody binds to an extracellular domain of an endogenous BBB receptor (e.g., the human insulin receptor). In some embodiments, the amino acid sequence of the enzyme is covalently linked to the carboxy terminus of the amino acid sequence of the immunoglobulin heavy chain.

In certain embodiments, the enzyme deficient in MPS-III is a lysosomal enzyme.

In some embodiments, the enzyme deficient in MPS-III is N-sulfoglucosamine sulfohydrolase (SGSH), alpha-N-acetylgulcosaminidase (NAGLU), heparin-alpha-glucosaminide N-acetyltransferase (HGSNAT), or N-acetylglucosamine-6-sulfatase (GNS).

In some embodiments, the fusion antibody is post-translationally modified by a sulfatase modifying factor type 1 (SUMF1). In some embodiments, the post-translational modification comprises a cysteine to formylglycine conversion. In some embodiments, the fusion antibody comprises formylglycine.

In some embodiments, the fusion antibody catalyzes hydrolysis of N-linked sulfate from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.

In some embodiments, the enzyme retains at least 20% of its activity compared to its activity as a separate entity. In some embodiments, the enzyme and the immunoglobulin each retains at least 20% of its activity compared to its activity as a separate entity. In some embodiments, the enzyme retains at least 5%, 10%, 15%, 20%, 25%, 30%, 40%, 50%, 60%, 70%, 75%, 80%, 90%, or 95% of its activity compared to its activity as a separate entity. In some embodiments, the enzyme and the immunoglobulin each retains at least 5%, 10%, 15%, 20%, 25%, 30%, 40%, 50%, 60%, 70%, 75%, 80%, 90%, or 95% of its activity compared to its activity as a separate entity.

In some embodiments, at least about 10 ug of the enzyme are delivered to the brain. In some embodiments at least about 20 ug of the enzyme are delivered to the brain. In some embodiments at least about 30 ug of the enzyme are delivered to the brain. In some embodiments at least about 40 ug of the enzyme are delivered to the brain. In some embodiments at least about 50 ug of the enzyme are delivered to the brain. In some embodiments at least about 100 ug of the enzyme are delivered to the brain. In some embodiments at least about 200 ug of the enzyme are delivered to the brain. In some embodiments at least about 300 ug of the enzyme are delivered to the brain. In some embodiments at least about 400 ug of the enzyme are delivered to the brain. In some embodiments at least about 500 ug of the enzyme are delivered to the brain. In some embodiments at least about 1000 ug of the enzyme are delivered to the brain. In some embodiments at least about 5 ug of the enzyme are delivered to the brain. In some embodiments at least about 1 ug of the enzyme are delivered to the brain. In some embodiments at least about 0.5 ug of the enzyme are delivered to the brain. In some embodiments at least about 0.1 ug of the enzyme are delivered to the brain.

In some embodiments, at least about 200 ug of the enzyme are delivered to the brain, normalized per 50 kg body weight. In some embodiments, at least about 250 ug of the enzyme are delivered to the brain, normalized per 50 kg body weight. In some embodiments, at least about 300 ug of the enzyme are delivered to the brain, normalized per 50 kg body weight. In some embodiments, at least about 400 ug of the enzyme are delivered to the brain, normalized per 50 kg body weight. In some embodiments, at least about 500 ug of the enzyme are delivered to the brain, normalized per 50 kg body weight. In some embodiments, at least about 1000 ug of the enzyme are delivered to the brain, normalized per 50 kg body weight. In some embodiments, at least about 2000 ug of the enzyme are delivered to the brain, normalized per 50 kg body weight. In some embodiments, at least about 150 ug of the enzyme are delivered to the brain, normalized per 50 kg body weight. In some embodiments, at least about 100 ug of the enzyme are delivered to the brain, normalized per 50 kg body weight. In some embodiments, at least about 50 ug of the enzyme are delivered to the brain, normalized per 50 kg body weight. In some embodiments, at least about 10 ug of the enzyme are delivered to the brain, normalized per 50 kg body weight.

In some embodiments, the therapeutically effective dose of the fusion antibody comprises at least about 0.5 mg/Kg of body weight. In some embodiments, the therapeutically effective dose of the fusion antibody comprises at least about 0.6 mg/Kg of body weight. In some embodiments, the therapeutically effective dose of the fusion antibody comprises at least about 0.7 mg/Kg of body weight. In some embodiments, the therapeutically effective dose of the fusion antibody comprises at least about 0.8 mg/Kg of body weight. In some embodiments, the therapeutically effective dose of the fusion antibody comprises at least about 0.9 mg/Kg of body weight. In some embodiments, the therapeutically effective dose of the fusion antibody comprises at least about 1 mg/Kg of body weight. In some embodiments, the therapeutically effective dose of the fusion antibody comprises at least about 2 mg/Kg of body weight. In some embodiments, the therapeutically effective dose of the fusion antibody comprises at least about 5 mg/Kg of body weight. In some embodiments, the therapeutically effective dose of the fusion antibody comprises at least about 0.4 mg/Kg of body weight. In some embodiments, the therapeutically effective dose of the fusion antibody comprises at least about 0.3 mg/Kg of body weight. In some embodiments, the therapeutically effective dose of the fusion antibody comprises at least about 0.2 mg/Kg of body weight. In some embodiments, the therapeutically effective dose of the fusion antibody comprises at least about 0.1 mg/Kg of body weight.

In some embodiments, the therapeutically effective dose of the fusion antibody comprises at least about 1000 units/Kg of body weight. In some embodiments, the therapeutically effective dose of the fusion antibody comprises at least about 1500 units/Kg of body weight. In some embodiments, the therapeutically effective dose of the fusion antibody comprises at least about 2000 units/Kg of body weight. In some embodiments, the therapeutically effective dose of the fusion antibody comprises at least about 3000 units/Kg of body weight. In some embodiments, the therapeutically effective dose of the fusion antibody comprises at least about 4000 units/Kg of body weight. In some embodiments, the therapeutically effective dose of the fusion antibody comprises at least about 5000 units/Kg of body weight. In some embodiments, the therapeutically effective dose of the fusion antibody comprises at least about 10,000 units/Kg of body weight. In some embodiments, the therapeutically effective dose of the fusion antibody comprises at least about 15,000 units/Kg of body weight. In some embodiments, the therapeutically effective dose of the fusion antibody comprises at least about 20,000 units/Kg of body weight. In some embodiments, the therapeutically effective dose of the fusion antibody comprises at least about 25,000 units/Kg of body weight. In some embodiments, the therapeutically effective dose of the fusion antibody comprises at least about 900 units/Kg of body weight. In some embodiments, the therapeutically effective dose of the fusion antibody comprises at least about 800 units/Kg of body weight. In some embodiments, the therapeutically effective dose of the fusion antibody comprises at least about 700 units/Kg of body weight. In some embodiments, the therapeutically effective dose of the fusion antibody comprises at least about 600 units/Kg of body weight. In some embodiments, the therapeutically effective dose of the fusion antibody comprises at least about 500 units/Kg of body weight. In some embodiments, the therapeutically effective dose of the fusion antibody comprises at least about 400 units/Kg of body weight. In some embodiments, the therapeutically effective dose of the fusion antibody comprises at least about 300 units/Kg of body weight. In some embodiments, the therapeutically effective dose of the fusion antibody comprises at least about 200 units/Kg of body weight. In some embodiments, the therapeutically effective dose of the fusion antibody comprises at least about 100 units/Kg of body weight.

In some embodiments, the enzyme specific activity of the fusion antibody is at least 1000 units/mg protein. In some embodiments, the enzyme specific activity of the fusion antibody is at least 1500 units/mg. In some embodiments, the enzyme specific activity of the fusion antibody is at least 2000 units/mg. In some embodiments, the enzyme specific activity of the fusion antibody is at least 3000 units/mg. In some embodiments, the enzyme specific activity of the fusion antibody is at least 4000 units/mg. In some embodiments, the enzyme specific activity of the fusion antibody is at least 5000 units/mg. In some embodiments, the enzyme specific activity of the fusion antibody is at least 10,000 units/mg. In some embodiments, the enzyme specific activity of the fusion antibody is at least 12,000 units/mg. In some embodiments, the enzyme specific activity of the fusion antibody is at least 15,000 units/mg.

In some embodiments, systemic administration is parenteral, intravenous, subcutaneous, intra-muscular, trans-nasal, intra-arterial, transdermal, or respiratory.

In some embodiments, the fusion antibody is a chimeric antibody. In some embodiments, the fusion antibody is a humanized antibody.

In some embodiments, the immunoglobulin heavy chain is an immunoglobulin heavy chain of IgG. In some embodiments, the immunoglobulin heavy chain is an immunoglobulin heavy chain of IgG1 class.

In some embodiments, the immunoglobulin heavy chain of the fusion antibody comprises a CDR1 corresponding to the amino acid sequence of SEQ ID NO:1 with up to 4 single amino acid mutations, a CDR2 corresponding to the amino acid sequence of SEQ ID NO:2 with up to 6 single amino acid mutations, or a CDR3 corresponding to the amino acid sequence of SEQ ID NO:3 with up to 3 single amino acid mutations, wherein the single amino acid mutations are substitutions, deletions, or insertions.

In other embodiments, the immunoglobulin heavy chain of the fusion antibody comprises a CDR1 corresponding to the amino acid sequence of SEQ ID NO:1 with up to 3 single amino acid mutations, a CDR2 corresponding to the amino acid sequence of SEQ ID NO:2 with up to 6 single amino acid mutations, and a CDR3 corresponding to the amino acid sequence of SEQ ID NO:3 with up to 3 single amino acid mutations.

In other embodiments, the immunoglobulin heavy chain of the fusion antibody comprises a CDR1 corresponding to the amino acid sequence of SEQ ID NO:1 with up to 3 single amino acid mutations, a CDR2 corresponding to the amino acid sequence of SEQ ID NO:2 with up to 6 single amino acid mutations, and a CDR3 corresponding to the amino acid sequence of SEQ ID NO:3 with a single amino acid mutation.

In other embodiments, the immunoglobulin heavy chain of the fusion antibody comprises a CDR1 corresponding to the amino acid sequence of SEQ ID NO:1 with a single amino acid mutations, a CDR2 corresponding to the amino acid sequence of SEQ ID NO:2 with a single amino acid mutations, and a CDR3 corresponding to the amino acid sequence of SEQ ID NO:3 with a single amino acid mutation.

In other embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, or a CDR3 corresponding to the amino acid sequence of SEQ ID NO:3.

In further embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, and a CDR3 corresponding to the amino acid sequence of SEQ ID NO:3.

In some embodiments, the immunoglobulin light chain is an immunoglobulin light chain of kappa or lambda class.

In some embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 5 single amino acid mutations, or a CDR3 corresponding to the amino acid sequence of SEQ ID NO:6 with up to 5 single amino acid mutations, wherein the single amino acid mutations are substitutions, deletions, or insertions.

In other embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 5 single amino acid mutations, and a CDR3 corresponding to the amino acid sequence of SEQ ID NO:6 with up to 5 single amino acid mutations.

In other embodiments, the immunoglobulin light chain of the fusion antibody comprises a CDR1 corresponding to the amino acid sequence of SEQ ID NO:4 with up to 3 single amino acid mutations, a CDR2 corresponding to the amino acid sequence of SEQ ID NO:5 with up to 3 single amino acid mutations, and a CDR3 corresponding to the amino acid sequence of SEQ ID NO:6 with up to 3 single amino acid mutations.

In other embodiments, the immunoglobulin light chain of the fusion antibody comprises a CDR1 corresponding to the amino acid sequence of SEQ ID NO:4 with a single amino acid mutations, a CDR2 corresponding to the amino acid sequence of SEQ ID NO:5 with a single amino acid mutations, and a CDR3 corresponding to the amino acid sequence of SEQ ID NO:6 with a single amino acid mutations.

In other embodiments, the immunoglobulin light chain of the fusion antibody comprises a CDR1 corresponding to the amino acid sequence of SEQ ID NO:4, a CDR2 corresponding to the amino acid sequence of SEQ ID NO:5, or a CDR3 corresponding to the amino acid sequence of SEQ ID NO:6.

In further embodiments, the immunoglobulin light chain of the fusion antibody comprises a CDR1 corresponding to the amino acid sequence of SEQ ID NO:4, a CDR2 corresponding to the amino acid sequence of SEQ ID NO:5, and a CDR3 corresponding to the amino acid sequence of SEQ ID NO:6.

In some embodiments, the immunoglobulin heavy chain of the fusion antibody comprises a CDR1 corresponding to the amino acid sequence of SEQ ID NO:1, a CDR2 corresponding to the amino acid sequence of SEQ ID NO:2, and a CDR3 corresponding to the amino acid sequence of SEQ ID NO:3; and the immunoglobulin light chain comprises a CDR1 corresponding to the amino acid sequence of SEQ ID NO:4, a CDR2 corresponding to the amino acid sequence of SEQ ID NO:5, and a CDR3 corresponding to the amino acid sequence of SEQ ID NO:6.

In some embodiments, the immunoglobulin heavy chain of the fusion antibody is at least 90% identical to SEQ ID NO:7 and the amino acid sequence of the light chain immunoglobulin is at least 90% identical to SEQ ID NO:8.

In some embodiments, the immunoglobulin heavy chain of the fusion antibody is at least 95% identical to SEQ ID NO:7 and the amino acid sequence of the light chain immunoglobulin is at least 95% identical to SEQ ID NO:8.

In some embodiments, the immunoglobulin heavy chain of the fusion antibody comprises SEQ ID NO:7 and the amino acid sequence of the light chain immunoglobulin comprises SEQ ID NO:8

In some embodiments, the enzyme comprises an amino acid sequence at least 90% identical to SEQ ID NO:9, SEQ ID NO:17, SEQ ID NO:19, or SEQ ID NO:21. In some embodiments, the enzyme comprises an amino acid sequence at least 95% identical to SEQ ID NO:9, SEQ ID NO: 17, SEQ ID NO:19, or SEQ ID NO:21. In some embodiments, the enzyme comprises an amino acid sequence of SEQ ID NO:9, SEQ ID NO:17, SEQ ID NO:19, or SEQ ID NO:21.

In some embodiments, the fusion antibody provided herein crosses the BBB by binding an endogenous BBB receptor-mediated transport system. In some embodiments, the fusion antibody crosses the BBB via an endogenous BBB receptor selected from the group consisting of the insulin receptor, transferrin receptor, leptin receptor, lipoprotein receptor, and the insulin-like growth factor (IGF) receptor. In some embodiments, the fusion antibody crosses the BBB by binding an insulin receptor.

In some embodiments, the systemic administration is parenteral, intravenous, subcutaneous, intra-muscular, trans-nasal, intra-arterial, transdermal, or respiratory.

In some embodiments, the enzyme deficiency in the central nervous system is mucopolysaccharidosis IIIA (MPS-IIIA), mucopolysaccharidosis IIIB (MPS-IIIB), mucopolysaccharidosis IIIC (MPS-IIIC), or mucopolysaccharidosis IIID (MPS-IIID).

In some aspects, provided herein is a method for treating an enzyme deficiency in the central nervous system of a subject in need thereof, comprising systemically administering to the subject a therapeutically effective dose of a fusion antibody comprising (a) a fusion protein comprising the amino acid sequences of an immunoglobulin light chain and an enzyme deficient in mucopolysaccharidosis III (MPS-III), and (b) an immunoglobulin heavy chain; wherein the fusion antibody crosses the blood brain barrier (BBB). In some embodiments, the amino acid sequence of the enzyme is covalently linked to the carboxy terminus of the amino acid sequence of the immunoglobulin light chain.

In some aspects, provided herein is a method for treating an enzyme deficiency in the central nervous system of a subject in need thereof, comprising systemically administering to the subject a therapeutically effective dose of a fusion antibody comprising (a) a fusion protein comprising an amino acid sequence that is at least 90% identical to SEQ ID NO:10, SEQ ID NO:18, SEQ ID NO:20, or SEQ ID NO:22; and (b) an immunoglobulin light chain. In some embodiments, the fusion antibody binds to an extracellular domain of an endogenous BBB receptor. In some embodiments, the endogenous BBB receptor is the human insulin receptor. In some embodiments, the fusion antibody catalyzes hydrolysis of N-linked sulfate from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate. In some embodiments, the fusion protein comprises an amino acid sequence that is at least 95% identical to SEQ ID NO: 10, SEQ ID NO:18, SEQ ID NO:20, or SEQ ID NO:22. In some embodiments, the fusion protein comprises the amino acid sequence of SEQ ID NO: 10, SEQ ID NO:18, SEQ ID NO:20, or SEQ ID NO:22.

In some aspects, provided herein is a fusion antibody comprising (a) a fusion protein comprising an amino acid sequence that is at least 90% identical to SEQ ID NO:10, SEQ ID NO:18, SEQ ID NO:20, or SEQ ID NO:22, and (b) an immunoglobulin light chain. In some embodiments, the fusion antibody binds to an extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate. In some embodiments, the fusion protein comprises an amino acid sequence that is at least 95% identical to SEQ ID NO: 10, SEQ ID NO: 18, SEQ ID NO:20, or SEQ ID NO:22. In some embodiments, the fusion protein comprises the amino acid sequence of SEQ ID NO: 10, SEQ ID NO:18, SEQ ID NO:20, or SEQ ID NO:22.

In some aspects, provided herein is a fusion antibody comprising (a) a fusion protein comprising the amino acid sequence of an immunoglobulin heavy chain and an enzyme deficient in mucopolysaccharidosis III (MPS-III), and (b) an immunoglobulin light chain. In some embodiments, the amino acid sequence of the enzyme is covalently linked to the carboxy terminus of the amino acid sequence of the immunoglobulin heavy chain. In some embodiments, provided herein is a fusion antibody comprising (a) a fusion protein comprising the amino acid sequence of an immunoglobulin light chain and an enzyme deficient in mucopolysaccharidosis III (MPS-III), and (b) an immunoglobulin heavy chain. In some embodiments, the amino acid sequence of the enzyme is covalently linked to the carboxy terminus of the amino acid sequence of the immunoglobulin light chain. In some embodiments, the fusion antibody binds to the extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.

In some embodiments, the fusion protein provided herein further comprises a linker between the amino acid sequence of the enzyme and the carboxy terminus of the amino acid sequence of the immunoglobulin heavy chain.

In some embodiments, provided herein is a pharmaceutical composition comprising a therapeutically effective amount of a fusion antibody described herein and a pharmaceutically acceptable excipient.

In some embodiments, provided herein is an isolated polynucleotide encoding the fusion antibody described herein. In some embodiments, the isolated polynucleotide comprises the nucleic acid sequence of SEQ ID NO:14, SEQ ID NO:23, SEQ ID NO:24, or SEQ ID NO:25. In some embodiments, provided herein is a vector comprising an isolated polynucleotide provided herein. In some embodiments, provided herein is a vector comprising the nucleic acid sequence of SEQ ID NO:14, SEQ ID NO:23, SEQ ID NO:24, or SEQ ID NO:25. In some embodiments, provided herein is a host cell comprising a vector described herein. In some embodiments, the host cell is a Chinese Hamster Ovary (CHO) cell.

In some aspects, provided herein is a method for treating an enzyme deficiency in the central nervous system of a subject in need thereof, comprising systemically administering to the subject a therapeutically effective dose of a fusion antibody comprising (a) a fusion protein comprising the amino acid sequence of an immunoglobulin heavy chain and an enzyme deficient in mucopolysaccharidosis III (MPS-III), and (b) an immunoglobulin light chain. In some embodiments, the amino acid sequence of the enzyme is covalently linked to the carboxy terminus of the amino acid sequence of the immunoglobulin heavy chain. In some embodiments, provided herein is a method for treating an enzyme deficiency in the central nervous system of a subject in need thereof, comprising systemically administering to the subject a therapeutically effective dose of a fusion antibody comprising (a) a fusion protein comprising the amino acid sequence of an immunoglobulin light chain and an enzyme deficient in mucopolysaccharidosis III (MPS-III), and (b) an immunoglobulin heavy chain. In some embodiments, the amino acid sequence of the enzyme is covalently linked to the carboxy terminus of the amino acid sequence of the immunoglobulin light chain. In some embodiments, the fusion antibody binds to the extracellular domain of an endogenous BBB receptor. In some embodiments, the endogenous BBB receptor is the human insulin receptor. In some embodiments, the fusion antibody is an antibody that binds to the endogenous BBB receptor. In some embodiments, the fusion antibody is an antibody that binds to the human insulin receptor. In some embodiments, the fusion antibody catalyzes hydrolysis of N-linked sulfate from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the present embodiments are set forth with particularity in the appended claims. A better understanding of the features and advantages of the present embodiments will be obtained by reference to the following detailed description that sets forth illustrative embodiments, in which the principles of the present embodiments are utilized, and the accompanying drawings, as follow:

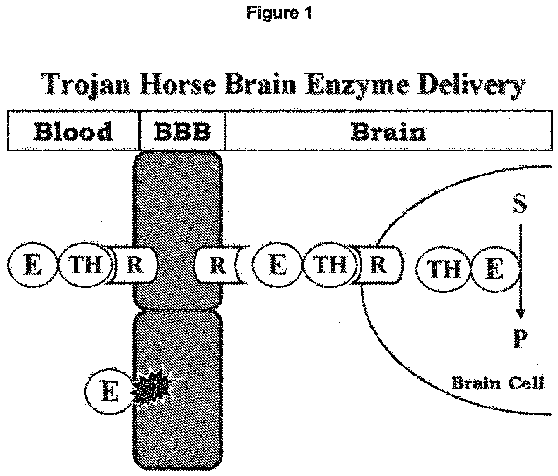

**Figure 1****.** Schematic depiction of a "molecular trojan horse" strategy in which the fusion antibody comprises an antibody to the extracellular domain of an endogenous BBB receptor (R), which acts as a molecular Trojan horse (TH), and SGSH, a lysosomal enzyme (E). Once inside brain cells, behind the BBB, the SGSH part of the fusion antibody then converts heparan sulfate (S) to degradable products (P).

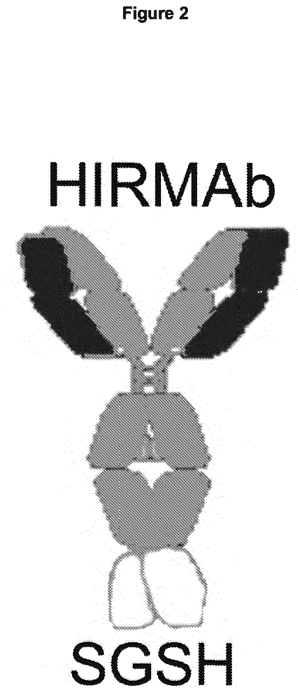

**Figure 2**. An exemplary HIR Ab-SGSH fusion antibody is formed by fusion of the amino terminus of the mature SGSH to the carboxyl terminus of the CH3 region of the heavy chain of the HIR Ab.

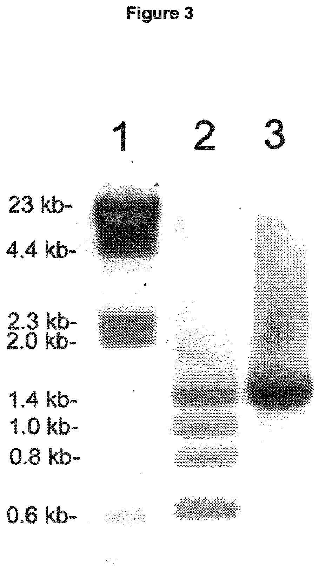

**Figure 3****.** Ethidium bromide stain of agarose gel of the 1.5 kb human SGSH cDNA (lane 3), which was produced by PCR from human liver cDNA, and SGSH-specific primers (Table 2). Lanes 1 and 2: Lambda HindIII digested DNA standard and PhiX174 HaeIII digested DNA standard, respectively.

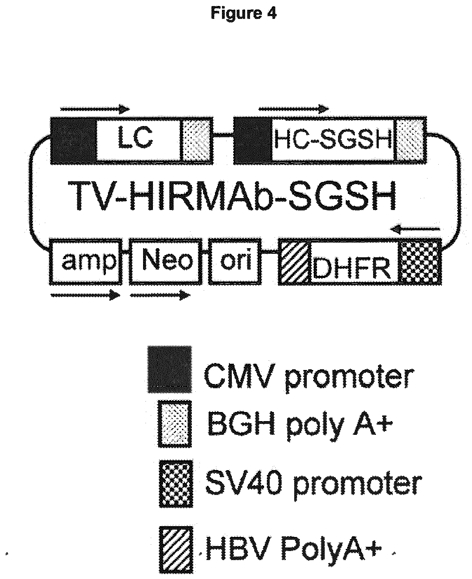

**Figure 4****.** Genetically engineered tandem vector (TV), designated TV-HIRMAb-SGSH, encoding 4 separate and tandem expression cassettes encoding the heavy chain (HC) fusion gene, the light chain (LC) gene, the dihydrofolate reductase (DHFR) gene, and the neomycin resistance (Neo) gene. Other elements include the ampicilin resistance gene (amp) and the origin of replication (ori). The heavy and light chain expression cassettes are 5'-flanked by the cytomegalovirus (CMV) promoter, and 3'-flanked by the bovine growth hormone (BGH) poly-A sequence. The DHFR expression cassette is 5'-flanked by the simian virus (SV)40 promoter and 3'-flanked by the hepatitis B virus (HBV) poly-A sequence.

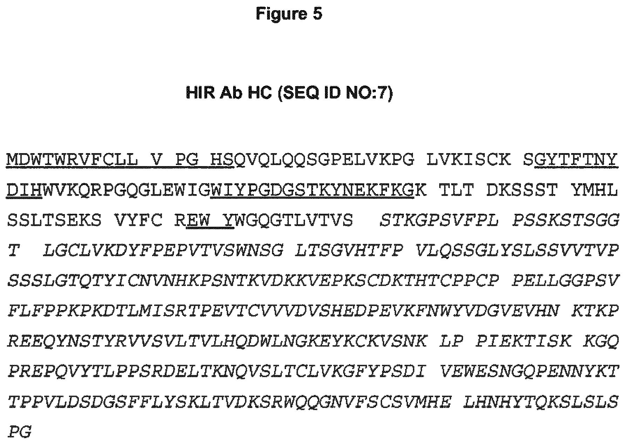

**Figure 5****.** Amino acid sequence of an immunoglobulin heavy chain variable region from an exemplary human insulin receptor antibody directed against the extracellular domain of the human insulin receptor. The underlined sequences are a signal peptide, CDR1, CDR2, and CDR3, respectively. The heavy chain constant region, taken from human IgG1, is shown in italics.

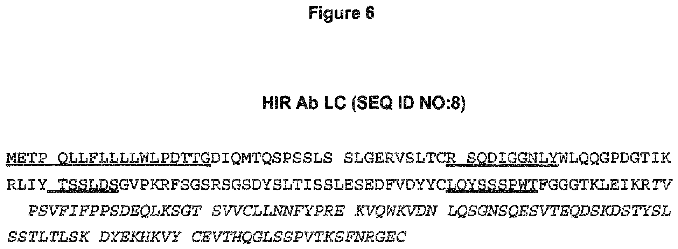

**Figure 6****.** Amino acid sequence of an immunoglobulin light chain variable region from an exemplary human insulin receptor antibody directed against the extracellular domain of the human insulin receptor. The underlined sequences are a signal peptide, CDR1, CDR2, and CDR3, respectively. The constant region, derived from human kappa light chain, is shown in italics.

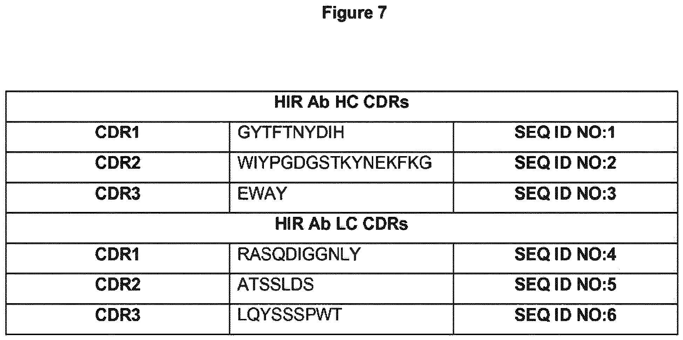

**Figure 7****.** A table showing the CDR1, CDR2, and CDR3 amino acid sequences from a heavy and light chain of an exemplary human insulin receptor antibody directed against the extracellular domain of the human insulin receptor.

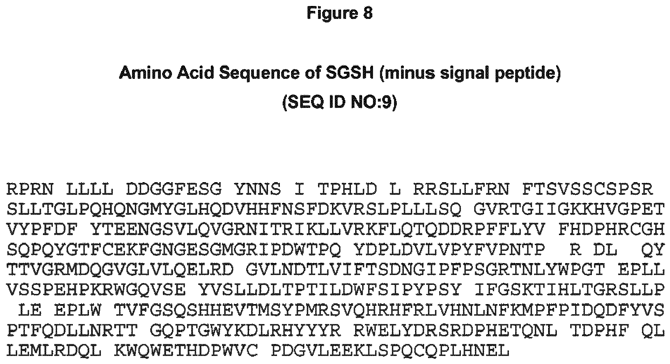

**Figure 8****.** Amino acid sequence of SGSH (NP_000190), not including the initial 20 amino acid signal peptide (mature SGSH).

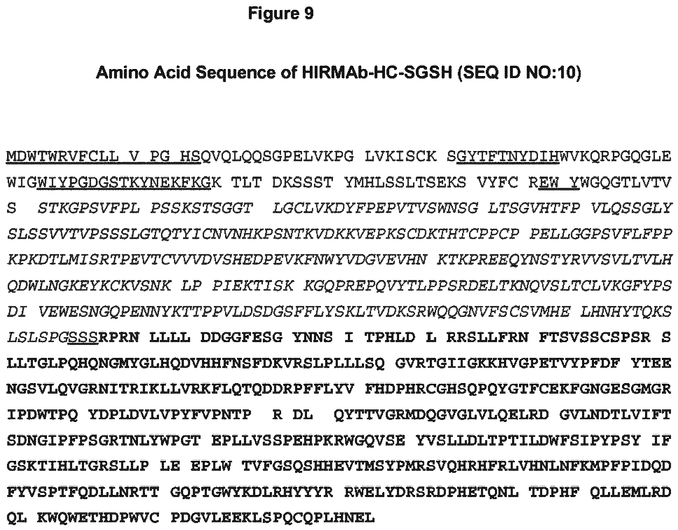

**Figure 9****.** Amino acid sequence of a fusion of an exemplary human insulin receptor antibody heavy chain to mature human SGSH. The underlined sequences are, in order, an IgG signal peptide, CDR1, CDR2, CDR3, and a peptide linker (Ser-Ser-Ser) linking the carboxy terminus of the heavy chain to the amino terminus of the SGSH. Sequence in italic corresponds to the heavy chain constant region, taken from human IgG1. The sequence in bold corresponds to human SGSH.

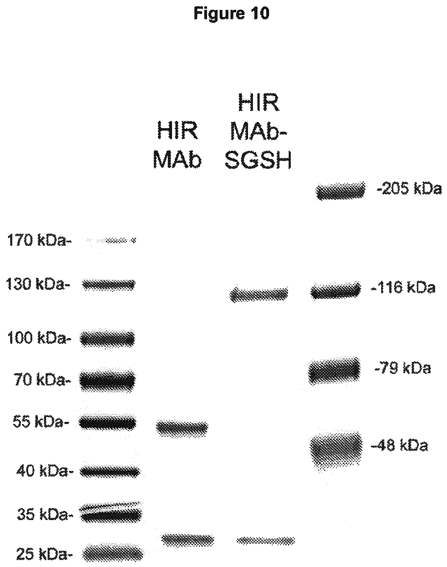

**Figure 10****.** Reducing SDS-PAGE of molecular weight standards (lanes 1 and 4), the purified HIRMAb (lane 2), and the purified HIRMAb-SGSH fusion protein (lane 3).

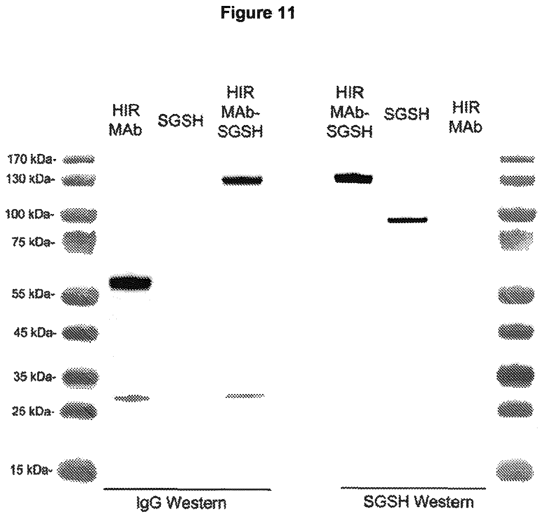

**Figure 11****.** Western blot with either anti-human (h) IgG primary antibody (left panel) or anti-human SGSH primary antiserum (right panel). The immunoreactivity of the HIRMAb-SGSH fusion protein is compared to the chimeric HIRMAb and to recombinant SGSH. Both the HIRMAb-SGSH fusion protein and the HIRMAb have identical light chains on the anti-hIgG Western. The HIRMAb-SGSH fusion heavy chain reacts with both the anti-hIgG and the anti-human SGSH antibody, whereas the HIRMAb heavy chain only reacts with the anti-hIgG antibody. The recombinant SGSH reacts only with the anti-human SGSH antibody.

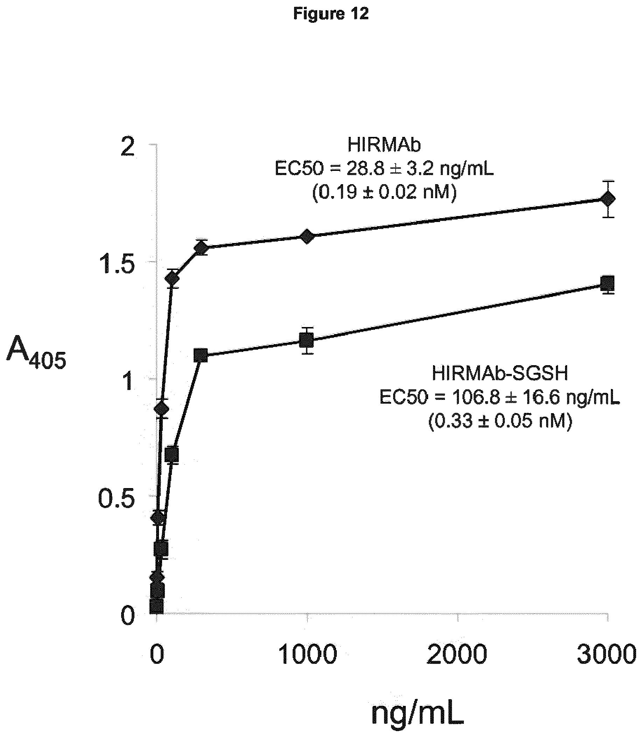

**Figure 12****.** Binding of either the chimeric HIRMAb or the HIRMAb-SGSH fusion protein to the HIR extracellular domain (ECD) is saturable. The ED₅₀ of HIRMAb-SGSH binding to the HIR ECD, 0.33 ± 0.05 nM, is comparable to the ED₅₀ of the binding of the chimeric HIRMAb, 0.19 ± 0.02 nM, after normalization for differences in molecular weight.

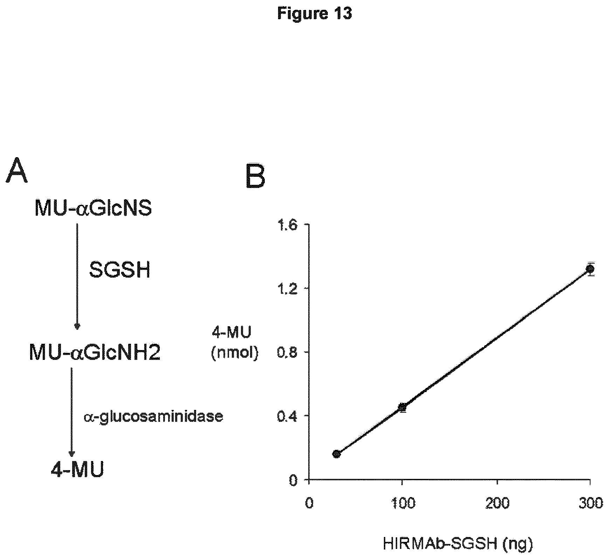

**Figure 13****.** (A) The SGSH flurometric enzyme assay is a 2-step assay. The substrate, 4-methylumbelliferyl-α-D-N-sulphoglucosaminide (MU-α-GlcNS), is converted by SGSH to methylumbelliferyl-α-D-glucosaminide (MU-α-GlcNH2), which is then converted to the fluorescent product, 4-methyl umbelliferone (4-MU) by the second step enzyme, α-glucosaminidase. (B) Linear formation of the 4-MU product by the addition of the 30 to 300 ng of the HIRMAb-SGSH fusion protein. Data are mean ± SD of 3 replicates.

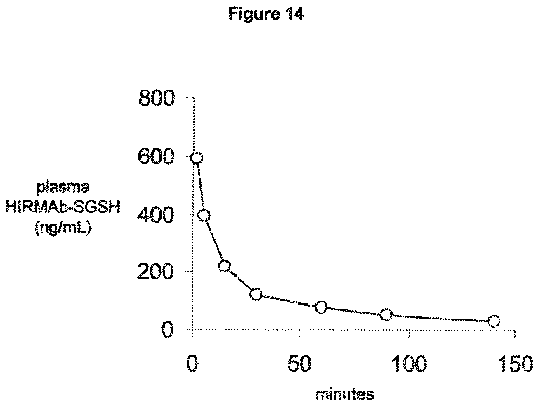

**Figure 14****.** The plasma TCA-precipitable [125I]-HIRMAb-SGSH fusion protein concentration, ng/mL, in the adult Rhesus monkey is plotted vs time over a 140 min period after a single IV injection of 19 ug/kg the fusion protein..

### DETAILED DESCRIPTION OF THE INVENTION

The blood brain barrier (BBB) is a severe impediment to the delivery of systemically administered lysosomal enzyme (e.g., recombinant SGSH) to the central nervous system. The methods and compositions described herein address the factors that are important in delivering a therapeutically significant level of an enzyme deficient in mucopolysaccharidosis III (MPS-III), such as SGSH, NAGLU, HGSNAT, GNS, across the BBB to the CNS: 1) Modification of an enzyme deficient in MPS-III to allow it to cross the BBB via transport on an endogenous BBB transporter; 2) the amount and rate of uptake of systemically administered modified enzyme into the CNS, via retention of enzyme activity following the modification required to produce BBB transport. Various aspects of the methods and compositions described herein address these factors, by (1) providing fusion antibodies comprising an enzyme (i.e., a protein having SGSH activity) fused, with or without intervening sequence, to an immunoglobulin (heavy chain or light chain) directed against the extracellular domain of an endogenous BBB receptor; and (2) establishing therapeutically effective systemic doses of the fusion antibodies based on the uptake in the CNS and the specific activity. In some embodiments, the antibody to the endogenous BBB receptor is an antibody to the human insulin receptor (HIR Ab).

Accordingly, provided herein are compositions and methods for treating an enzyme (e.g., SGSH) deficiency in the central nervous system by systemically administering to a subject in need thereof a therapeutically effective dose of a bifunctional BBB receptor Ab-enzyme fusion antibody having enzyme activity and selectively binding to the extracellular domain of an endogenous BBB receptor transporter such as the human insulin receptor.

### Some Definitions

"Treatment" or "treating" as used herein includes achieving a therapeutic benefit and/or a prophylactic benefit. By therapeutic benefit is meant eradication or amelioration of the underlying disorder or condition being treated. For example, in an individual with MPS-IIIA, therapeutic benefit includes partial or complete halting of the progression of the disorder, or partial or complete reversal of the disorder. Also, a therapeutic benefit is achieved with the eradication or amelioration of one or more of the physiological or psychological symptoms associated with the underlying condition such that an improvement is observed in the patient, notwithstanding the fact that the patient may still be affected by the condition. A prophylactic benefit of treatment includes prevention of a condition, retarding the progress of a condition (e.g., slowing the progression of a lysosomal storage disorder), or decreasing the likelihood of occurrence of a condition. As used herein, "treating" or "treatment" includes prophylaxis.

As used herein, the term "effective amount" can be an amount, which when administered systemically, is sufficient to effect beneficial or desired results in the CNS, such as beneficial or desired clinical results, or enhanced cognition, memory, mood, or other desired CNS results. An effective amount is also an amount that produces a prophylactic effect, e.g., an amount that delays, reduces, or eliminates the appearance of a pathological or undesired condition. Such conditions include, but are not limited to, mental retardation, hearing loss, and neurodegeneration. An effective amount can be administered in one or more administrations. In terms of treatment, an "effective amount" of a composition provided herein is an amount that is sufficient to palliate, ameliorate, stabilize, reverse or slow the progression of a disorder, e.g., a neurological disorder. An "effective amount" may be of any of the compositions provided herein used alone or in conjunction with one or more agents used to treat a disease or disorder. An "effective amount" of a therapeutic agent within the meaning of the present embodiments will be determined by a patient's attending physician or veterinarian. Such amounts are readily ascertained by one of ordinary skill in the art and will a therapeutic effect when administered in accordance with the present embodiments. Factors which influence what a therapeutically effective amount will be include, the enzyme specific activity of the fusion antibody administered, its absorption profile (e.g., its rate of uptake into the brain), time elapsed since the initiation of the disorder, and the age, physical condition, existence of other disease states, and nutritional status of the individual being treated. Additionally, other medication the patient may be receiving will affect the determination of the therapeutically effective amount of the therapeutic agent to administer.

A "subject" or an "individual," as used herein, is an animal, for example, a mammal. In some embodiments a "subject" or an "individual" is a human. In some embodiments, the subject suffers from MPS-IIIA.

In some embodiments, a pharmacological composition comprising a fusion antibody is "administered peripherally" or "peripherally administered." As used herein, these terms refer to any form of administration of an agent, e.g., a therapeutic agent, to an individual that is not direct administration to the CNS, i.e., that brings the agent in contact with the non-brain side of the blood-brain barrier. "Peripheral administration," as used herein, includes intravenous, intra-arterial, subcutaneous, intramuscular, intraperitoneal, transdermal, by inhalation, transbuccal, intranasal, rectal, oral, parenteral, sublingual, or trans-nasal.

A "pharmaceutically acceptable carrier" or "pharmaceutically acceptable excipient" herein refers to any carrier that does not itself induce the production of antibodies harmful to the individual receiving the composition. Such carriers are well known to those of ordinary skill in the art. A thorough discussion of pharmaceutically acceptable carriers/excipients can be found in Remington's Pharmaceutical Sciences, Gennaro, AR, ed., 20th edition, 2000: Williams and Wilkins PA, USA.. Exemplary pharmaceutically acceptable carriers can include salts, for example, mineral acid salts such as hydrochlorides, hydrobromides, phosphates, sulfates, and the like; and the salts of organic acids such as acetates, propionates, malonates, benzoates, and the like. For example, compositions described herein may be provided in liquid form, and formulated in saline based aqueous solution of varying pH (5-8), with or without detergents such polysorbate-80 at 0.01-1%, or carbohydrate additives, such mannitol, sorbitol, or trehalose. Commonly used buffers include histidine, acetate, phosphate, or citrate.

A "recombinant host cell" or "host cell" refers to a cell that includes an exogenous polynucleotide, regardless of the method used for insertion, for example, direct uptake, transduction, f-mating, or other methods known in the art to create recombinant host cells. The exogenous polynucleotide may be maintained as a nonintegrated vector, for example, a plasmid, or alternatively, may be integrated into the host genome.

The terms "polypeptide," "peptide" and "protein" are used interchangeably herein to refer to a polymer of amino acid residues. That is, a description directed to a polypeptide applies equally to a description of a peptide and a description of a protein, and vice versa. The terms apply to naturally occurring amino acid polymers as well as amino acid polymers in which one or more amino acid residues is a non-naturally occurring amino acid, e.g., an amino acid analog. As used herein, the terms encompass amino acid chains of any length, including full length proteins (*i.e*., antigens), wherein the amino acid residues are linked by covalent peptide bonds.

The term "amino acid" refers to naturally occurring and non-naturally occurring amino acids, as well as amino acid analogs and amino acid mimetics that function in a manner similar to the naturally occurring amino acids. Naturally encoded amino acids are the 20 common amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine) and pyrolysine and selenocysteine. Amino acid analogs refers to compounds that have the same basic chemical structure as a naturally occurring amino acid, *i*.*e*., an α carbon that is bound to a hydrogen, a carboxyl group, an amino group, and an R group, such as, homoserine, norleucine, methionine sulfoxide, methionine methyl sulfonium. Such analogs have modified R groups (such as, norleucine) or modified peptide backbones, but retain the same basic chemical structure as a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The term "nucleic acid" refers to deoxyribonucleotides, deoxyribonucleosides, ribonucleosides, or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless specifically limited otherwise, the term also refers to oligonucleotide analogs including PNA (peptidonucleic acid), analogs of DNA used in antisense technology (phosphorothioates, phosphoroamidates, and the like). Unless otherwise indicated, a particular nucleic acid sequence also implicitly encompasses conservatively modified variants thereof (including but not limited to, degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Cassol *et al.* (1992); Rossolini et al., Mol. Cell. Probes 8:91-98 (1994)).

The terms "isolated" and "purified" refer to a material that is substantially or essentially removed from or concentrated in its natural environment. For example, an isolated nucleic acid may be one that is separated from the nucleic acids that normally flank it or other nucleic acids or components (proteins, lipids, etc...) in a sample. In another example, a polypeptide is purified if it is substantially removed from or concentrated in its natural environment. Methods for purification and isolation of nucleic acids and proteins are well known in the art.

### The Blood Brain Barrier

In one aspect, provided herein are compositions and methods that utilize an enzyme deficient in MPS-III (e.g., SGSH) fused to an immunoglobulin capable of crossing the blood brain barrier (BBB) via receptor-mediated transport on an endogenous BBB receptor/transporter. An exemplary endogenous transporter for targeting is the insulin receptor on the BBB. The BBB insulin receptor mediates the transport of circulating insulin into the brain, as well as certain peptidomimetic monoclonal antibodies (MAb) such as the HIRMAb. Other endogenous transporters that might be targeted with either an endogenous ligand or a peptidomimetic MAb include the BBB transferrin receptor, the BBB insulin-like growth factor (IGF) receptor, the BBB leptin receptor, or the BBB low density lipoprotein (LDL) receptor. The compositions and methods are useful in transporting SGSH from the peripheral blood and across the blood brain barrier into the CNS. As used herein, the "blood-brain barrier" refers to the barrier between the peripheral circulation and the brain and spinal cord which is formed by tight junctions within the brain capillary endothelial plasma membranes and creates an extremely tight barrier that restricts the transport of molecules into the brain; the BBB is so tight that it is capable of restricting even molecules as small as urea, molecular weight of 60 Da. The blood-brain barrier within the brain, the blood-spinal cord barrier within the spinal cord, and the blood-retinal barrier within the retina, are contiguous capillary barriers within the central nervous system (CNS), and are collectively referred to as the blood-brain barrier or BBB.

The BBB limits the development of new neurotherapeutics, diagnostics, and research tools for the brain and CNS. Most large molecule therapeutics such as recombinant proteins, antisense drugs, gene medicines, purified antibodies, or RNA interference (RNAi)-based drugs do not cross the BBB in pharmacologically significant amounts. While it is generally assumed that small molecule drugs can cross the BBB, in fact, <2% of all small molecule drugs are active in the brain owing to the lack transport across the BBB. A molecule must be lipid soluble and have a molecular weight less than 400 Daltons (Da) in order to cross the BBB in pharmacologically significant amounts, and the vast majority of small molecules do not have these dual molecular characteristics. Therefore, most potentially therapeutic, diagnostic, or research molecules do not cross the BBB in pharmacologically active amounts. So as to bypass the BBB, invasive transcranial drug delivery strategies are used, such as intracerebro-ventricular (ICV) infusion, intracerebral (IC) administration, and convection enhanced diffusion (CED). Transcranial drug delivery to the brain is expensive, invasive, and largely ineffective. The ICV route, also called the intra-thecal (IT) route, delivers SGSH only to the ependymal or meningeal surface of the brain, not into brain parenchyma, which is typical for drugs given by the ICV route. The IC administration of an enzyme such as SGSH, only provides local delivery, owing to the very low efficiency of protein diffusion within the brain. Similarly, the CED route only provides local delivery in brain near the catheter tip, as drug penetration via diffusion is limited.

The methods described herein offer an alternative to these highly invasive and generally unsatisfactory methods for bypassing the BBB, allowing a functional SGSH to cross the BBB from the peripheral blood into the CNS following systemic administration of an HIRMAb-SGSH fusion antibody composition described herein. The methods described herein exploit the expression of insulin receptors (e.g., human insulin receptors) on the BBB to shuttle a desired bifunctional HIRMAb-SGSH fusion antibody from peripheral blood into the CNS.

### Endogenous Receptors

Certain endogenous small molecules in blood, such as glucose or amino acids, are water soluble, yet are able to penetrate the BBB, owing to carrier-mediated transport (CMT) on certain BBB carrier systems. For example, glucose penetrates the BBB via CMT on the GLUT1 glucose transporter. Amino acids, including therapeutic amino acids such as L-DOPA, penetrate the BBB via CMT on the LAT1 large neutral amino acid transporter. Similarly, certain endogenous large molecules in blood, such as insulin, transferrin, insulin-like growth factors, leptin, or low density lipoprotein are able to penetrate the BBB, owing to receptor-mediated transcytosis (RMT) on certain BBB receptor systems. For example, insulin penetrates the BBB via RMT on the insulin receptor. Transferrin penetrates the BBB via RMT on the transferrin receptor. Insulin-like growth factors may penetrate the BBB via RMT on the insulin-like growth factor receptor. Leptin may penetrate the BBB via RMT on the leptin receptor. Low density lipoprotein may penetrate the BBB via transport on the low density lipoprotein receptor.

The BBB has been shown to have specific receptors, including insulin receptors, that allow the transport from the blood to the brain of several macromolecules. In particular, insulin receptors are suitable as transporters for the HIR Ab-SGSH fusion antibodies described herein. The HIR-SGSH fusion antibodies described herein bind to the extracellular domain (ECD) of the human insulin receptor.

Insulin receptors and their extracellular, insulin binding domain (ECD) have been extensively characterized in the art both structurally and functionally. See, e.g., Yip et al. (2003), J Biol. Chem, 278(30):27329-27332; and Whittaker et al. (2005), J Biol Chem, 280(22):20932-20936. The amino acid and nucleotide sequences of the human insulin receptor can be found under GenBank accession No. NM_000208.

### Antibodies that bind to an insulin receptor-mediated transport system

One noninvasive approach for the delivery of an enzyme deficient in MPS-III (e.g., SGSH) to the CNS is to fuse the SGSH to an antibody that selectively binds to the ECD of the insulin receptor. Insulin receptors expressed on the BBB can thereby serve as a vector for transport of the SGSH across the BBB. Certain ECD-specific antibodies may mimic the endogenous ligand and thereby traverse a plasma membrane barrier via transport on the specific receptor system. Such insulin receptor antibodies act as molecular "Trojan horses," or "TH" as depicted schematically in Fig. 1. By itself, SGSH normally does not cross the blood-brain barrier (BBB). However, following fusion of the SGSH to the TH, the enzyme is able to cross the BBB, and the brain cell membrane, by trafficking on the endogenous BBB receptor such as the IR, which is expressed at both the BBB and brain cell membranes in the brain (Fig. 1).

Thus, despite the fact that antibodies and other macromolecules are normally excluded from the brain, they can be an effective vehicle for the delivery of molecules into the brain parenchyma if they have specificity for the extracellular domain of a receptor expressed on the BBB, e.g., the insulin receptor. In certain embodiments, an HIR Ab-SGSH fusion antibody binds an exofacial epitope on the human BBB HIR and this binding enables the fusion antibody to traverse the BBB via a transport reaction that is mediated by the human BBB insulin receptor.

The term "antibody" describes an immunoglobulin whether natural or partly or wholly synthetically produced. The term also covers any polypeptide or protein having a binding domain which is, or is homologous to, an antigen-binding domain. CDR grafted antibodies are also contemplated by this term.

"Native antibodies" and "native immunoglobulins" are usually heterotetrameric glycoproteins of about 150,000 daltons, composed of two identical light (L) chains and two identical heavy (H) chains. Each light chain is typically linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ("VH") followed by a number of constant domains ("CH"). Each light chain has a variable domain at one end ("VL") and a constant domain ("CL") at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable domain" refers to protein domains that differ extensively in sequence among family members (i.e., among different isoforms, or in different species). With respect to antibodies, the term "variable domain" refers to the variable domains of antibodies that are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the "framework region" or "FR". The variable domains of unmodified heavy and light chains each comprise four FRs (FR1, FR2, FR3 and FR4, respectively), largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from three "complementarity determining regions" or "CDRs", which directly bind, in a complementary manner, to an antigen and are known as CDR1, CDR2, and CDR3 respectively.

In the light chain variable domain, the CDRs typically correspond to approximately residues 24-34 (CDRL1), 50-56 (CDRL2) and 89-97 (CDRL3), and in the heavy chain variable domain the CDRs typically correspond to approximately residues 31-35 (CDRH1), 50-65 (CDRH2) and 95-102 (CDRH3); Kabat et al., Sequences of Proteins of Immunological Interest, 5th Ed. Public Health Service, National Institutes of Health, Bethesda, Md. (1991)) and/or those residues from a "hypervariable loop" (i.e., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, J. Mol. Biol. 196:901 917 (1987)).

As used herein, "variable framework region" or "VFR" refers to framework residues that form a part of the antigen binding pocket or groove and/or that may contact antigen. In some embodiments, the framework residues form a loop that is a part of the antigen binding pocket or groove. The amino acids residues in the loop may or may not contact the antigen. In an embodiment, the loop amino acids of a VFR are determined by inspection of the three-dimensional structure of an antibody, antibody heavy chain, or antibody light chain. The three-dimensional structure can be analyzed for solvent accessible amino acid positions as such positions are likely to form a loop and/or provide antigen contact in an antibody variable domain. Some of the solvent accessible positions can tolerate amino acid sequence diversity and others (e.g. structural positions) can be less diversified. The three dimensional structure of the antibody variable domain can be derived from a crystal structure or protein modeling. In some embodiments, the VFR comprises, consist essentially of, or consists of amino acid positions corresponding to amino acid positions 71 to 78 of the heavy chain variable domain, the positions defined according to Kabat *et al.,* 1991. In some embodiments, VFR forms a portion of Framework Region 3 located between CDRH2 and CDRH3. The VFR can form a loop that is well positioned to make contact with a target antigen or form a part of the antigen binding pocket.

Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g., IgG1, IgG2, IgG3, IgG4, IgA, and IgA2. The heavy-chain constant domains (Fc) that correspond to the different classes of immunoglobulins are called α, δ, ε, γ, and µ, respectively. The subunit structures and three-dimensional configurations of different classes of immunoglobulins are well known.

The "light chains" of antibodies (immunoglobulins) from any vertebrate species can be assigned to one of two clearly distinct types, called kappa or ("κ") and lambda or ("λ"), based on the amino acid sequences of their constant domains.

In referring to an antibody or fusion antibody described herein, the terms "selectively bind," "selectively binding," "specifically binds," or "specifically binding" refer to binding to the antibody or fusion antibody to its target antigen for which the dissociation constant (Kd) is about 10⁻⁶ M or lower, i.e., 10⁻⁷, 10⁻⁸, 10⁻⁹, 10⁻¹⁰, 10⁻¹¹, or 10⁻¹²M.

The term antibody as used herein will also be understood to mean one or more fragments of an antibody that retain the ability to specifically bind to an antigen, (see generally, Holliger et al., Nature Biotech. 23 (9) 1126-1129 (2005)). Non-limiting examples of such antibodies include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544 546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423 426; and Huston et al. (1988) Proc. Natl. Acad. Sci. USA 85:5879 5883; and Osbourn et al. (1998) Nat. Biotechnol. 16:778). Such single chain antibodies are also intended to be encompassed within the term antibody. Any VH and VL sequences of specific scFv can be linked to human immunoglobulin constant region cDNA or genomic sequences, in order to generate expression vectors encoding complete IgG molecules or other isotypes. VH and VL can also be used in the generation of Fab, Fv or other fragments of immunoglobulins using either protein chemistry or recombinant DNA technology. Other forms of single chain antibodies, such as diabodies are also encompassed.

"F(ab')2" and "Fab"' moieties can be produced by treating immunoglobulin (monoclonal antibody) with a protease such as pepsin and papain, and includes an antibody fragment generated by digesting immunoglobulin near the disulfide bonds existing between the hinge regions in each of the two H chains. For example, papain cleaves IgG upstream of the disulfide bonds existing between the hinge regions in each of the two H chains to generate two homologous antibody fragments in which an L chain composed of VL (L chain variable region) and CL (L chain constant region), and an H chain fragment composed of VH (H chain variable region) and CHγ1 (γ1 region in the constant region of H chain) are connected at their C terminal regions through a disulfide bond. Each of these two homologous antibody fragments is called Fab'. Pepsin also cleaves IgG downstream of the disulfide bonds existing between the hinge regions in each of the two H chains to generate an antibody fragment slightly larger than the fragment in which the two above-mentioned Fab' are connected at the hinge region. This antibody fragment is called F(ab')2.

The Fab fragment also contains the constant domain of the light chain and the first constant domain (CHI) of the heavy chain. Fab' fragments differ from Fab fragments by the addition of a few residues at the carboxyl terminus of the heavy chain CH1 domain including one or more cysteine(s) from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

"Single-chain Fv" or "sFv" antibody fragments comprise a VH, a VL, or both a VH and VL domain of an antibody, wherein both domains are present in a single polypeptide chain. In some embodiments, the Fv polypeptide further comprises a polypeptide linker between the VH and VL domains which enables the sFv to form the desired structure for antigen binding. For a review of sFv see, e.g., Pluckthun in The Pharmacology of Monoclonal Antibodies, Vol. 113, Rosenburg and Moore eds. Springer-Verlag, New York, pp. 269 315 (1994).

A "chimeric" antibody includes an antibody derived from a combination of different mammals. The mammal may be, for example, a rabbit, a mouse, a rat, a goat, or a human. The combination of different mammals includes combinations of fragments from human and mouse sources.

In some embodiments, an antibody provided herein is a monoclonal antibody (MAb), typically a chimeric human-mouse antibody derived by humanization of a mouse monoclonal antibody. Such antibodies are obtained from, e.g., transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas.

For use in humans, a HIR Ab is preferred that contains enough human sequence that it is not significantly immunogenic when administered to humans, e.g., about 80% human and about 20% mouse, or about 85% human and about 15% mouse, or about 90% human and about 10% mouse, or about 95% human and 5% mouse, or greater than about 95% human and less than about 5% mouse, or 100% human. A more highly humanized form of the HIR MAb can also be engineered, and the humanized HIR Ab has activity comparable to the murine HIR Ab and can be used in embodiments provided herein. See, e.g., U.S. Patent Application Publication Nos. 20040101904, filed Nov. 27, 2002 and 20050142141, filed Feb. 17, 2005. Humanized antibodies to the human BBB insulin receptor with sufficient human sequences for use in the present embodiments are described in, e.g., Boado et al. (2007), Biotechnol Bioeng, 96(2):381-391.

In exemplary embodiments, the HIR antibodies or fusion antibodies (e.g., HIR-SGHS, HIR-NGLU, HIR-HGSNAT, HIR-GNS) derived therefrom contain an immunoglobulin heavy chain comprising CDRs corresponding to the sequence of at least one of the HC CDRs listed in Fig. 7 (SEQ ID NOs 1-3) or a variant thereof. For example, a HC CDR1 corresponding to the amino acid sequence of SEQ ID NO:1 with up to 1, 2, 3, 4, 5, or 6 single amino acid mutations, a HC CDR2 corresponding to the amino acid sequence of SEQ ID NO:2 with up to 1, 2, 3, 4, 5, 6, 7, 8, 9, or 10 single amino acid mutations, or a HC CDR3 corresponding to the amino acid sequence of SEQ ID NO:3 with up to 1, or 2 single amino acid mutations, where the single amino acid mutations are substitutions, deletions, or insertions.

In other embodiments, the HIR Abs or fusion Abs (e.g., HIR Ab-SGHS, HIR Ab-NGLU, HIR-Ab HGSNAT, HIR Ab -GNS) contain an immunoglobulin HC the amino acid sequence of which is at least 50% identical (i.e., at least, 55, 60, 65, 70, 75, 80, 85, 90, 95, or any other percent up to 100% identical) to SEQ ID NO:7 (shown in Fig. 5).

In some embodiments, the HIR Abs or fusion Abs (e.g., HIR Ab-SGHS, HIR Ab-NGLU, HIR Ab-HGSNAT, HIR Ab-GNS) include an immunoglobulin light chain comprising CDRs corresponding to the sequence of at least one of the LC CDRs listed in Fig. 7 (SEQ ID NOs: 4-6) or a variant thereof. For example, a LC CDR1 corresponding to the amino acid sequence of SEQ ID NO:4 with up to 1, 2, 3, 4, or 5 single amino acid mutations, a LC CDR2 corresponding to the amino acid sequence of SEQ ID NO:5 with up to 1, 2, 3, or 4 single amino acid mutations, or a LC CDR3 corresponding to the amino acid sequence of SEQ ID NO:6 with up to 1, 2, 3, 4, or 5 single amino acid mutations.

In other embodiments, the HIR Abs or fusion Abs (e.g., HIR Ab-SGHS, HIR Ab-NGLU, HIR Ab-HGSNAT, HIR Ab-GNS) contain an immunoglobulin LC the amino acid sequence of which is at least 50% identical (i.e., at least, 55, 60, 65, 70, 75, 80, 85, 90, 95, or any other percent up to 100% identical) to SEQ ID NO:8 (shown in Fig. 6).

In yet other embodiments, the HIR Abs or fusion Abs (e.g., HIR Ab-SGHS, HIR Ab-NGLU, HIR Ab-HGSNAT, HIR Ab-GNS) contain both a heavy chain and a light chain corresponding to any of the above-mentioned HIR heavy chains and HIR light chains.

HIR antibodies provided herein may be glycosylated or non-glycosylated. If the antibody is glycosylated, any pattern of glycosylation that does not significantly affect the function of the antibody may be used. Glycosylation can occur in the pattern typical of the cell in which the antibody is made, and may vary from cell type to cell type. For example, the glycosylation pattern of a monoclonal antibody produced by a mouse myeloma cell can be different than the glycosylation pattern of a monoclonal antibody produced by a transfected Chinese hamster ovary (CHO) cell. In some embodiments, the antibody is glycosylated in the pattern produced by a transfected Chinese hamster ovary (CHO) cell.

One of ordinary skill in the art will appreciate that current technologies permit a vast number of sequence variants of candidate HIR Abs or known HIR Abs to be readily generated be (e.g., *in vitro*) and screened for binding to a target antigen such as the ECD of the human insulin receptor or an isolated epitope thereof. See, e.g., Fukuda et al. (2006) "In vitro evolution of single-chain antibodies using mRNA display," Nuc. Acid Res., 34(19) (published online) for an example of ultra high throughput screening of antibody sequence variants. See also, Chen et al. (1999), "In vitro scanning saturation mutagenesis of all the specificity determining residues in an antibody binding site," Prot Eng, 12(4): 349-356. An insulin receptor ECD can be purified as described in, e.g., Coloma et al. (2000) Pharm Res, 17:266-274, and used to screen for HIR Abs and HIR Ab sequence variants of known HIR Abs.

Accordingly, in some embodiments, a genetically engineered HIR Ab, with the desired level of human sequences, is fused to an enzyme deficient in MPS-III (e.g., SGSH), to produce a recombinant fusion antibody that is a bi-functional molecule. For example, the HIR Ab-SGSH fusion antibody: (i) binds to an extracellular domain of the human insulin receptor; (ii) catalyzes hydrolysis of sulfate linkages in heparan sulfate; and (iii) is able to cross the BBB, via transport on the BBB HIR, and retain SGSH activity once inside the brain, following peripheral administration.

### N-sulfoglucosamine sulfohydrolase (SGSH)

Systemic administration (e.g., by intravenous injection) of recombinant SGSH is not expected to rescue a deficiency of SGSH in the CNS of patients suffering from MPS-IIIA. SGSH does not cross the BBB, and the lack of transport of the enzyme across the BBB prevents it from having a significant therapeutic effect in the CNS following peripheral administration. However, present inventors have discovered that when the SGSH is fused to an antibody that crosses the BBB such as HIR Ab (e.g., by a covalent linker), this enzyme is now able to enter the CNS from blood following a non-invasive peripheral route of administration such as intravenous, intra-arterial, intramuscular, subcutaneous, intraperitoneal, or even oral administration. Administration of a HIR Ab-SGSH fusion antibody enables delivery of SGSH activity into the brain from peripheral blood. Described herein is the determination of a systemic dose of the HIR Ab-SGSH fusion antibody that is therapeutically effective for treating a SGSH deficiency in the CNS. As described herein, appropriate systemic doses of an HIR Ab-SGSH fusion antibody are established based on a quantitative determination of CNS uptake characteristics and enzymatic activity of an HIR Ab-enzyme fusion antibody.

Heparan sulfate is a sulfated glycosoaminoglycan synthesized in the oligodendrocytes in the central nervous system. As used herein, SGSH (e.g., the human SGSH sequence listed under GenBank Accession No. NP_000190) refers to any naturally occurring or artificial enzyme that can catalyze the hydrolysis of N-linked sulfate from heparan sulfate.

SGSH is a member of a family of sulfatases that requires a specific post-translational modification for expression of SGSH enzyme activity. The activity of the SGSH enzyme is activated following the conversion of Cys-70 (of the intact SGSH protein including the signal peptide) to a formylglycine residue by a sulfatase modifying factor type 1 (SUMF1), which is also called the formylglycine generating enzyme (FGE). In some embodiments, the fusion antibody comprising SGSH is post-translationally modified by a sulfatase modifying factor type 1 (SUMF1). In some embodiments, the post-translational modification comprises a cysteine to formylglycine conversion. In some embodiments, the fusion antibody comprises an SGSH that comprises a formylglycine residue.

In some embodiments, SGSH has an amino acid sequence that is at least 50% identical (i.e., at least, 55, 60, 65, 70, 75, 80, 85, 90, 95, or any other percent up to 100% identical) to the amino acid sequence of human SGSH, a 502 amino acid protein listed under Genbank NP_000190, or a 482 amino acid subsequence thereof, which lacks a 20 amino acid signal peptide, and corresponds to SEQ ID NO:9 (Fig. 8). The structure-function relationship of human SGSH has been investigated and Cys-70 is a residue conserved in sulfatases for post-translational modification as described by Scott et al. (1995), "Cloning of the sulphamidase gene and identification of mutations in Sanfilippo A syndrome, Nature Genetics, 11:465-467. The Asp-51 residue plays a role in divalent cation binding as described in Gliddon et al. (2004), "Purification and characterization of recombinant murine sulfamidase," Molec. Genet. Metab. 83:239-245. N-linked glycosylation sites are present at Asn-41, Asn-142, Asn-151, Asn-264, and Asn-413, as described by DiNatale et al.(2001), "Heparan N-sulfatase: in vitro mutagenesis of potential N-glycosylation sites," Biochem. Biophys. Res. Comm. 280:1251-1257, where the amino acid numbering system includes the 20 amino acid signal peptide.

In some embodiments, SGSH has an amino acid sequence at least 50% identical (i.e., at least, 55, 60, 65, 70, 75, 80, 85, 90, 95, or any other percent up to 100% identical) to SEQ ID NO:9 (shown in Fig. 8). Sequence variants of a canonical SGSH sequence such as SEQ ID NO:9 can be generated, e.g., by random mutagenesis of the entire sequence or specific subsequences corresponding to particular domains. Alternatively, site directed mutagenesis can be performed reiteratively while avoiding mutations to residues known to be critical to SGSH function such as those given above. Further, in generating multiple variants of an SGSH sequence, mutation tolerance prediction programs can be used to greatly reduce the number of non-functional sequence variants that would be generated by strictly random mutagenesis. Various programs) for predicting the effects of amino acid substitutions in a protein sequence on protein function (e.g., SIFT, PolyPhen, PANTHER PSEC, PMUT, and TopoSNP) are described in, e.g., Henikoff et al. (2006), "Predicting the Effects of Amino Acid Substitutions on Protein Function," Annu. Rev. Genomics Hum. Genet., 7:61-80. SGSH sequence variants can be screened for of SGSH activity/retention of SGSH activity by a fluorometric enzymatic assay known in the art, Karpova et al. (1996): A fluorimetric enzyme assay for the diagnosis of Sanfilippo disease type A (MPS IIIA), J. Inher. Metab. Dis. 19: 278-285. Accordingly, one of ordinary skill in the art will appreciate that a very large number of operable SGSH sequence variants can be obtained by generating and screening extremely diverse "libraries" of SGSH sequence variants by methods that are routine in the art, as described above.

Percent sequence identity is determined by conventional methods. See, for example, Altschul et al., Bull. Math. Bio. 48:603 (1986), and Henikoff and Henikoff, Proc. Natl. Acad. Sci. USA 89:10915 (1992). Briefly, two amino acid sequences are aligned to optimize the alignment scores using a gap opening penalty of 10, a gap extension penalty of 1, and the "BLOSUM62" scoring matrix of Henikoff and Henikoff (ibid.). The percent identity is then calculated as: ([Total number of identical matches]/[length of the longer sequence plus the number of gaps introduced into the longer sequence in order to align the two sequences])(100).

Those skilled in the art appreciate that there are many established algorithms available to align two amino acid sequences. The "FASTA" similarity search algorithm of Pearson and Lipman is a suitable protein alignment method for examining the level of identity shared by an amino acid sequence disclosed herein and the amino acid sequence of another peptide. The FASTA algorithm is described by Pearson and Lipman, Proc. Nat'lAcad. Sci. USA 85:2444 (1988), and by Pearson, Meth. Enzymol. 183:63 (1990). Briefly, FASTA first characterizes sequence similarity by identifying regions shared by the query sequence (e.g., SEQ ID NO:9 or SEQ ID NO: 16) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence and the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman and Wunsch, J. Mol. Biol. 48:444 (1970); Sellers, SIAM J. Appl. Math. 26:787 (1974)), which allows for amino acid insertions and deletions. Illustrative parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, Meth. Enzymol. 183:63 (1990).

The present embodiments also include proteins having a conservative amino acid change, compared with an amino acid sequence disclosed herein. Among the common amino acids, for example, a "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine. The BLOSUM62 table is an amino acid substitution matrix derived from about 2,000 local multiple alignments of protein sequence segments, representing highly conserved regions of more than 500 groups of related proteins (Henikoff and Henikoff, Proc. Nat'l Acad. Sci. USA 89:10915 (1992)). Accordingly, the BLOSUM62 substitution frequencies can be used to define conservative amino acid substitutions that may be introduced into the amino acid sequences of the present embodiments. Although it is possible to design amino acid substitutions based solely upon chemical properties (as discussed above), the language "conservative amino acid substitution" preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (e.g., 1, 2 or 3), while more preferred conservative amino acid substitutions are characterized by a BLOSUM62 value of at least 2 (e.g., 2 or 3).

It also will be understood that amino acid sequences may include additional residues, such as additional N- or C-terminal amino acids, and yet still be essentially as set forth in one of the sequences disclosed herein, so long as the sequence retains sufficient biological protein activity to be functional in the compositions and methods of the present embodiments.

### Alpha-N-acetylglucosaminidase (NAGLU)

Systemic administration (e.g., by intravenous injection) of recombinant NAGLU is not expected to rescue a deficiency of NAGLU in the CNS of patients suffering from MPS-IIIB. NAGLU does not cross the BBB, and the lack of transport of the enzyme across the BBB prevents it from having a significant therapeutic effect in the CNS following peripheral administration. However, present inventors have discovered that when the NAGLU is fused to an antibody that crosses the BBB such as HIR Ab (e.g., by a covalent linker), this enzyme is now able to enter the CNS from blood following a non-invasive peripheral route of administration such as intravenous, intra-arterial, intramuscular, subcutaneous, intraperitoneal, or even oral administration. Administration of a HIR Ab-NAGLU fusion antibody enables delivery of NAGLU activity into the brain from peripheral blood. Described herein is the determination of a systemic dose of the HIR Ab- NAGLU fusion antibody that is therapeutically effective for treating an NAGLU deficiency in the CNS. As described herein, appropriate systemic doses of an HIR Ab-NAGLU fusion antibody are established based on a quantitative determination of CNS uptake characteristics and enzymatic activity of an HIR Ab-enzyme fusion antibody.

As used herein, NAGLU (e.g., the human NAGLU sequence listed under GenBank Accession No. NP_000254) refers to any naturally occurring or artificial enzyme that can catalyze hydrolysis of N-acetyl-D-glucosamine residues in N-acetyl-alpha-D-glucosaminides.

In some embodiments, NAGLU has an amino acid sequence that is at least 50% identical (i.e., at least, 55, 60, 65, 70, 75, 80, 85, 90, 95, or any other percent up to 100% identical) to the amino acid sequence of human NAGLU, a protein listed under Genbank NP_000254. In some embodiments, NAGLU has an amino acid sequence at least 50% identical (i.e., at least, 55, 60, 65, 70, 75, 80, 85, 90, 95, or any other percent up to 100% identical) to SEQ ID NO:17. Sequence variants of a canonical NAGLU sequence such as SEQ ID NO:17 can be generated, e.g., by random mutagenesis of the entire sequence or specific subsequences corresponding to particular domains. Alternatively, site directed mutagenesis can be performed reiteratively while avoiding mutations to residues known to be critical to NAGLU function such as those given above. Further, in generating multiple variants of an NAGLU sequence, mutation tolerance prediction programs can be used to greatly reduce the number of non-functional sequence variants that would be generated by strictly random mutagenesis. Various programs for predicting the effects of amino acid substitutions in a protein sequence on protein function (e.g., SIFT, PolyPhen, PANTHER PSEC, PMUT, and TopoSNP) are described in, e.g., Henikoff et al. (2006), "Predicting the Effects of Amino Acid Substitutions on Protein Function," Annu. Rev. Genomics Hum. Genet., 7:61-80. NAGLU sequence variants can be screened for of NAGLU activity/retention of NAGLU activity by a fluorometric enzymatic assay known in the art. Accordingly, one of ordinary skill in the art will appreciate that a very large number of operable NAGLU sequence variants can be obtained by generating and screening extremely diverse "libraries" of NAGLU sequence variants by methods that are routine in the art, as described above.

### Heparin-alpha-glucosaminide N-acetyltransferase (HGSNAT)

Systemic administration (e.g., by intravenous injection) of recombinant HGSNAT is not expected to rescue a deficiency of HGSNAT in the CNS of patients suffering from MPS-IIIC. HGSNAT does not cross the BBB, and the lack of transport of the enzyme across the BBB prevents it from having a significant therapeutic effect in the CNS following peripheral administration. However, present inventors have discovered that when the HGSNAT is fused to an antibody that crosses the BBB such as HIR Ab (e.g., by a covalent linker), this enzyme is now able to enter the CNS from blood following a non-invasive peripheral route of administration such as intravenous, intra-arterial, intramuscular, subcutaneous, intraperitoneal, or even oral administration. Administration of a HIR Ab-HGSNAT fusion antibody enables delivery of HGSNAT activity into the brain from peripheral blood. Described herein is the determination of a systemic dose of the HIR Ab-HGSNAT fusion antibody that is therapeutically effective for treating an HGSNAT deficiency in the CNS. As described herein, appropriate systemic doses of an HIR Ab-HGSNAT fusion antibody are established based on a quantitative determination of CNS uptake characteristics and enzymatic activity of an HIR Ab-enzyme fusion antibody.

As used herein, HGSNAT (e.g., the human HGSNAT sequence listed under GenBank Accession No. NP_689632) refers to any naturally occurring or artificial enzyme that can catalyze acetylation of glucosamine residues of heparan sulphate.

In some embodiments, HGSNAT has an amino acid sequence that is at least 50% identical (i.e., at least, 55, 60, 65, 70, 75, 80, 85, 90, 95, or any other percent up to 100% identical) to the amino acid sequence of human HGSNAT, a protein listed under Genbank NP_689632. In some embodiments, HGSNAT has an amino acid sequence at least 50% identical (i.e., at least, 55, 60, 65, 70, 75, 80, 85, 90, 95, or any other percent up to 100% identical) to SEQ ID NO:19. Sequence variants of a canonical HGSNAT sequence such as SEQ ID NO:19 can be generated, e.g., by random mutagenesis of the entire sequence or specific subsequences corresponding to particular domains. Alternatively, site directed mutagenesis can be performed reiteratively while avoiding mutations to residues known to be critical to HGSNAT function such as those given above. Further, in generating multiple variants of an HGSNAT sequence, mutation tolerance prediction programs can be used to greatly reduce the number of non-functional sequence variants that would be generated by strictly random mutagenesis. Various programs for predicting the effects of amino acid substitutions in a protein sequence on protein function (e.g., SIFT, PolyPhen, PANTHER PSEC, PMUT, and TopoSNP) are described in, e.g., Henikoff et al. (2006), "Predicting the Effects of Amino Acid Substitutions on Protein Function," Annu. Rev. Genomics Hum. Genet., 7:61-80. HGSNAT sequence variants can be screened for of HGSNAT activity/retention of HGSNAT activity by a fluorometric enzymatic assay known in the art. Accordingly, one of ordinary skill in the art will appreciate that a very large number of operable HGSNAT sequence variants can be obtained by generating and screening extremely diverse "libraries" of HGSNAT sequence variants by methods that are routine in the art, as described above.

### N-acetylglucosamine-6-sulfatase (GNS)

Systemic administration (e.g., by intravenous injection) of recombinant GNS is not expected to rescue a deficiency of GNS in the CNS of patients suffering from MPS-IIID. GNS does not cross the BBB, and the lack of transport of the enzyme across the BBB prevents it from having a significant therapeutic effect in the CNS following peripheral administration. However, present inventors have discovered that when the GNS is fused to an antibody that crosses the BBB such as HIR Ab (e.g., by a covalent linker), this enzyme is now able to enter the CNS from blood following a non-invasive peripheral route of administration such as intravenous, intra-arterial, intramuscular, subcutaneous, intraperitoneal, or even oral administration. Administration of a HIR Ab-GNS fusion antibody enables delivery of GNS activity into the brain from peripheral blood. Described herein is the determination of a systemic dose of the HIR Ab-GNS fusion antibody that is therapeutically effective for treating an GNS deficiency in the CNS. As described herein, appropriate systemic doses of an HIR Ab-GNS fusion antibody are established based on a quantitative determination of CNS uptake characteristics and enzymatic activity of an HIR Ab-enzyme fusion antibody.

As used herein, GNS (e.g., the human GNS sequence listed under GenBank Accession No. NP_002067) refers to any naturally occurring or artificial enzyme that can catalyze hydrolysis of the 6-sulfate groups of heparan sulfate.

In some embodiments, GNS has an amino acid sequence that is at least 50% identical (i.e., at least, 55, 60, 65, 70, 75, 80, 85, 90, 95, or any other percent up to 100% identical) to the amino acid sequence of human GNS, a protein listed under Genbank NP_002067. In some embodiments, GNS has an amino acid sequence at least 50% identical (i.e., at least, 55, 60, 65, 70, 75, 80, 85, 90, 95, or any other percent up to 100% identical) to SEQ ID NO:21. Sequence variants of a canonical GNS sequence such as SEQ ID NO:21 can be generated, e.g., by random mutagenesis of the entire sequence or specific subsequences corresponding to particular domains. Alternatively, site directed mutagenesis can be performed reiteratively while avoiding mutations to residues known to be critical to GNS function such as those given above. Further, in generating multiple variants of an GNS sequence, mutation tolerance prediction programs can be used to greatly reduce the number of non-functional sequence variants that would be generated by strictly random mutagenesis. Various programs for predicting the effects of amino acid substitutions in a protein sequence on protein function (e.g., SIFT, PolyPhen, PANTHER PSEC, PMUT, and TopoSNP) are described in, e.g., Henikoff et al. (2006), "Predicting the Effects of Amino Acid Substitutions on Protein Function," Annu. Rev. Genomics Hum. Genet., 7:61-80. GNS sequence variants can be screened for of GNS activity/retention of GNS activity by a fluorometric enzymatic assay known in the art. Accordingly, one of ordinary skill in the art will appreciate that a very large number of operable GNS sequence variants can be obtained by generating and screening extremely diverse "libraries" of GNS sequence variants by methods that are routine in the art, as described above.

### Compositions

It has been found that the bifunctional fusion antibodies described herein, retain a high proportion of the activity of their separate constituent proteins, e.g., binding of the antibody capable of crossing the BBB (e.g., HIR Ab) to the extracellular domain of an endogenous receptor on the BBB (e.g., IR ECD), and the enzymatic activity of an enzyme deficient in MPS-III (e.g., SGSH). Construction of cDNAs and expression vectors encoding any of the proteins described herein, as well as their expression and purification are well within those of ordinary skill in the art, and are described in detail herein in, e.g., Examples 1-3, and, in Boado et al.(2007), Biotechnol Bioeng 96:381-391, U.S. Patent Application Serial No. 11/061,956, and U.S. Patent Application Serial No. 11/245,710.

Described herein are bifunctional fusion antibodies containing an antibody to an endogenous BBB receptor (e.g., HIR Ab), as described herein, capable of crossing the BBB fused to SGSH, where the antibody to the endogenous BBB receptor is capable of crossing the blood brain barrier and the SGSH each retain an average of at least about 10, 20, 30, 40, 50, 60, 70, 80, 90, 95, 99, or 100% of their activities, compared to their activities as separate entities. In some embodiments, provided herein is a HIR Ab-SGSH fusion antibody where the HIR Ab and SGSH each retain an average of at least about 50% of their activities, compared to their activities as separate entities. In some embodiments, provided herein is a HIR Ab-SGSH fusion antibody where the HIR Ab and SGSH each retain an average of at least about 60% of their activities, compared to their activities as separate entities. In some embodiments, provided herein is a HIR Ab-SGSH fusion antibody where the HIR Ab and SGSH each retain an average of at least about 70% of their activities, compared to their activities as separate entities. In some embodiments, provided herein is a HIR Ab-SGSH fusion antibody where the HIR Ab and SGSH each retain an average of at least about 80% of their activities, compared to their activities as separate entities. In some embodiments, provided herein is a fusion HIR Ab-SGSH fusion antibody where the HIR Ab and SGSH each retain an average of at least about 90% of their activities, compared to their activities as separate entities. In some embodiments, the HIR Ab retains at least about 10, 20, 30, 40, 50, 60, 70, 80, 90, 95, 99, or 100% of its activity, compared to its activity as a separate entity, and the SGSH retains at least about 10, 20, 30, 40, 50, 60, 70, 80, 90, 95, 99, or 100% of its activity, compared to its activity as a separate entity. Accordingly, described herein are compositions containing a bifunctional HIR Ab-SGSH fusion antibody capable of crossing the BBB, where the constituent HIR Ab and SGSH each retain, as part of the fusion antibody, an average of at least about 10, 20, 30, 40, 50, 60, 70, 80, 90, 95, 99, or 100% of their activities, i.e., HIR binding and SGSH activity, respectively, compared to their activities as separate proteins. An HIR Ab SGSH fusion antibody refers to a fusion protein comprising any of the HIR antibodies and SGSH described herein.

In any of the embodiments provided herein, HIR Ab may be replaced by an antibody to an endogenous BBB receptor described herein, such as an antibody to transferrin receptor, leptin receptor, lipoprotein receptor, or the insulin-like growth factor (IGF) receptor, or other similar endogenous BBB receptor-mediated transport system.

In the fusion antibodies described herein, the covalent linkage between the antibody and the SGSH may be to the carboxy or amino terminal of the antibody heavy or light chain and the amino or carboxy terminal of the SGSH as long as the linkage allows the fusion antibody to bind to the ECD of the IR and cross the blood brain barrier, and allows the SGSH to retain a therapeutically useful portion of its activity. In certain embodiments, the covalent link is between an HC of the antibody and the SGSH or a LC of the antibody and the SGSH. Any suitable linkage may be used, e.g., carboxy terminus of light chain to amino terminus of SGSH, carboxy terminus of heavy chain to amino terminus of SGSH, amino terminus of light chain to amino terminus of SGSH, amino terminus of heavy chain to amino terminus of SGSH, carboxy terminus of light chain to carboxy terminus of SGSH, carboxy terminus of heavy chain to carboxy terminus of SGSH, amino terminus of light chain to carboxy terminus of SGSH, or amino terminus of heavy chain to carboxy terminus of SGSH. In some embodiments, the linkage is from the carboxy terminus of the HC to the amino terminus of the SGSH.

The SGSH may be fused, or covalently linked, to the targeting antibody (e.g., MAb, HIR-MAb) through a linker. A linkage between terminal amino acids can be accomplished by an intervening peptide linker sequence that forms part of the fused amino acid sequence. The peptide sequence linker may be 0, 1, 2, 3, 4, 5, 6, 7, 8, 9, 10, or more than 10 amino acids in length. In some embodiments, including some preferred embodiments, the peptide linker is less than 20, 15, 14, 13, 12, 11, 10, 9, 8, 7, 6, 5, 4, 3, 2, or 1 amino acids in length. In some embodiments, including some preferred embodiments, the peptide linker is at least 0, 1, 2, 3, 4, 5, 6, 7, 8, 9, 10 amino acids in length. In some embodiments, the SGSH is directly linked to the targeting antibody, and is therefore 0 amino acids in length. In some embodiments, there is no linker linking the SGSH to the targeting antibody.

In some embodiments, the linker comprises glycine, serine, and/or alanine residues in any combination or order. In some cases, the combined percentage of glycine, serine, and alanine residues in the linker is at least 5%, 10%, 15%, 20%, 25%, 30%, 40%, 50%, 60%, 70%, 75%, 80%, 90%, or 95% of the total number of residues in the linker. In some preferred embodiments, the combined percentage of glycine, serine, and alanine residues in the linker is at least 50%, 60%, 70%, 75%, 80%, 90%, or 95% of the total number of residues in the linker. In some embodiments, any number of combinations of amino acids (including natural or synthetic amino acids) can be used for the linker. In some embodiments, a three amino acid linker is used. In some embodiments, the linker has the sequence Ser-Ser-Ser. In some embodiments, a two amino acid linker comprises glycine, serine, and/or alanine residues in any combination or order (e.g., Gly-Gly, Ser-Gly, Gly-Ser, Ser-Ser. Ala-Ala, Ser-Ala, or Ala-Ser linker). In some embodiments, a two amino acid linker consists of one glycine, serine, and/or alanine residue along with another amino acid (e.g., Ser-X, where X is any known amino acid). In still other embodiments, the two-amino acid linker consists of any two amino acids (e.g., X-X), exept gly, ser, or ala.

As described herein, in some embodiments a linker that is greater than two amino acids in length. Such linker may also comprise glycine, serine, and/or alanine residues in any combination or order, as described further herein. In some embodiments, the linker consists of one glycine, serine, and/or alanine residue along with other amino acids (e.g., Ser-nX, where X is any known amino acid, and n is the number of amino acids). In still other embodiments, the linker consists of any two amino acids (e.g., X-X). In some embodiments, said any two amino acids are Gly, Ser, or Ala, in any combination or order, and within a variable number of amino acids intervening between them. In an example of an embodiment, the linker consists of at least one Gly. In an example of an embodiment, the linker consists of at least one Ser. In an example of an embodiment, the linker consists of at least one Ala. In some embodiments, the linker consists of at least 1, 2, 3, 4, 5, 6, 7, 8, 9, or 10 Gly, Ser, and/or Ala residues. In preferred embodiments, the linker comprises Gly and Ser in repeating sequences, in any combination or number, such as (Gly₄Ser)₃, or other variations.

A linker for use in the present embodiments may be designed by using any method known in the art. For example, there are multiple publicly-available programs for determining optimal amino acid linkers in the engineering of fusion proteins. Publicly-available computer programs (such as the LINKER program) that automatically generate the amino acid sequence of optimal linkers based on the user's input of the sequence of the protein and the desired length of the linker may be used for the present methods and compositions. Often, such programs may use observed trends of naturally-occurring linkers joining protein subdomains to predict optimal protein linkers for use in protein engineering. In some cases, such programs use other methods of predicting optimal linkers. Examples of some programs suitable for predicting a linker for the present embodiments are described in the art, see, e.g., Xue et al. (2004) Nucleic Acids Res. 32, W562-W565 (Web Server issue providing internet link to LINKER program to assist the design of linker sequences for constructing functional fusion proteins) ; George and Heringa, (2003), Protein Engineering, 15(11):871-879 (providing an internet link to a linker program and describing the rational design of protein linkers); Argos, (1990), J. Mol. Biol. 211:943-958; Arai et al. (2001) Protein Engineering, 14(8):529-532; Crasto and Feng, (2000) Protein Engineering 13(5):309-312.

The peptide linker sequence may include a protease cleavage site, however this is not a requirement for activity of the SGSH; indeed, an advantage of these embodiments is that the bifunctional HIR Ab-SGSH fusion antibody, without cleavage, is partially or fully active both for transport and for activity once across the BBB. Fig. 9 shows an exemplary embodiment of the amino acid sequence of a HIR Ab-SGSH fusion antibody (SEQ ID NO:10) in which the HC is fused through its carboxy terminus via a three amino acid "ser-ser-ser" linker to the amino terminus of the SGSH. In some embodiments, the fused SGSH sequence is devoid of its 20 amino acid signal peptide, as shown in Fig. 8.

In some embodiments, a HIR Ab-SGSH fusion antibody provided herein comprises both a HC and a LC. In some embodiments, the HIR Ab-SGSH fusion antibody is a monovalent antibody. In other embodiments, the HIR Ab-SGSH fusion antibody is a divalent antibody, as described herein in the Example section.

In some embodiments, the HIR Ab used as part of the HIR Ab-SGSH fusion antibody can be glycosylated or nonglycosylated; in some embodiments, the antibody is glycosylated, e.g., in a glycosylation pattern produced by its synthesis in a CHO cell.

As used herein, "activity" includes physiological activity (e.g., ability to cross the BBB and/or therapeutic activity), binding affinity of the HIR Ab for the IR ECD, or the enzymatic activity of SGSH.

Transport of a HIR Ab-SGSH fusion antibody across the BBB may be compared to transport across the BBB of the HIR Ab alone by standard methods. For example, pharmacokinetics and brain uptake of the HIR Ab-SGSH fusion antibody by a model animal, e.g., a mammal such as a primate, may be used. Similarly, standard models for determining SGSH activity may also be used to compare the function of the SGSH alone and as part of a HIR Ab-SGSH fusion antibody. See, e.g., Example 4, which demonstrates the enzymatic activity of SGSH versus HIR Ab-SGSH fusion antibody. Binding affinity for the IR ECD can be compared for the HIR Ab-SGSH fusion antibody versus the HIR Ab alone. See, e.g., Example 4 herein.

Also included herein are pharmaceutical compositions that contain one or more HIR Ab-SGSH fusion antibodies described herein and a pharmaceutically acceptable excipient. A thorough discussion of pharmaceutically acceptable carriers/excipients can be found in Remington's Pharmaceutical Sciences, Gennaro, AR, ed., 20th edition, 2000: Williams and Wilkins PA, USA. Pharmaceutical compositions of the present embodiments include compositions suitable for administration via any peripheral route, including intravenous, subcutaneous, intramuscular, intraperitoneal injection; oral, rectal, transbuccal, pulmonary, transdermal, intranasal, or any other suitable route of peripheral administration.

The compositions provided herein are particular suited for injection, e.g., as a pharmaceutical composition for intravenous, subcutaneous, intramuscular, or intraperitoneal administration. Aqueous compositions provided herein comprise an effective amount of a composition of the present embodiments, which may be dissolved or dispersed in a pharmaceutically acceptable carrier or aqueous medium. The phrases "pharmaceutically or pharmacologically acceptable" refer to molecular entities and compositions that do not produce an adverse, allergic or other untoward reaction when administered to an animal, e.g., a human, as appropriate. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

Exemplary pharmaceutically acceptable carriers for injectable compositions can include salts, for example, mineral acid salts such as hydrochlorides, hydrobromides, phosphates, sulfates, and the like; and the salts of organic acids such as acetates, propionates, malonates, benzoates, and the like. For example, compositions provided herein may be provided in liquid form, and formulated in saline based aqueous solution of varying pH (5-8), with or without detergents such polysorbate-80 at 0.01-1%, or carbohydrate additives, such mannitol, sorbitol, or trehalose. Commonly used buffers include histidine, acetate, phosphate, or citrate. In some embodiments, the pharmaceucial compositions provided herein include a monosaccharide such as glucose or dextrose. For example, the fusion antibody can be administered with a solution of dextrose about 0.1%, about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20% or more of dextrose and/or glucose. In some embodiements, the composition can comprise glucose and/or dextrose at a concentration of about 5% (w/v or v/v). For exampleIf plasma and CSF glucose levels change significantly to indicate hypoglycemia, Under ordinary conditions of storage and use, these preparations can contain a preservative to prevent the growth of microorganisms. The prevention of the action of microorganisms can be brought about by various antibacterial and antifungal agents, for example, parabens, chlorobutanol; phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate, and gelatin.

For human administration, preparations meet sterility, pyrogenicity, general safety, and purity standards as required by FDA and other regulatory agency standards. The active compounds will generally be formulated for parenteral administration, e.g., formulated for injection via the intravenous, intramuscular, subcutaneous, intralesional, or intraperitoneal routes. The preparation of an aqueous composition that contains an active component or ingredient will be known to those of skill in the art in light of the present disclosure. Typically, such compositions can be prepared as injectables, either as liquid solutions or suspensions; solid forms suitable for use in preparing solutions or suspensions upon the addition of a liquid prior to injection can also be prepared; and the preparations can also be emulsified.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, methods of preparation include vacuum-drying and freeze-drying techniques which yield a powder of the active ingredient plus any additional desired ingredient from a previously sterile-filtered solution thereof.

Upon formulation, solutions will be systemically administered in a manner compatible with the dosage formulation and in such amount as is therapeutically effective based on the criteria described herein. The formulations are easily administered in a variety of dosage forms, such as the type of injectable solutions described above, but drug release capsules and the like can also be employed

The appropriate quantity of a pharmaceutical composition to be administered, the number of treatments, and unit dose will vary according to the CNS uptake characteristics of a HIR Ab-SGSH fusion antibody as described herein, and according to the subject to be treated, the state of the subject and the effect desired. The person responsible for administration will, in any event, determine the appropriate dose for the individual subject.

In addition to the compounds formulated for parenteral administration, such as intravenous or intramuscular injection, other alternative methods of administration of the present embodiments may also be used, including but not limited to intradermal administration (See U.S. Pat. Nos. 5,997,501; 5,848,991; and 5,527,288), pulmonary administration (See U.S. Pat. Nos. 6,361,760; 6,060,069; and 6,041,775), buccal administration (See U.S. Pat. Nos. 6,375,975; and 6,284,262), transdermal administration (See U.S. Pat. Nos. 6,348,210; and 6,322,808) and transmucosal administration (See U.S. Pat. No. 5,656,284). Such methods of administration are well known in the art. One may also use intranasal administration of the present embodiments, such as with nasal solutions or sprays, aerosols or inhalants. Nasal solutions are usually aqueous solutions designed to be administered to the nasal passages in drops or sprays. Nasal solutions are prepared so that they are similar in many respects to nasal secretions. Thus, the aqueous nasal solutions usually are isotonic and slightly buffered to maintain a pH of 5.5 to 6.5. In addition, antimicrobial preservatives, similar to those used in ophthalmic preparations and appropriate drug stabilizers, if required, may be included in the formulation. Various commercial nasal preparations are known and include, for example, antibiotics and antihistamines and are used for asthma prophylaxis.

Additional formulations, which are suitable for other modes of administration, include suppositories and pessaries. A rectal pessary or suppository may also be used. Suppositories are solid dosage forms of various weights and shapes, usually medicated, for insertion into the rectum or the urethra. After insertion, suppositories soften, melt or dissolve in the cavity fluids. For suppositories, traditional binders and carriers generally include, for example, polyalkylene glycols or triglycerides; such suppositories may be formed from mixtures containing the active ingredient in any suitable range, e.g., in the range of 0.5% to 10%, preferably 1%-2%.

Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations, or powders. In certain defined embodiments, oral pharmaceutical compositions will comprise an inert diluent or assimilable edible carrier, or they may be enclosed in a hard or soft shell gelatin capsule, or they may be compressed into tablets, or they may be incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations can contain at least 0.1% of active compound. The percentage of the compositions and preparations may, of course, be varied, and may conveniently be between about 2 to about 75% of the weight of the unit, or between about 25-60%. The amount of active compounds in such therapeutically useful compositions is such that a suitable dosage will be obtained.

The tablets, troches, pills, capsules and the like may also contain the following: a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent, methylene and propyl parabens as preservatives, a dye and flavoring, such as cherry or orange flavor. In some embodiments, an oral pharmaceutical composition may be enterically coated to protect the active ingredients from the environment of the stomach; enteric coating methods and formulations are well-known in the art.

### Methods

Described herein are methods for delivering an effective dose of an enzyme deficient in MPS-III (e.g., SGSH) to the CNS across the BBB by systemically administering a therapeutically effective amount of a fusion antibody, as described herein. In some embodiments, the fusion antibody provided herein is a HIR Ab-SGSH. Suitable systemic doses for delivery of a HIR Ab-SGSH fusion antibody is based on its CNS uptake characteristics and SGSH specific activity as described herein. Systemic administration of a HIR Ab-SGSH fusion antibody to a subject suffering from an SGSH deficiency is an effective approach to the non-invasive delivery of SGSH to the CNS. In addition, described herein are methods for treating (e.g., therapeutically treating) MPS-III in a subject by systemically administering a therapeutically effective amount of a fusion antibody, as described herein. In some embodiments, the treatment is a therapeutic treatment. In some embodiments, the treatment alleviates one or more symptoms of MPS-III. In some embodiments, the treatment alleviates one or more symptoms of MPS-III by about 5%, 10%, 15%, 20%, 25%, 30%, 40%, 50%, 60%, 70%, 75%, 80%, 90%, 95%, 99%, or 100%.

The amount of a fusion antibody that is a therapeutically effective systemic dose of a fusion antibody depends, in part, on the CNS uptake characteristics of the fusion antibody to be administered, as described herein., e.g., the percentage of the systemically administered dose to be taken up in the CNS.

In some embodiments, 1% (i.e., about 0.3%, 0.4%, 0.48%, 0.6%, 0.74%, 0.8%, 0.9%, 1.05, 1.1, 1.2, 1.3%, 1.5%, 2%, 2.5%, 3%, or any % from about 0.3% to about 3%) of the systemically administered HIR Ab-SGSH fusion antibody is delivered to the brain as a result of its uptake from peripheral blood across the BBB. In some embodiments, at least 0.5%, (i.e., about 0.3%, 0.4%, 0.48%, 0.6%, 0.74%, 0.8%, 0.9%, 1.05, 1.1, 1.2, 1.3%, 1.5%, 2%, 2.5%, 3%, or any % from about 0.3% to about 3%) of the systemically administered dose of the HIR Ab-SGSH fusion antibody is delivered to the brain within two hours or less, i.e., 1.8, 1.7, 1.5, 1.4, 1.3, 1.2, 1.1, 0.9, 0.8, 0.6, 0.5 or any other period from about 0.5 to about two hours after systemic administration.

Accordingly, in some embodiments provided herein are methods of administering a therapeutically effective amount of a fusion antibody described herein systemically, to a 5 to 50 kg human, such that the amount of the fusion antibody to cross the BBB provides at least 0.5 ng of SGSH protein/mg protein in the subject's brain, e.g., 0.5, 1, 3, 10, 30, or 50 or any other value from 0.5 to 50 ng of SGSH protein/mg protein in the subject's brain.

In some embodiments, the total number of units of enzyme (e.g., SGSH) activity delivered to a subject's brain is at least, 50 milliunits per gram brain, e.g., at least 100, 300, 1000, 3000, 10000, 30000, or 50000 or any other total number of SGSH units from about 50 to 50,000 milliunits of SGSH activity delivered per gram brain.

In some embodiments, a therapeutically effective systemic dose comprises at least 5000, 10000, 30000, 100000, 300000, 1000000, 5000000 or any other systemic dose from about 5,000 to 5,000,000 units of enzyme (e.g., SGSH) activity.

In other embodiments, a therapeutically effective systemic dose is at least about 1000 units of enzyme (e.g., SGSH) activity/kg body weight, at least about 1000, 3000, 10000, 30000, 100000 or any other number of units from about 1,000 to 100,000 units of enzyme activity/kg of body weight.

One of ordinary skill in the art will appreciate that the mass amount of a therapeutically effective systemic dose of a fusion antibody provided herein will depend, in part, on its enzyme (e.g., SGSH) specific activity. In some embodiments, the specific activity of a fusion antibody is at least 1,000 U/mg of protein, at least about 1500, 2500, 3500, 6000, 7500, 9000 or any other specific activity value from about 1,000 units/mg to about 10,000 units/mg.

Thus, with due consideration of the specific activity of a fusion antibody provided herein and the body weight of a subject to be treated, a systemic dose of the fusion antibody can be at least 5 mg, e.g., 5, 10, 15, 20, 25, 30, 35, 40, 45, 50, 55, 60, 65, 70, 75, 100, 300, or any other value from about 5 mg to about 500 mg of fusion antibody (e.g., HIR Ab-SGSH).

The term "systemic administration" or "peripheral administration," as used herein, includes any method of administration that is not direct administration into the CNS, i.e., that does not involve physical penetration or disruption of the BBB. "Systemic administration" includes, but is not limited to, intravenous , intra-arterial intramuscular, subcutaneous, intraperitoneal, intranasal, transbuccal, transdermal, rectal, transalveolar (inhalation), or oral administration. Any suitable fusion antibody, as described herein, may be used.

An SGSH deficiency as referred to herein includes, one or more conditions known as Sanfilippo syndrome type A, or MPS-IIIA. SGSH deficiency is characterized by the buildup of heparan sulfate that occurs in the brain and other organs.

The compositions provided herein, e.g., an HIR Ab-SGSH fusion antibody, may be administered as part of a combination therapy. The combination therapy involves the administration of a composition of the present embodiments in combination with another therapy for treatment or relief of symptoms typically found in a patient suffering from an SGSH deficiency. If the composition of the present embodiments is used in combination with another CNS disorder method or composition, any combination of the composition of the present embodiments and the additional method or composition may be used. Thus, for example, if use of a composition of the present embodiments is in combination with another CNS disorder treatment agent, the two may be administered simultaneously, consecutively, in overlapping durations, in similar, the same, or different frequencies, etc. In some cases a composition will be used that contains a composition of the present embodiments in combination with one or more other CNS disorder treatment agents.

In some embodiments, the composition, e.g., an HIR Ab-SGSH fusion antibody is co-administered to the patient with another medication, either within the same formulation or as a separate composition. For example, the fusion antibody provided herein may be formulated with another fusion protein that is also designed to deliver across the human blood-brain barrier a recombinant protein other than SGSH. Further, the fusion antibody may be formulated in combination with other large or small molecules.

### EXAMPLES

The following specific examples are to be construed as merely illustrative, and not limitative of the remainder of the disclosure in any way whatsoever. Without further elaboration, it is believed that one skilled in the art can, based on the description herein, utilize the present embodiments to its fullest extent. All publications cited herein are hereby incorporated by reference in their entirety. Where reference is made to a URL or other such identifier or address, it is understood that such identifiers can change and particular information on the internet can come and go, but equivalent information can be found by searching the internet Reference thereto evidences the availability and public dissemination of such information.

### Example 1. Expression and functional analysis of HIR Ab-GUSB fusion protein

The lysosomal enzyme mutated in MPS-VII, also called Sly syndrome, is β-glucuronidase (GUSB). MPS-VII results in accumulation of glycosoaminoglycans in the brain. Enzyme replacement therapy (ERT) of MPS-VII would not likely be effective for treatment of the brain because the GUSB enzyme does not cross the BBB. In an effort to re-engineer human GUSB to cross the BBB, a HIR Ab-GUSB fusion protein project was initiated.

Human GUSB cDNA corresponding to amino acids Met₁-Thr₆₅₁ of the human GUSB protein (NP_000172), including the 22 amino acid signal peptide, and the 18 amino acid carboxyl terminal propeptide, was cloned by reverse transcription (RT) polymerase chain reaction (PCR) and custom oligodexoynucleotides (ODNs). PCR products were resolved in 1% agarose gel electrophoresis, and the expected major single band of ∼2.0 kb corresponding to the human GUSB cDNA was isolated. The cloned human GUSB was inserted into a eukaryotic expression plasmid, and this GUSB expression plasmid was designated pCD-GUSB. The entire expression cassette of the plasmid was confirmed by bidirectional DNA sequencing. Transfection of COS cells in a 6-well format with the pCD-GSUB resulted in high GUSB enzyme activity in the conditioned medium at 7 days (Table 1, Experiment A), which validated the successful engineering of a functional human GUSB cDNA. The GUSB enzyme activity was determined with a fluorometric assay using 4-methylumbelliferyl beta-L-glucuronide (MUGlcU), which is commercially available. This substrate is hydolyzed to 4-methylumbelliferone (4-MU) by GUSB, and the 4-MU is detected fluorometrically with a fluorometer using an emission wavelength of 450 nm and an excitation wavelength of 365 nm. A standard curve was constructed with known amounts of 4-MU. The assay was performed at 37C with 60 min incubations at pH=4.8, and was terminated by the addition of glycine-carbonate buffer (pH=10.5).

A new pCD-HC-GUSB plasmid expression plasmid was engineered, which expresses the fusion protein wherein the carboxyl terminus of the heavy chain (HC) of the HIR Ab is fused to the amino terminus of human GUSB, minus the 22 amino acid GUSB signal peptide, and minus the 18 amino acid carboxyl terminal GUSB propeptide. The GUSB cDNA was cloned by PCR using the pCD-GUSB as template. The forward PCR primer introduces "CA" nucleotides to maintain the open reading frame and to introduce a Ser-Ser linker between the carboxyl terminus of the CH3 region of the HIR Ab HC and the amino terminus of the GUSB minus the 22 amino acid signal peptide of the enzyme. The GUSB reverse PCR primer introduces a stop codon, "TGA," immediately after the terminal Thr of the mature human GUSB protein. DNA sequencing of the expression cassette of the pCD-HC-GUSB encompassed 4,321 nucleotides (nt), including a 714 nt cytomegalovirus (CMV) promoter, a 9 nt Kozak site (GCCGCCACC), a 3,228 nt HC-GUSB fusion protein open reading frame, and a 370 nt bovine growth hormone (BGH) transcription termination sequence. The plasmid encoded for a 1,075 amino acid protein, comprised of a 19 amino acid IgG signal peptide, the 443 amino acid HIRMAb HC, a 2 amino acid linker (Ser-Ser), and the 611 amino acid human GUSB minus the enzyme signal peptide and carboxyl terminal propeptide. The GUSB sequence was 100% identical to Leu²³-Thr⁶³³ of human GUSB (NP_000172). The predicted molecular weight of the heavy chain fusion protein, minus glycosylation, is 119,306 Da, with a predicted isoelectric point (pI) of 7.83.

COS cells were plated in 6-well cluster dishes, and were dual transfected with pCD-LC and pCD-HC-GUSB, where pCD-LC is the expression plasmid encoding the light chain (LC) of the chimeric HIR Ab. Transfection was performed using Lipofectamine 2000, with a ratio of 1:2.5, ug DNA:uL Lipofectamine 2000, and conditioned serum free medium was collected at 3 and 7 days. However, there was no specific increase in GUSB enzyme activity following dual transfection of COS cells with the pCD-HC-GUSB and pCD-LC expression plasmids (Table 1, Experiment B). However, the low GUSB activity in the medium could be attributed to the low secretion of the HIRMAb-GUSB fusion protein, as the medium IgG was only 23 ± 2 ng/mL, as determined by a human IgG-specific ELISA. Therefore, COS cell transfection was scaled up to 10xT500 plates, and the HIRMAb-GUSB fusion protein was purified by protein A affinity chromatography. IgG Western blotting demonstrated the expected increase in size of the fusion protein heavy chain. However, the GUSB enzyme activity of the HIRMAb-GUSB fusion protein was low at 6.1 ± 0.1 nmol/hr/ug protein. In contrast, the specific activity of human recombinant GUSB is 2,000 nmol/hr/ug protein [Sands et al.(1994) Enzyme replacement therapy for murine mucopolysaccharidosis type VII. J Clin Invest 93, 2324-2331]. These results demonstrated the GUSB enzyme activity of the HIR Ab-GUSB fusion protein was >95% lost following fusion of the GUSB to the carboxyl terminus of the HC of the HIR Ab. The affinity of HIR Ab-GUSB fusion protein binding to the extracellular domain (ECD) of the HIR was examined with an ELISA. CHO cells permanently transfected with the HIR ECD were grown in serum free media (SFM), and the HIR ECD was purified with a wheat germ agglutinin affinity column. The HIR ECD was plated on 96-well dishes and the binding of the HIR Ab, and the HIR Ab-GUSB fusion protein to the HIR ECD was detected with a biotinylated goat anti-human IgG (H+L) secondary antibody, followed by avidin and biotinylated peroxidase. The concentration of protein that gave 50% maximal binding, ED₅₀, was determined with a non-linear regression analysis. The HIR receptor assay showed there was no decrease in affinity for the HIR following fusion of the 611 amino acid GUSB to the carboxyl terminus of the HIRMAb heavy chain. The ED50 of the HIR Ab binding to the HIR ECD was 0.77 ± 0.10 nM and the ED50 of binding of the HIR Ab-GUSB fusion protein was 0.81 ± 0.04 nM.

In summary, fusion of the GUSB to the carboxyl terminus of the HIR Ab HC resulted in no loss in affinity of binding of the fusion protein to the HIR. However, the GUSB enzyme activity of the fusion protein was decreased by >95%.

In an effort to successfully produce a fusion protein of the HIR Ab and GUSB, a new approach was undertaken, in which the carboxyl terminus of the mature human GUSB, including the GUSB signal peptide, was fused to the amino terminus of the HC of the HIR Ab. This fusion protein was designated GUSB-HIR Ab. The first step was to engineer a new expression plasmid encoding this new fusion protein, and this plasmid was designated pCD-GUSB-HC. The pCD-GUSB-HC plasmid expresses the fusion protein wherein the amino terminus of the heavy chain (HC) of the HIRMAb, minus its 19 amino acid signal peptide, is fused to the carboxyl terminus of human GUSB, including the 22 amino acid GUSB signal peptide, but minus the 18 amino acid carboxyl terminal GUSB propeptide. The pCD-GUSB vector was used as template for PCR amplification of the GUSB cDNA expressing a GUSB protein that contained the 22 amino acid GUSB signal peptide, but lacking the 18 amino acid propeptide at the GUSB carboxyl terminus. The GUSB 18 amino acid carboxyl terminal propeptide in pCD-GUSB was deleted by site-directed mutagenesis (SDM). The latter created an AfeI site on the 3'-flanking region of the Thr⁶³³ residue of GUSB, and it was designated pCD-GUSB-AfeI. The carboxyl terminal propeptide was then deleted with AfeI and HindIII (located on the 3'-non coding region of GUSB). The HIRMAb HC open reading frame, minus the 19 amino acid IgG signal peptide and including the HIRMAb HC stop codon, was generated by PCR using the HIRMAb HC cDNA as template. The PCR generated HIRMAb HC cDNA was inserted at the AfeI-HindIII sites of pCD-GUSB-AfeI to form the pCD-GUSB-HC. A Ser-Ser linker between the carboxyl terminus of GUSB and amino terminus of the HIRMAb HC was introduced within the AfeI site by the PCR primer used for the cloning of the HIRMAb HC cDNA. DNA sequencing of the pCD-GUSB-HC expression cassette showed the plasmid expressed 1,078 amino acid protein, comprised of a 22 amino acid GUSB signal peptide, the 611 amino acid GUSB, a 2 amino acid linker (Ser-Ser), and the 443 amino acid HIRMAb HC. The GUSB sequence was 100% identical to Met¹-Thr⁶³³ of human GUSB (NP_000172).

Dual transfection of COS cells in a 6-well format with the pCD-LC and pCD-GUSB-HC expression plasmids resulted in higher GUSB enzyme activity in the conditioned medium at 7 days, as compared to dual transfection with the pCD-LC and pCD-HC-GUSB plasmids (Table 1, Experiment C). However, the GUSB-HIRMAb fusion protein was also secreted poorly by the COS cells, as the medium human IgG concentration in the 7 day conditioned medium was only 13 ± 2 ng/mL, as determined by ELISA. COS cell transfection was scaled up to 10xT500 plates, and the GUSB-HIRMAb fusion protein was purified by protein A affinity chromatography. SDS-PAGE demonstrated the expected increase in size of the fusion protein heavy chain. The GUSB enzyme activity of the purified GUSB-HIRMAb fusion protein was high at 226 ± 8 nmol/hr/ug protein, which is 37-fold higher than the specific GUSB enzyme activity of the HIRMAb-GUSB fusion protein. However, the HIR receptor assay showed there was a marked decrease in affinity for the HIR following fusion of the GUSB to the amino terminus of the HIRMAb heavy chain, which resulted in a 95% reduction in receptor binding affinity. The ED50 of the HIR Ab binding to the HIR ECD was 0.25 ± 0.03 nM and the ED50 of binding of the HIR Ab-GUSB fusion protein was 4.8 ± 0.4 nM.

In summary, fusion of the GUSB to the amino terminus of the HIR Ab HC resulted in retention of GUSB enzyme activity of the fusion protein, but caused a 95% reduction in binding of the GUSB-HIR Ab fusion protein to the HIR. In contrast, fusion of the GUSB to the carboxyl terminus of the HIR Ab HC resulted in no loss in affinity of binding of the HIR Ab-GUSB fusion protein to the HIR. However, the GUSB enzyme activity of this fusion protein was decreased by >95%. These findings illustrate the unpredictable nature of the art of fusion of lysosomal enzymes to IgG molecules in such a way that bi-functionality of the IgG-enzyme fusion protein is retained, i.e., high affinity binding of the IgG part to the cognate antigen, as well as high enzyme activity.

**Table 1. GUSB enzyme activity in COS cells following transfection [Mean ± SE (n=3 dishes per point)]**

| **Experiment** | **Treatment** | **Medium GUSB activity (nmol/hour/mL)** |
|---|---|---|
| A | Lipofectamine 2000 | 65 ± 1 |
| | pCD-GUSB | 6892 ± 631 |
| B | Lipofectamine 2000 | 76 ± 3 |
| | pCD-HC-GUSB, pCD-LC | 72 ± 3 |
| C | Lipofectamine 2000 | 162 ± 7 |
| | pCD-HC-GUSB, pCD-LC | 155 ± 2 |
| | pCD-GUSB-HC, CD-LC | 1119 ± 54 |

### Example 2. Expression and functional analysis of HIR Ab-GCR fusion protein

The lysosomal enzyme, mutated in Gaucher's disease (GD) is β-glucocerebrosidase (GCR). Neuronopathic forms of GD affect the CNS, and this results in accumulation of lysosomal inclusion bodies in brain cells, owing to the absence of GCR enzyme activity in the brain. Enzyme replacement therapy (ERT) of GD is not an effective for treatment of the brain because the GCR enzyme does not cross the BBB. In an effort to re-engineer human GCR to cross the BBB, a HIR Ab-GCR fusion protein project was engineered, expressed, and tested for enzyme activity. The human GCR cDNA corresponding to amino acids Ala₄₀-Gln₅₃₆ of the human GCR protein (NP_000148), minus the 39 amino acid signal peptide, was custom synthesized by a commercial DNA production company. The GCB cDNA was comprised of 1522 nucleotides (nt), which included the GCB open reading frame, minus the signal peptide through the TGA stop codon. On the 5'-end, a StuI restriction endonuclease (RE) sequence was added, and on the 3'-end, a 14 nt fragment from the 3'-untranslated region of the GCR mRNA was followed by a HindIII RE site. Internal HindIII and StuI sites within the GCR gene were mutated without change of amino acid sequence. The GCR gene was released from the pUC plasmid provided by the vendor with StuI and HindIII, and was inserted at HpaI and HindIII sites of a eukaryotic expression plasmid encoding the HIR Ab heavy chain, and this expression plasmid was designated, pCD-HC-GCR. This expression plasmid expresses the fusion protein wherein the carboxyl terminus of the heavy chain (HC) of the HIR Ab is fused to the amino terminus of human GCR, minus the 39 amino acid GCR signal peptide, with a 3 amino acid linker (Ser-Ser-Ser) between the HIR Ab HC and the GCR. DNA sequencing confirmed the identity of the pCD-HC-GCR expression cassette. The expression cassette was comprised of 5,390 nt, which included a 2134 nt CMV promoter sequence, a 2,889 nt expression cassette, and a 367 BGH polyA sequence. The plasmid encoded for a 963 amino acid protein, which was comprised of a 19 amino acid IgG signal peptide, the 443 amino acid HIRMAb HC, a 3 amino acid linker (Ser-Ser-Ser), and the 497 amino acid human GCR minus the enzyme signal peptide. The GCR sequence was 100% identical to Als⁴⁰-Gln⁵³⁶ of human GCR (NP_000148). The predicted molecular weight of the heavy chain fusion protein, minus glycosylation, is 104,440 Da, with a predicted isoelectric point (pI) of 8.42.

The HIR Ab-GCR fusion protein was expressed in transiently transfected COS cells. COS cells were plated in 6-well cluster dishes, and were dual transfected with pCD-LC and pCD-HC-GCR, where pCD-LC is the expression plasmid encoding the light chain (LC) of the chimeric HIR Ab. Transfection was performed using Lipofectamine 2000, with a ratio of 1:2.5, ug DNA:uL Lipofectamine 2000, and conditioned serum free medium was collected at 3 and 7 days. Fusion protein secretion into the serum free medium (SFM) was monitored by human IgG ELISA. The conditioned medium was clarified by depth filtration, and the HIR Ab-GCR fusion protein was purified by protein A affinity chromatography. The purity of the fusion protein was confirmed by reducing SDS-PAGE, and the identity of the fusion protein was confirmed by Western blotting using primary antibodies against either human IgG or human GCR. The IgG and GCR antibodies both reacted with the 130 kDa heavy chain of the HIR Ab-GCR fusion protein.

The GCR enzyme activity of the fusion protein was measured with a fluorometric enzyme assay using 4-methylbumbelliferyl beta-D glucopyranoside (4-MUG) as the enzyme substrate as described previously for enzyme assay of recombinant GCR (J.B. Novo, et al, Generation of a Chinese hamster ovary cell line producing recombinant human glucocerebrosidase, J. Biomed. Biotechnol., Article ID 875383, 1-10, 2012). The GCR enzyme assay was performed with a final concentration of 4-MUG of 5 mM in citrate/phosphate buffer/pH=5.5 with 0.25% Triton X-100, and 0.25% sodium taurocholate, and the incubation was performed at 37C for 60 minutes. Enzyme activity was stopped by the addition of 0.1 M glycine/0.1 M NaOH. The GCR enzyme converts the 4-MUG substrate to the product, 4-methlyumbelliferone (4-MU). An assay standard curve was constructed with 4-MU (0.03 to 3 nmol/tube). Enzyme activity was reported as units/mg protein, where 1 unit = 1 umol/min. The enzyme activity of recombinant human GCR is 40 units/mg (Novo *et al,* 2012). However, the GCR enzyme activity of the HIR Ab-GCR fusion protein was only 0.07 units/mg, which is 99% reduced compared to the specific activity of recombinant GCR.

Examples 1 and 2 illustrate the unpredictability of engineering biologically active IgG-lysosomal enzyme fusion proteins. In both cases, the fusion of either GUSB or GCR to the carboxyl terminus of the heavy chain of the HIR Ab resulted in a >95% loss of enzyme activity. Described in the examples below is the surprising finding that SGSH enzyme activity is preserved following fusion to the carboxyl terminus of the heavy chain of the HIR Ab.

### Example 3. Construction of human HIR Ab heavy chain-SGSH fusion protein expression vector

The lysosomal enzyme mutated in MPS-IIIA is SGSH. MPS-IIIA results in accumulation of heparan sulfate in the brain. Enzyme replacement therapy of MPS-IIIA is not effective for treatment of the brain because the SGSH enzyme does not cross the BBB, as described by Hemsley et al.(2009): Examination of intravenous and intra-CSF protein delivery for treatment of neurological disease," Eur. J. Neurosci., 29:1197-1214. SGSH was fused to the HIR Ab in order to develop a bifunctional molecule capable of both crossing the BBB and exhibiting enzymatic activity. In one embodiment the amino terminus of the mature SGSH is fused to the carboxyl terminus of each heavy chain of the HIR Ab (Fig. 2).

It was unclear whether the enzymatic activity of the SGSH would be retained when it was fused to the HIR Ab. The experience with IgG-GUSB fusion proteins described in Example 1 illustrates the unpredictable nature of the art, and the chance that either the IgG part or the lysosomal enzyme part could loose biological activity following construction of the IgG-enzyme fusion protein, The situation with SGSH is even more complex, since the SGSH enzyme does become catalytically active until there is a post-translational modification of the protein, wherein the Cys residue near the amino terminus (Cys-514 of SEQ ID NO 10) undergoes a post-translational modification within the endoplasmic reticulum, and it was not known whether that process would be compromised when SGSH was fused to HIR Ab. SGSH is a member of a family of sulfatases, wherein the activity of the enzyme is activated following the conversion of a specific Cys residue to a formylglycine residue by a sulfatase modifying factor type 1 (SUMF1), also called formylglycine-generating enzyme (FGE), in the endoplasmic reticulum [Fraldi et al.(2007), "SUMF1 enhances sulfatase activities in vivo in five sulfatase deficiencies," Biochem. J., 403: 305-312.] Without this conversion of the internal cysteine into a formylglycine residue, the enzyme has little activity. If the SGSH was fused to the carboxyl terminus of the HC of the HIR Ab, e.g. in an effort to retain high affinity binding of the fusion protein to the HIR, then the IgG heavy chain would fold into the 3-dimensional structure following translation within the host cell, followed by folding of the SGSH part of the fusion protein. It was uncertain as to whether the SGSH part of the HIR Ab HC-SGSH fusion protein would fold into a 3-dimensional structure that would be recognized by, and activated by, the SGSH-modifying factors in the endoplasmic reticulum, resulting in expression of full SGSH enzyme activity in the HIR Ab-SGSH fusion protein.

The cDNA for the human SGSH was produced by the polymerase chain reaction (PCR) using oligodeoxynucleotides (ODN) derived from the nucleotide sequence of the human SGSH mRNA (GenBank accession #NM_000199). The cDNA encoding human SGSH minus its signal peptide, Arg21-Leu-502, was generated by reverse transcription (RT) PCR using the ODNs described in Table 2, and commercially available human liver PolyA+ RNA. The forward (FOR) ODN primer has "CC" on the 5'-flanking region to maintain the open reading frame (orf) with the CH3 region of human IgG1 in the tandem vector (TV) expression plasmid and to introduce a Ser-Ser linker between the human IgG1-CH3 and SGSH cDNA. The reverse (REV) ODN is complementary to the end of SGSH orf and includes its stop codon, TGA. RT-PCR was completed and the expected single band of ∼1.5 kb corresponding to SGSH orf cDNA was detected by agarose gel electrophoresis (Figure 3, lane 3) and gel purified. Both forward and reverse ODNs are phosphorylated for direct insertion into the expression vector. The SGSH cDNA was inserted into at the HpaI site of a precursor TV, pUTV-1, with T4 DNA ligase to form TV-HIRMAb-SGSH, which is outlined in Figure 4. The pUTV-1 was linearized with HpaI and digested with alkaline phosphatase to prevent self ligation. The TV-HIRMAb-SGSH is a tandem vector that encompasses the genes for both the light chain (LC) and heavy chain (HC), respectively, of the HIRMAb-SGSH fusion protein followed by the murine dihydrofolate reductase (DHFR) gene. The genes for the light and heavy chain of the HIRMAb-SGSH fusion protein are driven by the CMV promoter and the orfs are followed by the bovine growth hormone (BGH) polyadenylation sequence. The DHFR gene is under the influence of the SV40 promoter and contains the hepatitis B virus (HBV) polyadenylation termination sequence. The DNA sequence of the TV-HIRMAb-SGSH plasmid was confirmed by bidirectional DNA sequencing performed at Sequetech Corp (Mountan View, CA) using custom ODNs synthesized at Midland (Midland, TX). The entire expression cassette of the plasmid was confirmed by sequencing both strands. The fusion of the SGSH monomer to the carboxyl terminus of each HC is depicted in Fig. 2.

**Table 2. Oligodeoxynucleotide primers used in the RT-PCR cloning of human SGSH minus signal peptide and in the engineering of the HIRMAb-SGSH expression vector, derived from human SGSH mRNA sequence (GenBank NM_000199).**

| | |
|---|---|
| SGSH-FWD: 5'-Phosphate-CACGTCCCCGGAACGCACTGCTG | (SEQ ID NO. 11) |
| SGSH-REV: 5'-Phosphate-TCACAGCTCATTGTGGAGGGGCTG | (SEQ ID NO. 12) |

DNA sequencing of the TV-HIRMAb-SGSH plasmid encompassed 10,000 nucleotides (nt), which covered the expression cassettes for the LC gene, the HC-SGSH gene, and the DHFR gene (Figure 4). Beginning at the 5'-end, the plasmid was comprised of a cytomegalovirus (CMV) promoter, a 9 nt full Kozak site, GCCGCCACC (nt 1-9 of SEQ ID NO: 13), a 705 nt open reading frame (orf) for the LC (nt 10-714 of SEQ ID NO: 13), followed by a bovine growth hormone (BGH) polyA sequence, followed by a linker sequence, followed by a tandem CMV promoter, followed by a full Kozak site (nt 1-9 of SEQ ID NO: 14), followed by a 2,841 nt HIRMAb HC-SGSH fusion protein orf (nt 10-2850 of SEQ ID NO: 14), followed by a tandem BGH poly A sequence, followed by the SV40 promoter, followed by a full Kozak site (nt 1-9 of SEQ ID NO: 15), followed by the 564 nt of the DHFR orf (nt 10-573 of SEQ ID NO: 15), followed by the hepatitis B virus poly A sequence (Figure 4). The TV encoded for a 234 amino acid HIRMAb LC (SEQ ID NO: 8), which included a 20 amino acid signal peptide; a 946 amino acid protein fusion protein of the HIRMAb HC and SGSH (SEQ ID NO:1 0). The fusion protein HC was comprised of a 19 amino acid IgG signal peptide, the 442 amino acid HIRMAb HC, a 3 amino acid linker (Ser-Ser-Ser), and the 482 amino acid human SGSH minus the enzyme signal peptide. The predicted molecular weight of the heavy chain fusion protein, minus glycosylation, is 103,412 Da, with a predicted isoelectric point (pI) of 7.44. The amino acid sequence of the SGSH domain of the HC fusion protein is 100% identical to the sequence of amino acids 21-502 of human SGSH (NP_000190), with the exception of the R456H polymorphism within the SGSH domain of the fusion protein, where this numbering system includes the 20 amino acid signal peptide of SGSH. This residue is frequently an arginine (R or Arg) residue, but is also known to be a histidine (H or His) residue. The R456H polymorphism has no significant effect on the enzyme activity of SGSH [Montfort et al. (2004), Expression and functional characterization of human mutant sulfamidase in insect cells, Mol. Genet. Metab. 83: 246-251]. The TV also encodes for the product of the selection gene, DHFR, which is a 187 amino acid protein (SEQ ID NO: 16).

### Example 4. Stable transfection of Chinese hamster ovary cells with TV-HIRMAb-SGSH

Chinese hamster ovary (CHO) cells were grown in serum free HyQ SFM4CHO utility medium (HyClone), containing 1 x HT supplement (hypoxanthine and thymidine). CHO cells (5 x 10⁶ viable cells) were electroporated with 5 µg PvuI-linearized TV-HIRMAb-SGSH plasmid DNA. The cell-DNA suspension was then incubated for 10 min on ice. Cells were electroporated with BioRad pre-set protocol for CHO cells, i.e. square wave with pulse of 15 msec and 160 volts. After electroporation, cells were incubated for 10 min on ice. The cell suspension was transferred to 50 ml culture medium and plated at 125 µl per well in 4 x 96-well plates (10,000 cells per well). A total of 10 electroporations and 4,000 wells were performed per study.

Following electroporation (EP), the CHO cells were placed in the incubator at 37 C and 8% CO2. Owing to the presence of the neo gene in the TV, transfected cell lines were initially selected with G418. The TV-HIRMAb-SGSH also contains the gene for DHFR (Figure 4), so the transfected cells were also selected with 20 nM methotrexate (MTX) and HT deficient medium. Once visible colonies were detected at about 21 days after EP, the conditioned medium was sampled for human IgG by ELISA. Wells with high human IgG signals in the ELISA were transferred from the 96-well plate to a 24-well plate with 1mL of HyQ SFM4CHO-Utility. The 24-well plates were returned to the incubator at 37 C and 8% CO2. The following week IgG ELISA was performed on the clones in the 24-well plates. This was repeated through the 6-well plates to T75 flasks and finally to 60 mL and 125 mL square plastic bottles on an orbital shaker. At this stage, the final MTX concentration was 80 nM, and the medium IgG concentration, which was a measure of HIRMAb-SGSH fusion protein in the medium is >10 mg/L at a cell density of 10⁶/mL.

Clones selected for dilutional cloning (DC) were removed from the orbital shaker in the incubator and transferred to the sterile hood. The cells were diluted to 500 mL in F-12K medium with 5% dialyzed fetal bovine serum (d-FBS) and Penicillin/Streptomycin, and the final dilution is 8 cells per mL, so that 4,000 wells in 40 x 96-well plates can be plated at a cell density of 1 cell per well (CPW). Once the cell suspension was prepared, within the sterile hood, a 125uL aliquot was dispensed into each well of a 96-well plate using an 8-channel pipettor or a precision pipettor system. The plates were returned to the incubator at 37 C and 8% CO2. The cells diluted to 1 cell/well cannot survive without serum. On day 6 or 7, DC plates were removed from the incubator and transferred to the sterile hood where 125 µl of F-12K medium with 5% dialyzed fetal bovine serum (d-FBS) was added to each well. This selection media now contained 5% d-FBS, 30 nM MTX and 0.25 mg/mL Geneticin. On day 21 after the initial 1 CPW plating, aliquots from each of the 4,000 wells were removed for human IgG ELISA, using robotics equipment. DC plates were removed from the incubator and transferred to the sterile hood, where 100 µl of media was removed per well of the 96-well plate and transferred into a new, sterile sample 96-well plate using an 8-channel pipettor or the precision pipettor system.

On day 20 after the initial 1 CPW plating, 40 x 96-well Immunoassay plates were plated with 100uL of 1µg/mL solution of Primary antibody, a mouse anti-human IgG in 0.1M NaHCO3. Plates are incubated overnight in the 4C refrigerator, The following day, the ELISA plates were washed with 1x TBST 5 times, and 100uL of 1ug/mL solution of secondary antibody and blocking buffer were added. Plates are washed with 1x TBST 5 times. 100uL of 1mg/mL of 4-nitrophenyl phosphate di(2-amino-2-ethyl-1,3-propanediol) salt in 0.1M glycine buffer are added to the 96-well immunoassay plates. Plates were read on a microplate reader. The assay produced IgG output data for 4,000 wells/experiment. The highest producing 24-48 wells were selected for further propagation.

The highest producing 24-well plates from the 1 CPW DC were transferred to the sterile hood and gradually subcloned through 6-well dishes, T75 flasks, and 125 mL square plastic bottles on an orbital shaker. During this process the serum was reduced to zero, at the final stage of centrifugation of the cells and resuspension in SFM.

The above procedures were repeated with a second round of dilutional cloning, at 0.5-1 cells/well (CPW). At this stage, approximately 40% of the wells showed any cell growth, and all wells showing growth also secreted human IgG. These results confirmed that on average only 1 cell is plated per well with these procedures, and that the CHO cell line originates from a single cell.

The HIR Ab-SGSH fusion protein was secreted to the medium by the stably transfected CHO cells in high amounts at medium concentrations of 10 mg/L at a cell density of 1-2 million cells/mL. The high production of the HIR Ab-SGSH fusion protein by the stably transfected CHO cells was observed, even though there was no dual transfection of the host cell with the fusion protein genes and the gene encoding SUMF1. In cells transfected with the SGSH gene, it was necessary to co-transfect with the SUMF1 co-factor in order to detect secretion of the SGSH to the medium conditioned by the transfected host cell [Fraldi et al. (2007), "SUMF1 enhances sulfatase activities in vivo in five sulfatase deficiencies," Biochem. J., 403: 305-312]. An unexpected advantage of engineering SGSH and an IgG-SGSH fusion protein is that the host cell secretes the fusion protein without the requirement for the co-transfection with SUMF1.

The CHO-derived HIRMAb-SGSH fusion protein was purified by protein A affinity chromatography. The purity of the HIRMAb-SGSH fusion protein was verified by reducing SDS-PAGE as shown in Fig. 10. Only the HC and LC proteins are detected for either the HIRMAb alone or the HIRMAb-SGSH fusion protein. The identity of the fusion protein was verified by Western blotting using primary antibodies to either human IgG (Fig. 11, left panel) or human SGSH (Fig. 11, right panel). The molecular weight (MW) of the HIRMAb-SGSH heavy and light chains, and the MW of the HIRMAb heavy and light chains are estimated by linear regression based on the migration of the MW standards. The size of the HIRMAb-SGSH fusion heavy chain, 136 kDa, is larger than the size of the heavy chain of the HIRMAb, 62 kDa, owing to the fusion of the SGSH to the HIRMAb heavy chain. The size of the light chain, 27 kDa, is identical for both the HIRMAb-SGSH fusion protein and the HIRMAb antibody, as both proteins use the same light chain. The estimated MW of the hetero-tetrameric HIRMAb-SGSH fusion protein shown in Fig. 2 is 325 kDa, based on migration in the SDS-PAGE of the Western blot.

### Example 5. Analysis of HIR binding and SGSH activity of the bi-functional IgG-SGSH fusion protein

The affinity of the fusion protein for the HIR extracellular domain (ECD) was determined with an ELISA. CHO cells permanently transfected with the HIR ECD were grown in serum free media (SFM), and the HIR ECD was purified with a wheat germ agglutinin affinity column, as previously described in Coloma et al. (2000) Pharm Res, 17:266-274. The HIR ECD was plated on Nunc-Maxisorb 96 well dishes and the binding of the HIR Ab, or the HIR Ab-SGSH fusion protein, to the HIR ECD was detected with a biotinylated goat anti-human IgG (H+L) secondary antibody, followed by avidin and biotinylated peroxidase (Vector Labs, Burlingame, CA). The concentration of either HIR Ab or HIR Ab-SGSH fusion protein that gave 50% maximal binding, ED50, was determined with a non-linear regression analysis. The ED50 of binding to the HIR is 29 ± 3 ng/mL and the ED50 of binding to the HIR of the HIR Ab-SGSH fusion protein is 107 ± 17 ng/mL (Fig. 12). The MW of the HIR Ab is 150 kDa, and the MW of the HIR Ab-SGSH fusion protein is 325 kDa. Therefore, after normalization for MW differences, there was comparable binding of either the chimeric HIR Ab or the HIR Ab-SGSH fusion protein for the HIR ECD with ED50 of 0.19±0.02 nM and 0.33±0.05 nM, respectively (Fig 12). These findings show that the affinity of the HIR Ab-SGSH fusion protein binding to the HIR is retained, despite fusion of a SGSH molecule to the carboxyl termini of both heavy chains of the IgG.

The SGSH enzyme activity was determined with a 2-step fluorometric assay developed by Karpova *et al.* (1996) [A fluorimetric enzyme assay for the diagnosis of Sanfilippo disease type A (MPS IIIA), J. Inher. Metab. Dis. 19: 278-285], which uses 4-methylumbelliferyl-α-N-sulfo-D-glucosaminide (MU-αGlcNS) as the assay substrated. This substrate was custom synthesized by Sigma-Aldrich (St Louis, MO), and the structure of the substrate is outlined in Figure 13A. This substrate is hydrolyzed by SGSH to 4-methylumbelliferyl-α-D-glucosaminide (MU-αGlcNH2), which is then hydrolyzed to the fluorescent product, 4-methylumbelliferone (4-MU) by the α-glucosaminidase side-activity in commercial yeast α-glucosidiase. (Figure 13A). The assay was performed by incubation of the HIRMAb-SGSH fusion protein (30, 100, or 300 ng) and the MU-αGlcNS substrate in 0.03 M sodium barbital/0.03 M sodium acetate/0.13 M NaCl/pH=5.5/0.02% sodum azide for 37C for 17 hours. Mcllvaine's buffer and 0.1 unit of yeast α-glucosidase was added followed by incubation at 37C for 24 hours. The reaction was stopped by the addition of 0.5 M sodium carbonate/pH=10.7. Fluorescense was measured with a Farrand fluorometer with a 365 nm excitation filter and a 450 nm emission filter. A standard curve was generated with 0.03 to 3.0 nmol/tube of the 4-MU product, which allowed for conversion of fluorescent units to nmol/tube. The enzyme activity was measured as units/mg protein of the HIRMAb-SGSH fusion protein, where 1 unit=nmol of 4-MU product formed over the 17 hour primary incubation (Karpova *et al,* 1996). The assay was linear with respect to mass of fusion protein (Fig. 13B), and the average enzyme activity was 4,712 ± 388 units/mg protein. The SGSH enzyme specific activity of the 60 kDa recombinant human SGSH, using the same assay, is 15,000 units/mg protein [Urayama et al. (2008): Mannose 6-phosphate receptor-mediated transport of sulfamidase across the blood-brain barrier in the newborn mouse. Mol Ther 16: 1261-1266.]. However, following re-engineering of the SGSH as a 325 kDa hetero-tetrameric IgG-SGSH fusion protein (Figure 2), the effective MW of the SGSH is 163 kDa, whereas the MW of SGSH is 60 kDa. After normalization for MW differences, the effective SGSH specific activity is 12,800 units/mg protein, which is 85% of the enzyme activity of recombinant SGSH. Therefore, fusion of the SGSH to the carboxyl terminus of the HC of the HIR Ab had minimal effect on the enzyme activity of the SGSH enzyme, in contrast to the result observed with the IgG-GUSB fusion protein (Table 1). The high SGSH enzyme activity of the CHO-derived HIR Ab-SGSH fusion protein is surprising, because SGSH is a member of a family of sulfatases that requires a specific post-translational modification for expression of SGSH enzyme activity. The activity of the SGSH enzyme is activated following the conversion of Cys-50 to a formylglycine residue by a sulfatase modifying factor type 1 (SUMF1), which is also called the formylglycine generating enzyme (FGE). The retention of SGSH enzyme activity in the HIRMAb-SGSH fusion protein produced by the stably transfected CHO cells indicates the SGSH enzyme is activated within the host cell despite fusion to the HIRMAb heavy chain.

### Example 6. Amino acid linker joining the SGSH and the targeting antibody

The mature human SGSH is fused to the carboxyl terminus of the HC of the HIR Ab with a 3-amino acid linker, Ser-Ser-Ser (underlined in Figure 9). Any number of variations of linkers are used as substitutions for the Ser-Ser-Ser linker. The 3-amino acid linker may be retained, but the amino acid sequence is changed to alternative amino acids, such as Gly-Gly-Gly, or Ser-Gly-Ser, or Ala-Ser-Gly, or any number of combinations of the 20 natural amino acids. Or, the linker is reduced to a two, one or zero amino acids. In the case of a zero amino acid linker, the amino terminus of the SGSH is fused directly to the carboxyl terminus of the HC of the HIR Ab. Alternatively, the length of the linker is expanded to 3,4,5,6,7,8,9,10,11,12,13,14,15, or 20 amino acids. Such linkers are well known in the art, as there are multiple publicly available programs for determining optimal amino acid linkers in the engineering of fusion proteins. A frequently used linker includes various combinations of Gly and Ser in repeating sequences, such as (Gly₄Ser)₃, or other variations

### Example 7. HIR Ab-SGSH fusion protein biological activity and lysosomal distribution in Sanfilippo Type A fibroblasts

The SGSH enzyme hydrolyzes the sulfate group from heparan sulfate GAGs, which allows for subsequent degradation of the GAG by other enzymes in the cell. The effect of treatment with the HIRMAb-SGSH fusion protein on the release of sulfate from GAGs was examined in Sanfilippo Type A fibroblasts (MPS-IIIA fibroblasts). The cells are obtained from the Coriell Institute for Medical Research (Camden, NJ), and grown in 6-well cluster dishes to confluency, and then a pulse-chase experiment with 35S is performed. For the Pulse phase, the confluent cells are washed with phosphate buffered saline (PBS), and incubated with 1 mL of low sulfate Ham's F12 medium with 10% dialyzed fetal bovine serum (FBS) and 47 uCi/mL of [35S]sodium sulfate for 48 hours at 37C in a humidified incubator. The medium is aspirated, and the wells are washed with PBS, and the cells are incubated with 1 mL/well of radioactivity-free DMEM/F12 medium with 10% regular FBS, and different concentrations of the HIRMAb-SGSH fusion protein for 48 hours at 37C in a humidified incubator. The medium is aspirated, and the cells washed with PBS. The cells are removed from the well with 0.4 mL/well of 0.05% trypsin/EDTA at 37C for 4 min, as this step removes radioactivity adhered to the cell surface, and not incorporated in lysosomal GAGs. The cells are suspended in serum free medium to stop the trypsin reaction and centrifuged at 1000g. The supernatant is removed and discarded and the cell pellet is solubilized in 0.4 mL IN NaOH at 60C for 1 hr. The protein content is measured with the bicinchoninic acid (BCA) protein assay. The 35S radioactivity is measured with a Perkin Elmer liquid scintillation counter in Ultima-gold counting solution. The CPM radioactivity is divided by the mg protein well and the data reported as CPM/mg protein (Table 3). This study shows that sub-therapeutic concentrations of the HIRMAb-SGSH fusion protein, 0.25-0.5 ug/mL, cause a 72%-83% reduction in GAGs labeled with sulfate in MPS-IIIA fibroblasts.

The reduction in sulfated GAGs in the MPS-IIIA fibroblasts suggests the HIRMAb-SGSH fusion protein is not only taken up by the target cells, but is triaged to the lysosomal compartment of the target cell. This was confirmed by confocal microscopy. The MPS-IIIA fibroblasts were incubated with the HIRMAb-SGSH fusion protein for 6-24 hrs, and then washed, fixed in 4% paraformaldehyde, permeabilized with Triton X-100, and incubated with a rabbit primary antibody to human SGSH and a mouse primary antibody to human lysosomal associated membrane protein type 1 (LAMP1), followed by incubation with a Alexa Fluor-488 conjugated donkey anti-mouse IgG (green channel) and Alexa Fluor-594 conjugated donkey anti-rabbit IgG (red channel). The washed slides were stained with 4',6-diamidino-2-phenylindole (DAPI) (Life Technologies), washed, air dried, and mounted in Prolong Gold Antifade (Life Technologies). Confocal microscopy was performed with a Leica TCS SP2 AOBS inverted fluorescence microscope with a Leitz PI Apo 100X oil immersion objective and a Leica confocal laser scanning adapter utilizing argon (476 and 488 nm), new diode (561 nm) and helium-neon (633 nm) lasers, respectively. The SGSH immunoreactivity is detected in the red channel, and the LAMP1 immunoreactivity within the cell is detected in the green channel. The overlap of the SGSH and LAMP1 immunoreactivity indicates the HIRMAb-SGSH fusion protein is triaged to the lysosomal compartment following uptake into MPSIIIA fibroblasts. There was no immunoreactivity in the cells labeled with isotype control antibodies.

**Table 3. Reduction in GAGs in MPS-IIIA fibroblasts with treatment with the HIRMAb-SGSH fusion protein**

| Concentration of HIRMAb-SGSH fusion protein (ug/mL) | 35S CPM/mg protein | % GAG reduction |
|---|---|---|
| 0 | 41,402 ± 6,199 | 0 |
| 0.25 | 11,468 ± 1,343 | 72% |
| 0.5 | 6,952 ± 771 | 83% |

### Example 8. Receptor-mediated delivery of SGSH to the primate brain

The BBB-penetration in vivo of the HIRMAb-SGSH fusion protein was evaluated in the rhesus monkey. The HIRMAb domain of the fusion protein cross reacts with the insulin receptor of Old World primates, such as the Rhesus monkey, but not with the insulin receptor in New World primates, such as the squirrel monkey, or with the insulin receptor in the mouse. Therefore, in vivo delivery to brain was evaluated in the rhesus monkey. The HIRMAb-SGSH fusion protein was radiolabeled with [¹²⁵I]-monoiodinated-Bolton-Hunter reagent to a specific activity of 3.7 uCi/ug. The trichloroacetic acid (TCA) precipitability of the [¹²⁵I]-HIRMAb-SGSH fusion protein was >97% for least 8 days after labeling during storage at -70C. Prior to labeling, the fusion protein was buffer exchanged with 0.01 M sodium acetate/140 mM NaCl/pH=5.5/0.001% Tween-80 and an Amicon Ultracel-30K centrifugal filter unit. The labeled HIRMAb-SGSH fusion protein was purified by gel filtration with a 1x28 cm column of Sephadex G-25 and an elution buffer of 0.01 M sodium acetate/140 mM NaCl/pH=5.5/0.001% Tween-80. An adult male Rhesus monkey, 17.3 kg, was investigated at MPI Research (Mattawan, MI). The animal was injected intravenously (IV) with 1200 uCi of [¹²⁵I]-HIRMAb-SGSH fusion protein by bolus injection over 30 seconds in the left femoral vein. The injection dose (ID) of the HIRMAb-SGSH fusion protein was 19 ug/kg. The animal was anesthetized with intramuscular ketamine. All procedures were carried out in accordance with the Guide for the Care and Use of Laboratory Animals as adopted and promulgated by the U.S. National Institutes of Health. Following intravenous drug administration, femoral venous plasma was obtained at 2,5, 15, 30, 60, 90, and 140 min for determination of total plasma [¹²⁵I] radioactivity (DPM/mL) and plasma radioactivity that is precipitated by 10% cold TCA. The animal was euthanized, and samples of major organs (heart, liver, spleen, lung, skeletal muscle, and omental fat) were removed, weighed, and processed for determination of radioactivity. The cranium was opened and the brain was removed. Samples of frontal cortical gray matter, cerebellar gray matter, and choroid plexus were removed for radioactivity determination. Plasma and tissue samples were analyzed for ¹²⁵I radioactivity with a Wizard model 1470 gamma counter. The TCA precipitable [¹²⁵I] radioactivity in plasma, DPM/mL, was converted to ng/mL, based on the specific activity of the injected fusion protein, and a bi-exponential equation, %ID/mL = Ale^{-k1t} + A2e^{-k2t}, was fit to the plasma fusion protein concentration. The intercepts (A1, A2) and the slopes (kl, k2) were used to compute the median residence time (MRT), the central volume of distribution (Vc), the steady state volume of distribution (Vss), the area under the plasma concentration curve (AUC), and the systemic clearance (CL). Non-linear regression analysis used the AR subroutine of the BMDP Statistical Software (Statistical Solutions Ltd, Cork, Ireland). Data were weighted by 1/(%ID/mL)².

Samples (∼2 gram) of frontal cortex were removed for capillary depletion analysis to confirm transport of the HIRMAb-SGSH fusion protein across the BBB. The capillary depletion method separates the vascular tissue in brain from the post-vascular compartment. Based on measurements of the specific activity of brain capillary-specific enzymes, such as γ-glutamyl transpeptidase or alkaline phosphatase, the post-vascular supernatant is >95% depleted of brain vasculature. To separate the vascular and post-vascular compartments, the brain was homogenized in 8 mL cold PBS in a tissue grinder. The homogenate was supplemented with 8 mL cold 40% dextran (70 kDa, Sigma Chemical Co.), and an aliquot of the homogenate was taken for radioactivity measurement. The homogenate was centrifuged at 3200 g at 4C for 10 min in a fixed angle rotor. The brain microvasculature quantitatively sediments as the pellet, and the post-vascular supernatant is a measure of capillary depleted brain parenchyma. The vascular pellet and supernatant were counted for ¹²⁵I radioactivity in parallel with the homogenate. The volume of distribution (VD) was determined for each of the 3 fractions from the ratio of total [¹²⁵I] radioactivity in the brain fraction (DPM/gram brain) divided by the total [¹²⁵I] radioactivity in the 140 min terminal plasma (DPM/uL plasma). The percent of radioactivity in the post-vascular supernatant that was precipitable with 10% cold TCA was determined.

The [¹²⁵I]-HIRMAb-SGSH fusion protein (1200 uCi, 324 ug) was injected IV in a male Rhesus monkey, and the time course of TCA precipitable [¹²⁵I]- HIRMAb-SGSH fusion protein concentration in plasma is shown in Figure 14. The percent of total plasma radioactivity that was precipitable by TCA was 96 ± 1%, 95 ± 1%, 94 ± 1%, 89 ± 1%, 84 ± 2%, 79 ± 1%, and 72 ± 2%, respectively at 2, 5, 15, 30, 60, 90, and 140 min after IV injection. A 2-exponential equation was fit to the plasma profile of TCA-precipitable fusion protein to yield the pharmacokinetic (PK) parameters shown in Table 4. The [¹²⁵I]-HIRMAb-SGSH fusion protein is rapidly cleared from plasma with a mean residence time of 62 ± 4 minutes, a systemic volume of distribution (Vss) that is 2.5-fold greater the central compartment volume (Vc), and a high rate of systemic clearance, 1.11 ± 0.03 mL/min/kg (Table 4).

**Table 4. Pharmacokinetic parameters of the HIRMAb-SHSH fusion protein**

| **parameter** | **units** | **value** |
|---|---|---|
| T1/2¹ | min | 5.5 ± 0.8 |
| T1/2² | min | 55 ± 4 |
| MRT | min | 62 ± 4 |
| Vc | mL/kg | 28 ± 2 |
| Vss | mL/kg | 69 ± 4 |
| AUCss | ug·min/mL | 16.8 ± 0.5 |
| CL | mL/min/kg | 1.11 ± 0.03 |

| | | |
|---|---|---|
| Parameters computed from the plasma profile in figure 14. | | |

The volume of distribution (VD) of the HIRMAb-SGSH fusion protein in total brain homogenate at 140 minutes after injection is high, 782 ± 36 uL/gram, compared to the brain VD of a non-specific human IgG1 isotype control antibody, 20 ± 6 ul/gram (Table 5). The brain VD of the IgG1 isotype control antibody represents the brain uptake of a molecule that is sequestered within the blood volume of brain, and which does not cross the BBB. The VD of the HIRMAb-SGSH fusion protein in the post-vascular supernatant, 666 ± 71 uL/gram, is greater than the VD of the HIRMAb-SGSH fusion protein in the vascular pellet of brain, 24 ± 17 uL/gram (Table 5), which indicates that the majority of the HIRMAb-SGSH fusion protein has traversed the BBB and penetrated the brain parenchyma. The radioactivity in the post-vascular supernatant represents intact HIRMAb-SGSH fusion protein, and not labeled metabolites, as the TCA precipitation of the post-vascular supernatant radioactivity is 95.9 ± 0.7% (Table 5).

**Table 5. Capillary depletion analysis of the brain uptake of the HIRMAb-SGSH fusion protein**

| **Molecule** | **Brain fraction** | **VD (µL/g)** |
|---|---|---|
| HIRMAb-SGSH fusion protein | Brain homogenate | 782 ± 36 |
| | Post-vascular supernatant | 666 ± 71 |
| | Vascular pellet | 24 ± 17 |
| Human IgG1 isotype control | Brain homogenate | 20 ± 6 |

| | | |
|---|---|---|
| Mean ± S.D. The fusion protein was administered by IV injection, and brain measurements made 140 min following injection. The radioactivity in the post-vascular supernatant was 95.9 ± 0.7 % precipitable by cold 10% trichloroacetic acid. The homogenate VD for the human IgG1 isotype control antibody is reported previously (Boado, R.J.; Pardridge, W.M. Comparison of blood-brain barrier transport of GDNF and an IgG-GDNF fusion protein in the Rhesus monkey. Drug Metab. Disp. 2009, 37, 2299-2304). | | |

The organ uptake of the HIRMAb-SGSH fusion protein is expressed as % of injected dose (ID) per 100 grams wet organ weight (Table 6), because the brain of the adult Rhesus monkey weighs 100 grams. The major organs accounting for the removal of the HIRMAb-SGSH fusion protein from plasma are liver and spleen (Table 6). The brain cortical uptake of the HIRMAb-SGSH fusion protein is 0.81 ± 0.07 % ID/100 gram brain (Table 6).

**Table 6. Organ uptake of the HIRMAb-SGSH fusion protein in the Rhesus monkey**

| **organ** | **Organ uptake (%ID/100 grams)** |
|---|---|
| Frontal cortex | 0.81 ± 0.07 |
| Cerebellar cortex | 0.60 ± 0.04 |
| Choroid plexus | 1.13 ± 0.06 |
| liver | 16.2 ± 0.4 |
| spleen | 14.7 ± 0.5 |
| lung | 1.1 ± 0.2 |
| heart | 0.93 ± 0.11 |
| fat | 0.044 ± 0.004 |
| Skeletal muscle | 0.31 ± 0.21 |

| | |
|---|---|
| Data are mean ± SD of triplicate samples. | |

In summary, the primate study shows the HIRMAb-SGSH fusion protein is rapidly cleared from plasma following IV administration, owing to uptake by peripheral organs. However, unlike SGSH alone, the HIRMAb-SGSH fusion protein rapidly penetrates the BBB (Tables 5 and 6). Conversely, SGSH alone does not cross the BBB [Urayama, A.; Grubb, J.H.; Sly, W.S.; Banks, W.A. Mannose 6-phosphate receptor-mediated transport of sulfamidase across the blood-brain barrier in the newborn mouse. Mol Ther. 2008, 16, 1261-1266].

### Example 9. Receptor-mediated delivery of SGSH to the human brain

Sanfilippo Type A, or MPS-IIIA, is a lysosomal storage disorder caused by defects in the gene encoding the lysosomal enzyme, sulfamidase (SGSH). In the absence of SGSH, certain GAGs such as heparan sulfate accumulate in the cells. The accumulation of the heparan sulfate in the brain leads to the clinical manifestations of MPS-IIIA, which includes severe behavioral disturbances, loss of speech generally by the age of 7 years, impaired walking leading to wheelchair existence typically by the age of 12, and death at a mean age of 18 years [Valstar et al. (2010): Mucopolysaccharidosis Type IIIA: clinical spectrum and genotype-phenotype correlations. Ann. Neurol. 68:876-887].

The nucleotide sequence of the SGSH mRNA and the amino acid sequence of the human SGSH protein is known [Scott et al. (1995), Cloning of the sulphamidase gene and identification of mutations in Sanfilippo A syndrome, Nature Genetics, 11:465-467]. This sequence enables the production of recombinant SGSH for the enzyme replacement therapy (ERT) of MPS-IIIA. SGSH produced in Chinese hamster ovary (CHO) cells has a specific activity of 15 units/ug enzyme [Urayama et al. (2008), Mannose 6-phosphate receptor-mediated transport of sulfamidase across the blood-brain barrier in the newborn mouse, Mol. Ther. 16: 1261-1266]. The SGSH specific activity is determined by the same 2-step fluorometric enzyme assay described above [Karpova et al. (1996) A fluorimetric enzyme assay for the diagnosis of Sanfilippo disease type A (MPS IIIA), J. Inher. Metab. Dis. 19: 278-285]. The problem with ERT of MPS-IIIA with recombinant SGSH is that SGSH, like other large molecule pharmaceuticals, does not cross the BBB, and is not an effective treatment of MPS-IIIA when given intravenously [Hemsley et al. (2009) Examination of intravenous and intra-CSF protein delivery for treatment of neurological disease, Eur. J. Neurosci. 29: 1197-1214]. Owing to the lack of transport of SGSH across the BBB, it is not possible to increase SGSH in brain following the systemic injection of large doses of the enzyme. Accordingly, intravenous ERT in MPS-IIIA patients with recombinant SGSH is not expected to have any beneficial effect on the brain. In an attempt to by-pass the BBB by the direct intracerebroventricular (ICV) infusion of SGSH into the brains of MPS-IIIA dogs, the enzyme was infused into the ventricle [Jolly et al. (2011), Intracisternal enzyme replacement therapy in lysosomal storage diseases: routes of absorption into brain, Neuropathol Appl. Neurobiol. 37: 414-422]. The ICV route of drug delivery to the brain is well known to distribute drug only to the ependymal and meningeal surface of the brain and this is what was observed following ICV administration of SGSH in MPS-IIIA dogs.

ICV enzyme administration is an invasive procedure that requires implantation of chronic catheter into the brain. The preferred approach to the delivery of SGSH to the brain of MPS-IIIA patients is via an intravenous infusion of a form of SGSH that is re-engineered to cross the BBB via receptor-mediated transport (RMT). The HIRMAb-SGSH fusion protein retains high affinity binding to the human insulin receptor, which enables the SGSH to penetrate the BBB and enter brain from blood via RMT on the endogenous BBB insulin receptor. The brain uptake of the HIRMAb-SGSH fusion protein is 1% of injected dose (ID) per 100 grams brain in the Rhesus monkey (Table 6). Given this level of brain uptake of the fusion protein, the SGSH enzyme activity in brain following the IV administration of the HIRMAb-SGSH fusion protein can be calculated, and compared to the normal endogenous SGSH enzyme activity in the brain. Given, a SGSH specific activity of the SGSH fusion protein of 5000 units/mg fusion protein (Example 4), and an intravenous administration of 5 mg/kg of the IgG-SGSH fusion protein, in a 50 kg human, then the injection dose is equal to 250 mg, or 1,250,000 units of the fusion protein. The brain uptake of the HIRMAb-SGSH fusion protein is ∼1% ID/100 gram brain in the Rhesus monkey (Table 6), and the brain of the Rhesus monkey weighs 100 grams. Therefore, the brain uptake of the HIRMAb-SGSH fusion protein is about 1% of the dose injected intravenously, which is equal to 12,500 units/brain in a 50 kg human administered 3 mg/kg of the fusion protein. This level of brain SGSH enzyme activity is equal to 12.5 units/gram brain, since the human brain weighs about 1,000 grams, and is equal to a brain SGSH enzyme activity of 0.125 units/mg brain protein, since 1 gram of brain is equal to about 100 mg of protein. The SGSH enzyme activity in normal brain ranges from 0.12 units/mg protein [Tomatsu, S.; Vogler, C.; Montano, A.M.; Gutierrez, M.; Oikawa, H.; Dung, V.C.; Orii, T.; Noguchi, A.; Sly, W.S. Murine model (Galnstm(C76S)slu) of MPS IVA with missense mutation at the active site cysteine conserved among sulfatase proteins. Mol. Genet. Metab. 2007, 91, 251-258] to 1.4 units/mg protein [Fraldi et al. (2007) Functional correction of CNS lesions in an MPS-IIIA mouse model by intracerebral AAV-mediated delivery of sulfamidase and SUMF1 genes, Human Mol. Genet 16: 2693-2702]. Therefore, the administration of a dose of HIRMAb-SGSH fusion protein of 3 mg/kg produces a level of SGSH enzyme activity in the brain that is 10-100% of the normal endogenous SGSH enzyme activity. Enzyme replacement therapy in patients with lysosomal storage disorders that produces a cellular enzyme activity of just 1-2% of normal do not develop signs and symptoms of the disease (J. Muenzer and A. Fisher, Advances in the treatment of mucopolysaccharidosis type I, N. Engl J Med, 350: 1932-1934, 2004). These considerations show that a clinically significant SGSH enzyme replacement of the human brain is possible following the intravenous infusion of the HIRMAb-SGSH fusion protein at a systemic dose of approximately 3 mg/kg, or a range of 1-10 mg/kg of the HIRMAb-SGSH fusion protein.

The following numbered paragraphs (paras.) contain statements of broad combinations of the inventive technical features herein disclosed:-
1. A method for treating an N-sulfoglucosamine sulfohydrolase (SGSH) deficiency in the central nervous system of a subject in need thereof, comprising systemically administering to the subject a therapeutically effective dose of a fusion antibody having SGSH activity, wherein the fusion antibody comprises: (a) a fusion protein comprising the amino acid sequences of an immunoglobulin heavy chain and a SGSH, and (b) an immunoglobulin light chain; wherein the fusion antibody crosses the blood brain barrier (BBB).
2. The method of para. 1, wherein the amino acid sequence of the SGSH is covalently linked to the carboxy terminus of the amino acid sequence of the immunoglobulin heavy chain.
3. The method of para. 1, wherein the fusion antibody is post-translationally modified by a sulfatase modifying factor type 1 (SUMF1).
4. The method of para. 3, wherein the post-translational modification comprises a cysteine to formylglycine conversion.
5. The method of para. 1, wherein the fusion antibody comprises formylglycine.
6. The method of para. 1, wherein the fusion antibody catalyzes hydrolysis of sulfate groups from heparan sulfate.
7. The method of para. 1, wherein the SGSH retains at least 20% of its activity compared to its activity as a separate entity.
8. The method of para. 1, wherein the SGSH and the immunoglobulin each retains at least 20% of its activity compared to its activity as a separate entity.
9. The method of para. 1, wherein at least about 200 ug of SGSH enzyme are delivered to the brain, normalized per 50 kg body weight.
10. The method of para. 1, wherein the therapeutically effective dose comprises at least about 1000 units/Kg of body weight.
11. The method of para. 1, wherein the SGSH specific activity of the fusion antibody is at least 1000 units/mg.
12. The method of para. 1, wherein the immunoglobulin heavy chain is an immunoglobulin heavy chain of IgG.
13. The method of para. 1, wherein the immunoglobulin heavy chain is an immunoglobulin heavy chain of IgG1 class.
14. The method of para. 1, wherein the immunoglobulin heavy chain comprises a CDR1 corresponding to the amino acid sequence of SEQ ID NO:1, a CDR2 corresponding to the amino acid sequence of SEQ ID NO:2, or a CDR3 corresponding to the amino acid sequence of SEQ ID NO:3.
15. The method of para. 1, wherein the immunoglobulin light chain is an immunoglobulin light chain of kappa or lambda class.
16. The method of para. 1, wherein the immunoglobulin light chain comprises a CDR1 corresponding to the amino acid sequence of SEQ ID NO:4, a CDR2 corresponding to the amino acid sequence of SEQ ID NO:5, or a CDR3 corresponding to the amino acid sequence of SEQ ID NO:6.
17. The method of para. 1, wherein the fusion antibody crosses the BBB by binding an endogenous BBB receptor-mediated transport system.
18. The method of para. 1, wherein the fusion antibody crosses the BBB via an endogenous BBB receptor selected from the group consisting of the insulin receptor, transferrin receptor, leptin receptor, lipoprotein receptor, and the insulin-like growth factor (IGF) receptor.
19. The method of para. 1, wherein the fusion antibody crosses the BBB by binding an insulin receptor.
20. The method of para. 1, wherein the systemic administration is parenteral, intravenous, subcutaneous, intra-muscular, trans-nasal, intra-arterial, transdermal, or respiratory.
21. The method of para. 1, wherein the SGSH deficiency in the central nervous system is mucopolysaccharidosis Type IIIA (MPS-IIIA) or Sanfilippo syndrome type A.
22. A method for treating an N-sulfoglucosamine sulfohydrolase (SGSH) deficiency in the central nervous system of a subject in need thereof, comprising systemically administering to the subject a therapeutically effective dose of a fusion antibody having SGSH activity, wherein the fusion antibody comprises: (a) a fusion protein comprising the amino acid sequence that is at least 90% identical to SEQ ID NO:10, and (b) an immunoglobulin light chain; wherein the fusion antibody crosses the blood brain barrier (BBB).
23. The method of para. 22, wherein the fusion antibody catalyzes hydrolysis of sulfate groups from heparan sulfate.
24. The method of para. 22, wherein the fusion antibody is post-translationally modified by a sulfatase modifying factor type 1 (SUMF1).
25. The method of para. 24, wherein the post-translational modification comprises a cysteine to formylglycine conversion.
26. The method of para. 22, wherein the fusion antibody comprises formylglycine.
27. The method of para. 22, wherein at least about 200 ug of SGSH enzyme are delivered to the brain, normalized per 50 kg body weight.
28. The method of para. 22, wherein the therapeutically effective dose comprises at least about 1000 units of SGSH activity/Kg of body weight.
29. The method of para. 22, wherein the SGSH specific activity of the fusion antibody is at least about 1000 units/mg.
30. The method of para. 22, wherein the SGSH retains at least 20% of its activity compared to its activity as a separate entity.
31. The method of para. 22, wherein the SGSH and the immunoglobulin each retains at least 20% of its activity compared to its activity as a separate entity.
32. The method of para. 22, wherein the systemic administration is parenteral, intravenous, subcutaneous, intra-muscular, trans-nasal, intra-arterial, transdermal, or respiratory.
33. The method of para. 22, wherein the immunoglobulin heavy chain is an immunoglobulin heavy chain of IgG.
34. The method of para. 22, wherein the immunoglobulin heavy chain is an immunoglobulin heavy chain of IgG1 class.
35. The method of para. 22, wherein the immunoglobulin light chain is an immunoglobulin light chain of kappa or lambda class.
36. The method of para. 22, wherein the immunoglobulin light chain comprises a CDR1 corresponding to the amino acid sequence of SEQ ID NO:4, a CDR2 corresponding to the amino acid sequence of SEQ ID NO:5, or a CDR3 corresponding to the amino acid sequence of SEQ ID NO:6.
37. The method of para. 22, wherein the fusion antibody crosses the BBB by binding an endogenous BBB receptor-mediated transport system.
38. The method of para. 22, wherein the fusion antibody crosses the BBB via an endogenous BBB receptor selected from the group consisting of the insulin receptor, transferrin receptor, leptin receptor, lipoprotein receptor, and the IGF receptor.
39. The method of para. 22, wherein the fusion antibody crosses the BBB by binding an insulin receptor.
40. The method of para. 22, wherein the SGSH deficiency in the central nervous system is mucopolysaccharidosis Type IIIA (MPS-IIIA) or Sanfilippo syndrome type A.
41. A method for treating an N-sulfoglucosamine sulfohydrolase (SGSH) deficiency in the central nervous system of a subject in need thereof, comprising systemically administering to the subject a therapeutically effective dose of a fusion antibody having SGSH activity, wherein the fusion antibody comprises: (a) a fusion protein comprising the amino acid sequences of an immunoglobulin light chain and a SGSH, and (b) an immunoglobulin heavy chain; wherein the fusion antibody crosses the blood brain barrier (BBB).
42. The method of para. 41, wherein the amino acid sequence of the SGSH is covalently linked to the carboxy terminus of the amino acid sequence of the immunoglobulin light chain.
43. The method of para. 41, wherein the fusion antibody catalyzes hydrolysis of sulfate groups from heparan sulfate.
44. The method of para. 41, wherein the fusion antibody is post-translationally modified by a sulfatase modifying factor type 1 (SUMF1).
45. The method of para. 44, wherein the post-translational modification comprises a cysteine to formylglycine conversion.
46. The method of para. 41, wherein the fusion antibody comprises formylglycine.
47. The method of para. 41, wherein at least about 200 ug of SGSH enzyme are delivered to the brain, normalized per 50 kg body weight.
48. The method of para. 41, wherein the therapeutically effective dose comprises at least about least about 1000 units of SGSH activity/Kg body weight.
49. The method of para. 41, wherein the SGSH specific activity of the fusion antibody is about 1000 units/mg.
50. The method of para. 41, wherein the SGSH retains at least 20% of its activity compared to its activity as a separate entity.
51. The method of para. 41, wherein the SGSH and the immunoglobulin each retains at least 20% of its activity compared to its activity as a separate entity.
52. The method of para. 41, wherein the immunoglobulin heavy chain is an immunoglobulin heavy chain of IgG.
53. The method of para. 41, wherein the immunoglobulin heavy chain is an immunoglobulin heavy chain of IgG1 class.
54. The method of para. 41, wherein the immunoglobulin heavy chain comprises a CDR1 corresponding to the amino acid sequence of SEQ ID NO:1, a CDR2 corresponding to the amino acid sequence of SEQ ID NO:2, or a CDR3 corresponding to the amino acid sequence of SEQ ID NO:3.
55. The method of para. 41, wherein the immunoglobulin light chain is an immunoglobulin light chain of kappa or lambda class.
56. The method of para. 41, wherein the immunoglobulin light chain comprises a CDR I corresponding to the amino acid sequence of SEQ ID NO:4, a CDR2 corresponding to the amino acid sequence of SEQ ID NO:5, or a CDR3 corresponding to the amino acid sequence of SEQ ID NO:6.
57. The method of para. 41, wherein the fusion antibody crosses the BBB by binding an endogenous BBB receptor-mediated transport system.
58. The method of para. 41, wherein the fusion antibody crosses the BBB via an endogenous BBB receptor selected from the group consisting of the insulin receptor, transferrin receptor, leptin receptor, lipoprotein receptor, and the IGF receptor.
59. The method of para. 41, wherein the fusion antibody crosses the BBB the insulin receptor.
60. The method of para. 41, wherein the systemic administration is parenteral, intravenous, subcutaneous, intra-muscular, trans-nasal, intra-arterial, transdermal, or respiratory.
61. The method of para. 41, wherein the SGSH deficiency in the central nervous system is mucopolysaccharidosis Type IIIA (MPS-IIIA) or Sanfilippo syndrome type A.
62. A fusion antibody comprising: (a) a fusion protein comprising the amino acid sequences of an immunoglobulin heavy chain and a SGSH, and (b) an immunoglobulin light chain; wherein the fusion antibody crosses the blood brain barrier (BBB).
63. The fusion antibody of para. 62, wherein the amino acid sequence of the SGSH is covalently linked to the carboxy terminus of the amino acid sequence of the immunoglobulin heavy chain.
64. The fusion antibody of para. 62, wherein the fusion antibody catalyzes hydrolysis of sulfate groups from heparan sulfate.
65. The fusion antibody of para. 62, wherein the fusion antibody is post-translationally modified by a sulfatase modifying factor type 1 (SUMF1).
66. The fusion antibody of para. 65, wherein the post-translational modification comprises a cysteine to formylglycine conversion.
67. The fusion antibody of para. 62, wherein fusion antibody comprises a formylglycine.
68. The fusion antibody of para. 62, wherein the fusion protein further comprises a linker between the amino acid sequence of the SGSH and the carboxy terminus of the amino acid sequence of the immunoglobulin heavy chain.
69. The fusion antibody of para. 62, wherein the SGSH specific activity of the fusion antibody is at least about 1000 units/mg.
70. The fusion antibody of para. 62, wherein the SGSH retains at least 20% of its activity compared to its activity as a separate entity.
71. The fusion antibody of para. 62, wherein the SGSH and the immunoglobulin each retains at least 20% of its activity compared to its activity as a separate entity,
72. The fusion antibody of para. 62, wherein the immunoglobulin heavy chain is an immunoglobulin heavy chain of IgG.
73. The fusion antibody of para. 62, wherein the immunoglobulin heavy chain is an immunoglobulin heavy chain of IgG1 class.
74. The fusion antibody of para. 62, wherein the immunoglobulin heavy chain comprises a CDR1 corresponding to the amino acid sequence of SEQ ID NO:1, a CDR2 corresponding to the amino acid sequence of SEQ ID NO:2, or a CDR3 corresponding to the amino acid sequence of SEQ ID NO:3.
75. The fusion antibody of para. 62, wherein the immunoglobulin light chain is an immunoglobulin light chain of kappa or lambda class.
76. The fusion antibody of para. 62, wherein the immunoglobulin light chain comprises a CDR1 corresponding to the amino acid sequence of SEQ ID NO:4, a CDR2 corresponding to the amino acid sequence of SEQ ID NO:5, or a CDR3 corresponding to the amino acid sequence of SEQ ID NO:6.
77. The fusion antibody of para. 62, wherein the fusion antibody crosses the BBB by binding an endogenous BBB receptor-mediated transport system.
78. The fusion antibody of para. 62, wherein the fusion antibody crosses the BBB via an endogenous BBB receptor selected from the group consisting of the insulin receptor, transferrin receptor, leptin receptor, lipoprotein receptor, and the IGF receptor.
79. The fusion antibody of para. 62, wherein the fusion antibody crosses the BBB by binding an insulin receptor.
80. A pharmaceutical composition comprising a therapeutically effective amount of a fusion antibody of para. 62, and a pharmaceutically acceptable excipient.
81. An isolated polynucleotide encoding the fusion antibody of para. 62.
82. The isolated polynucleotide of para. 81, wherein the isolated polynucleotide comprises the nucleic acid sequence of SEQ ID NO: 14.
83. A vector comprising the isolated polynucleotide of para. 81.
84. The vector of para. 83 comprising the nucleic acid sequence of SEQ ID NO: 14.
85. A host cell comprising the vector of para. 83.
86. The host cell of para. 85, wherein the host cell is a Chinese Hamster Ovary (CHO) cell.
87. A method for treating an enzyme deficiency in the central nervous system of a subject in need thereof, comprising systemically administering to the subject a therapeutically effective dose of a fusion antibody comprising (a) a fusion protein comprising the amino acid sequences of an immunoglobulin heavy chain and an enzyme deficient in mucopolysaccharidosis III (MPS-III), and (b) an immunoglobulin light chain; wherein the fusion antibody crosses the blood brain barrier (BBB).
88. The method of para. 87, wherein the enzyme deficient in MPS-III is N-sulfoglucosamine sulfohydrolase (SGSH), alpha-N-acetylglucosaminidase (NAGLU), heparin-alpha-glucosaminide N-acetyltransferase (HGSNAT), or N-acetylglucosamine-6-sulfatase (GNS).
89. The method of para. 87, wherein the amino acid sequence of the enzyme is covalently linked to the carboxy terminus of the amino acid sequence of the immunoglobulin heavy chain.
90. The method of para. 87, wherein the fusion antibody is post-translationally modified by a sulfatase modifying factor type 1 (SUMF1).
91. The method of para. 90, wherein the post-translational modification comprises a cysteine to formylglycine conversion.
92. The method of para. 87, wherein the fusion antibody comprises formylglycine.
93. The method of para. 87, wherein the fusion antibody catalyzes hydrolysis of sulfate groups from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.
94. The method of para. 87, wherein the enzyme retains at least 20% of its activity compared to its activity as a separate entity.
95. The method of para. 87, wherein the enzyme and the immunoglobulin each retains at least 20% of its activity compared to its activity as a separate entity.
96. The method of para. 87, wherein at least about 200 ug of the enzyme are delivered to the brain, normalized per 50 kg body weight.
97. The method of para. 87, wherein the therapeutically effective dose comprises at least about 1000 units/Kg of body weight.
98. The method of para. 87, wherein the enzyme specific activity of the fusion antibody is at least 1000 units/mg.
99. The method of para. 87, wherein the immunoglobulin heavy chain is an immunoglobulin heavy chain of IgG.
100. The method of para. 87, wherein the immunoglobulin heavy chain is an immunoglobulin heavy chain of IgG1 class.
101. The method of para. 87, wherein the immunoglobulin light chain is an immunoglobulin light chain of kappa or lambda class.
102. The method of para. 87, wherein the enzyme comprises an amino acid sequence selected from SEQ ID NO:9, SEQ ID NO:17, SEQ ID NO:19, and SEQ ID NO:21.
103. The method of para. 87, wherein the fusion antibody crosses the BBB by binding an endogenous BBB receptor-mediated transport system.
104. The method of para. 87, wherein the fusion antibody crosses the BBB via an endogenous BBB receptor selected from the group consisting of the insulin receptor, transferrin receptor, leptin receptor, lipoprotein receptor, and the insulin-like growth factor (IGF) receptor.
105. The method of para. 87, wherein the fusion antibody crosses the BBB by binding an insulin receptor.
106. The method of para. 87, wherein the systemic administration is parenteral, intravenous, subcutaneous, intra-muscular, trans-nasal, intra-arterial, transdermal, or respiratory.
107. The method of para. 87, wherein the enzyme deficiency in the central nervous system is mucopolysaccharidosis IIIA (MPS-IIIA), mucopolysaccharidosis IIIB (MPS-IIIB), mucopolysaccharidosis IIIC (MPS-IIIC), or mucopolysaccharidosis IIID (MPS-IIID).
108. A method for treating an enzyme deficiency in the central nervous system of a subject in need thereof, comprising systemically administering to the subject a therapeutically effective dose of a fusion antibody comprising (a) a fusion protein comprising the amino acid sequence that is at least 90% identical to SEQ ID NO:10, SEQ ID NO:18, SEQ ID NO:20, or SEQ ID NO:22, and (b) an immunoglobulin light chain; wherein the fusion antibody crosses the blood brain barrier (BBB).
109. The method of para. 108, wherein the fusion antibody catalyzes hydrolysis of sulfate groups from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.
110. The method of para. 108, wherein the fusion antibody is post-translationally modified by a sulfatase modifying factor type 1 (SUMF1).
111. The method of para. 110, wherein the post-translational modification comprises a cysteine to formylglycine conversion.
112. The method of para. 108, wherein the fusion antibody comprises formylglycine.
113. The method of para. 108, wherein at least about 200 ug of the enzyme are delivered to the brain, normalized per 50 kg body weight.
114. The method of para. 108, wherein the therapeutically effective dose comprises at least about 1000 units of enzyme activity/Kg of body weight.
115. The method of para. 108, wherein the enzyme specific activity of the fusion antibody is at least about 1000 units/mg.
116. The method of para. 108, wherein the enzyme retains at least 20% of its activity compared to its activity as a separate entity.
117. The method of para. 108, wherein the enzyme and the immunoglobulin each retains at least 20% of its activity compared to its activity as a separate entity.
118. The method of para. 108, wherein the systemic administration is parenteral, intravenous, subcutaneous, intra-muscular, trans-nasal, intra-arterial, transdermal, or respiratory.
119. The method of para. 108, wherein the immunoglobulin heavy chain is an immunoglobulin heavy chain of IgG.
120. The method of para. 108, wherein the immunoglobulin heavy chain is an immunoglobulin heavy chain of IgG1 class.
121. The method of para. 108, wherein the immunoglobulin light chain is an immunoglobulin light chain of kappa or lambda class.
122. The method of para. 108, wherein the fusion protein comprises the amino acid sequence of SEQ ID NO:10, SEQ ID NO:18, SEQ ID NO:20, or SEQ ID NO:22.
123. The method of para. 108, wherein the fusion antibody crosses the BBB by binding an endogenous BBB receptor-mediated transport system.
124. The method of para. 108, wherein the fusion antibody crosses the BBB via an endogenous BBB receptor selected from the group consisting of the insulin receptor, transferrin receptor, leptin receptor, lipoprotein receptor, and the IGF receptor.
125. The method of para. 108, wherein the fusion antibody crosses the BBB by binding an insulin receptor.
126. The method of para. 108, wherein the enzyme deficiency in the central nervous system is mucopolysaccharidosis IIIA (MPS-IIIA), mucopolysaccharidosis IIIB (MPS-IIIB), mucopolysaccharidosis IIIC (MPS-IIIC), or mucopolysaccharidosis IIID (MPS-IIID).
127. A method for treating an enzyme deficiency in the central nervous system of a subject in need thereof, comprising systemically administering to the subject a therapeutically effective dose of a fusion antibody compring (a) a fusion protein comprising the amino acid sequences of an immunoglobulin light chain and an enzyme deficient in mucopolysaccharidosis III (MPS-III), and (b) an immunoglobulin heavy chain; wherein the fusion antibody crosses the blood brain barrier (BBB).
128. The method of para. 127, wherein the enzyme deficient in MPS-III is N-sulfoglucosamine sulfohydrolase (SGSH), alpha-N-acetylgulcosaminidase (NAGLU), heparin-alpha-glucosaminide N-acetyltransferase (HGSNAT), or N-acetylglucosamine-6-sulfatase (GNS).
129. The method of para. 127, wherein the amino acid sequence of the enzyme is covalently linked to the carboxy terminus of the amino acid sequence of the immunoglobulin light chain.
130. The method of para. 127, wherein the fusion antibody catalyzes hydrolysis of sulfate groups from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.
131. The method of para. 127, wherein the fusion antibody is post-translationally modified by a sulfatase modifying factor type 1 (SUMF1).
132. The method of para. 127, wherein the post-translational modification comprises a cysteine to formylglycine conversion.
133. The method of para. 127, wherein the fusion antibody comprises formylglycine.
134. The method of para. 127, wherein at least about 200 ug of SGSH enzyme are delivered to the brain, normalized per 50 kg body weight.
135. The method of para. 127, wherein the therapeutically effective dose comprises at least about least about 1000 units of enzyme activity/Kg body weight.
136. The method of para. 127, wherein the enzyme specific activity of the fusion antibody is about 1000 units/mg.
137. The method of para. 127, wherein the enzyme retains at least 20% of its activity compared to its activity as a separate entity.
138. The method of para. 127, wherein the SGSH and the immunoglobulin each retains at least 20% of its activity compared to its activity as a separate entity.
139. The method of para. 127, wherein the immunoglobulin heavy chain is an immunoglobulin heavy chain of IgG.
140. The method of para. 127, wherein the immunoglobulin heavy chain is an immunoglobulin heavy chain of IgG1 class.
141. The method of para. 127, wherein the immunoglobulin light chain is an immunoglobulin light chain of kappa or lambda class.
142. The method of para. 127, wherein the fusion antibody crosses the BBB by binding an endogenous BBB receptor-mediated transport system.
143. The method of para. 127, wherein the fusion antibody crosses the BBB via an endogenous BBB receptor selected from the group consisting of the insulin receptor, transferrin receptor, leptin receptor, lipoprotein receptor, and the IGF receptor.
144. The method of para. 127, wherein the fusion antibody crosses the BBB the insulin receptor.
145. The method of para. 127, wherein the systemic administration is parenteral, intravenous, subcutaneous, intra-muscular, trans-nasal, intra-arterial, transdermal, or respiratory.
146. The method of para. 127, wherein the enzyme deficiency in the central nervous system is mucopolysaccharidosis IIIA (MPS-IIIA), mucopolysaccharidosis IIIB (MPS-IIIB), mucopolysaccharidosis IIIC (MPS-IIIC), or mucopolysaccharidosis IIID (MPS-IIID).
147. A fusion antibody comprising: (a) a fusion protein comprising the amino acid sequences of an immunoglobulin heavy chain and an enzyme deficient in mucopolysaccharidosis III (MPS-III), and (b) an immunoglobulin light chain; wherein the fusion antibody crosses the blood brain barrier (BBB).
148. The fusion antibody of para. 147, wherein the enzyme deficient in MPS-III is N-sulfoglucosamine sulfohydrolase (SGSH), alpha-N-acetylgulcosaminidase (NAGLU), heparin-alpha-glucosaminide N-acetyltransferase (HGSNAT), or N-acetylglucosamine-6-sulfatase (GNS).
149. The fusion antibody of para. 147, wherein the amino acid sequence of the enzyme is covalently linked to the carboxy terminus of the amino acid sequence of the immunoglobulin heavy chain.
150. The fusion antibody of para. 147, wherein the fusion antibody catalyzes hydrolysis of sulfate groups from heparan sulfate, catalyzes hydrolysis of N-acetyl-D-glucosamine residues in N-acetyl-alpha-D-glucosaminides, catalyzes acetylation of glucosamine residues of heparan sulphate, or catalyzes hydrolysis of the 6-sulfate groups of heparan sulfate.
151. The fusion antibody of para. 147, wherein the fusion antibody is post-translationally modified by a sulfatase modifying factor type 1 (SUMF1).
152. The fusion antibody of para. 147, wherein the post-translational modification comprises a cysteine to formylglycine conversion.
153. The fusion antibody of para. 147, wherein fusion antibody comprises a formylglycine.
154. The fusion antibody of para. 147, wherein the fusion protein further comprises a linker between the amino acid sequence of the enzyme and the carboxy terminus of the amino acid sequence of the immunoglobulin heavy chain.
155. The fusion antibody of para. 147, wherein the enzyme specific activity of the fusion antibody is at least about 1000 units/mg.
156. The fusion antibody of para. 147, wherein the enzyme retains at least 20% of its activity compared to its activity as a separate entity.
157. The fusion antibody of para. 147, wherein the enzyme and the immunoglobulin each retains at least 20% of its activity compared to its activity as a separate entity.
158. The fusion antibody of para. 147, wherein the immunoglobulin heavy chain is an immunoglobulin heavy chain of IgG.
159. The fusion antibody of para. 147, wherein the immunoglobulin heavy chain is an immunoglobulin heavy chain of IgG1 class.
160. The fusion antibody of para. 147, wherein the immunoglobulin light chain is an immunoglobulin light chain of kappa or lambda class.
161. The fusion antibody of para. 147, wherein the fusion protein comprises the amino acid sequence of SEQ ID NO:10, SEQ ID NO:18, SEQ ID NO:20, or SEQ ID NO:22.
162. The fusion antibody of para. 147, wherein the fusion antibody crosses the BBB by binding an endogenous BBB receptor-mediated transport system.
163. The fusion antibody of para. 147, wherein the fusion antibody crosses the BBB via an endogenous BBB receptor selected from the group consisting of the insulin receptor, transferrin receptor, leptin receptor, lipoprotein receptor, and the IGF receptor.
164. The fusion antibody of para. 147, wherein the fusion antibody crosses the BBB by binding an insulin receptor.
165. A pharmaceutical composition comprising a therapeutically effective amount of a fusion antibody of para. 147, and a pharmaceutically acceptable excipient.
166. An isolated polynucleotide encoding the fusion antibody of para. 147.
167. The isolated polynucleotide of para. 166, wherein the isolated polynucleotide comprises the nucleic acid sequence of SEQ ID NO:14, SEQ ID NO:23, SEQ ID NO:24, or SEQ ID NO:25.
168. A vector comprising the isolated polynucleotide of para. 166.
169. The vector of para. 168 comprising the nucleic acid sequence of SEQ ID NO:14, SEQ ID NO:23, SEQ ID NO:24, or SEQ ID NO:25.
170. A host cell comprising the vector of para. 168.
171. The host cell of para. 170, wherein the host cell is a Chinese Hamster Ovary (CHO) cell.

## Claims

1. A fusion antibody comprising:
(a) a fusion protein comprising the amino acid sequences of an immunoglobulin heavy chain from a human transferrin receptor monoclonal antibody and a SGSH, wherein the amino acid sequences of the SGSH is covalently linked to the carboxy terminus of the amino acid sequence of the immunoglobulin heavy chain, and
(b) an immunoglobulin light chain; wherein the fusion antibody crosses the blood brain barrier (BBB) via a human transferrin receotor.

2. The fusion antibody of claim 1, wherein the fusion antibody catalyzes hydrolysis of sulfate groups from heparan sulfate.

3. The fusion antibody of claim 1, wherein the fusion antibody is post-translationally modified by a sulfatase modifying factor type 1 (SUMF1).

4. The fusion antibody of claim 3, wherein the post-translational modification comprises a cysteine to formylglycine conversion.

5. The fusion antibody of claim 1, wherein fusion antibody comprises a formylglycine.

6. The fusion antibody of claim 1, wherein the fusion protein further comprises a linker between the amino acid sequence of the SGSH and the carboxy terminus of the amino acid sequence of the immunoglobulin heavy chain.

7. The fusion antibody of claim 1, wherein the SGSH specific activity of the fusion antibody is at least about 1000 units/mg.

8. The fusion antibody of claim 1, wherein the SGSH retains at least 20% of its activity compared to its activity as a separate entity.

9. The fusion antibody of claim 1, wherein the SGSH and the immunoglobulin each retains at least 20% of its activity compared to its activity as a separate entity.

10. The fusion antibody of claim 1, wherein the amino acid sequences of the SGSH comprises an amino acid sequence at least 90% identical to SEQ ID NO:9.

11. The fusion antibody of claim 1, wherein the immunoglobulin heavy chain is an immunoglobulin heavy chain of IgG, optionally the immunoglobulin heavy chain is an immunoglobulin heavy chain of IgG1 class.

12. The fusion antibody of claim 1, wherein the fusion antibody has SGSH activity for use in a method for treating an N-sulfoglucosamine sulfohydrolase (SGSH) deficiency in the central nervous system of a subject in need thereof.

13. The fusion antibody of claim 12, wherein the method comprises systemically administering to the subject a therapeutically effective dose of the fusion antibody.

14. The fusion antibody for the use according to claim 13, wherein the therapeutically effective does comprises at least about 1000 units/Kg of body weight.

15. The fusion antibody for the use according to claim 12, wherein at least about 200µg of SGSH enzyme are delivered to the brain, normalized per 50 kg body weight.


SEQUENCE LISTING

<110>  PARDRIDGE, WILLIAM M
       BOADO, RUBEN J

<120>  METHODS AND COMPOSITIONS FOR INCREASING ENZYME ACTIVITY IN THE
       CNS

<130>  007686181

<140>  EP14733790.1
<141>  2014-05-19

<150>  US 61/857140
<151>  2013-07-22

<160>  25

<170>  PatentIn version 3.5

<210>  1
<211>  10
<212>  PRT
<213>  Artificial Sequence

<220>
<223>  Synthetic polypeptide

<400>  1

Gly Tyr Thr Phe Thr Asn Tyr Asp Ile His
1               5                   10

<210>  2
<211>  17
<212>  PRT
<213>  Artificial Sequence

<220>
<223>  Synthetic polypeptide

<400>  2

Trp Ile Tyr Pro Gly Asp Gly Ser Thr Lys Tyr Asn Glu Lys Phe Lys
1               5                   10                  15

Gly

<210>  3
<211>  4
<212>  PRT
<213>  Artificial Sequence

<220>
<223>  Synthetic polypeptide

<400>  3

Glu Trp Ala Tyr

1



<210>  4
<211>  11
<212>  PRT
<213>  Artificial Sequence

<220>
<223>  Synthetic polypeptide

<400>  4

Arg Ala Ser Gln Asp Ile Gly Gly Asn Leu Tyr
1               5                   10


<210>  5
<211>  7
<212>  PRT
<213>  Artificial Sequence

<220>
<223>  Synthetic polypeptide

<400>  5

Ala Thr Ser Ser Leu Asp Ser
1               5


<210>  6
<211>  9
<212>  PRT
<213>  Artificial Sequence

<220>
<223>  Synthetic polypeptide

<400>  6

Leu Gln Tyr Ser Ser Ser Pro Trp Thr
1               5


<210>  7
<211>  461
<212>  PRT
<213>  Artificial Sequence

<220>
<223>  Synthetic polypeptide

<400>  7

Met Asp Trp Thr Trp Arg Val Phe Cys Leu Leu Ala Val Ala Pro Gly
1               5                   10                  15


Ala His Ser Gln Val Gln Leu Gln Gln Ser Gly Pro Glu Leu Val Lys
            20                  25                  30

Pro Gly Ala Leu Val Lys Ile Ser Cys Lys Ala Ser Gly Tyr Thr Phe
        35                  40                  45

Thr Asn Tyr Asp Ile His Trp Val Lys Gln Arg Pro Gly Gln Gly Leu
    50                  55                  60

Glu Trp Ile Gly Trp Ile Tyr Pro Gly Asp Gly Ser Thr Lys Tyr Asn
65                  70                  75                  80

Glu Lys Phe Lys Gly Lys Ala Thr Leu Thr Ala Asp Lys Ser Ser Ser
                85                  90                  95

Thr Ala Tyr Met His Leu Ser Ser Leu Thr Ser Glu Lys Ser Ala Val
            100                 105                 110

Tyr Phe Cys Ala Arg Glu Trp Ala Tyr Trp Gly Gln Gly Thr Leu Val
        115                 120                 125

Thr Val Ser Ala Ala Ser Thr Lys Gly Pro Ser Val Phe Pro Leu Ala
    130                 135                 140

Pro Ser Ser Lys Ser Thr Ser Gly Gly Thr Ala Ala Leu Gly Cys Leu
145                 150                 155                 160

Val Lys Asp Tyr Phe Pro Glu Pro Val Thr Val Ser Trp Asn Ser Gly
                165                 170                 175

Ala Leu Thr Ser Gly Val His Thr Phe Pro Ala Val Leu Gln Ser Ser
            180                 185                 190

Gly Leu Tyr Ser Leu Ser Ser Val Val Thr Val Pro Ser Ser Ser Leu
        195                 200                 205

Gly Thr Gln Thr Tyr Ile Cys Asn Val Asn His Lys Pro Ser Asn Thr
    210                 215                 220

Lys Val Asp Lys Lys Val Glu Pro Lys Ser Cys Asp Lys Thr His Thr
225                 230                 235                 240

Cys Pro Pro Cys Pro Ala Pro Glu Leu Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser His Glu Asp Pro Glu Val
        275                 280                 285

Lys Phe Asn Trp Tyr Val Asp Gly Val Glu Val His Asn Ala Lys Thr
    290                 295                 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Asp Glu Leu Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Lys Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro Gly
    450                 455                 460

<210>  8
<211>  234
<212>  PRT
<213>  Artificial Sequence

<220>
<223>  Synthetic polypeptide

<400>  8

Met Glu Thr Pro Ala Gln Leu Leu Phe Leu Leu Leu Leu Trp Leu Pro
1               5                   10                  15

Asp Thr Thr Gly Asp Ile Gln Met Thr Gln Ser Pro Ser Ser Leu Ser
            20                  25                  30

Ala Ser Leu Gly Glu Arg Val Ser Leu Thr Cys Arg Ala Ser Gln Asp
        35                  40                  45

Ile Gly Gly Asn Leu Tyr Trp Leu Gln Gln Gly Pro Asp Gly Thr Ile
    50                  55                  60

Lys Arg Leu Ile Tyr Ala Thr Ser Ser Leu Asp Ser Gly Val Pro Lys
65                  70                  75                  80

Arg Phe Ser Gly Ser Arg Ser Gly Ser Asp Tyr Ser Leu Thr Ile Ser
                85                  90                  95

Ser Leu Glu Ser Glu Asp Phe Val Asp Tyr Tyr Cys Leu Gln Tyr Ser
            100                 105                 110

Ser Ser Pro Trp Thr Phe Gly Gly Gly Thr Lys Met Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  9
<211>  482
<212>  PRT
<213>  Artificial Sequence

<220>
<223>  Synthetic polypeptide

<400>  9

Arg Pro Arg Asn Ala Leu Leu Leu Leu Ala Asp Asp Gly Gly Phe Glu

1                   5                       10                      15

Ser Gly Ala Tyr Asn Asn Ser Ala Ile Ala Thr Pro His Leu Asp Ala
            20                      25                      30

Leu Ala Arg Arg Ser Leu Leu Phe Arg Asn Ala Phe Thr Ser Val Ser
        35                      40                      45

Ser Cys Ser Pro Ser Arg Ala Ser Leu Leu Thr Gly Leu Pro Gln His
    50                      55                      60

Gln Asn Gly Met Tyr Gly Leu His Gln Asp Val His His Phe Asn Ser
65                      70                      75                      80

Phe Asp Lys Val Arg Ser Leu Pro Leu Leu Leu Ser Gln Ala Gly Val
                85                      90                      95

Arg Thr Gly Ile Ile Gly Lys Lys His Val Gly Pro Glu Thr Val Tyr
            100                     105                     110

Pro Phe Asp Phe Ala Tyr Thr Glu Glu Asn Gly Ser Val Leu Gln Val
        115                     120                     125

Gly Arg Asn Ile Thr Arg Ile Lys Leu Leu Val Arg Lys Phe Leu Gln
    130                     135                     140

Thr Gln Asp Asp Arg Pro Phe Phe Leu Tyr Val Ala Phe His Asp Pro
145                     150                     155                     160

His Arg Cys Gly His Ser Gln Pro Gln Tyr Gly Thr Phe Cys Glu Lys
                165                     170                     175

Phe Gly Asn Gly Glu Ser Gly Met Gly Arg Ile Pro Asp Trp Thr Pro
            180                     185                     190

Gln Ala Tyr Asp Pro Leu Asp Val Leu Val Pro Tyr Phe Val Pro Asn
        195                     200                     205

Thr Pro Ala Ala Arg Ala Asp Leu Ala Ala Gln Tyr Thr Thr Val Gly
    210                     215                     220

Arg Met Asp Gln Gly Val Gly Leu Val Leu Gln Glu Leu Arg Asp Ala
225                     230                     235                     240

Gly Val Leu Asn Asp Thr Leu Val Ile Phe Thr Ser Asp Asn Gly Ile
                245                     250                     255

Pro Phe Pro Ser Gly Arg Thr Asn Leu Tyr Trp Pro Gly Thr Ala Glu
            260                 265                 270

Pro Leu Leu Val Ser Ser Pro Glu His Pro Lys Arg Trp Gly Gln Val
        275                 280                 285

Ser Glu Ala Tyr Val Ser Leu Leu Asp Leu Thr Pro Thr Ile Leu Asp
    290                 295                 300

Trp Phe Ser Ile Pro Tyr Pro Ser Tyr Ala Ile Phe Gly Ser Lys Thr
305                 310                 315                 320

Ile His Leu Thr Gly Arg Ser Leu Leu Pro Ala Leu Glu Ala Glu Pro
                325                 330                 335

Leu Trp Ala Thr Val Phe Gly Ser Gln Ser His His Glu Val Thr Met
            340                 345                 350

Ser Tyr Pro Met Arg Ser Val Gln His Arg His Phe Arg Leu Val His
        355                 360                 365

Asn Leu Asn Phe Lys Met Pro Phe Pro Ile Asp Gln Asp Phe Tyr Val
    370                 375                 380

Ser Pro Thr Phe Gln Asp Leu Leu Asn Arg Thr Thr Ala Gly Gln Pro
385                 390                 395                 400

Thr Gly Trp Tyr Lys Asp Leu Arg His Tyr Tyr Tyr Arg Ala Arg Trp
                405                 410                 415

Glu Leu Tyr Asp Arg Ser Arg Asp Pro His Glu Thr Gln Asn Leu Ala
            420                 425                 430

Thr Asp Pro His Phe Ala Gln Leu Leu Glu Met Leu Arg Asp Gln Leu
        435                 440                 445

Ala Lys Trp Gln Trp Glu Thr His Asp Pro Trp Val Cys Ala Pro Asp
    450                 455                 460

Gly Val Leu Glu Glu Lys Leu Ser Pro Gln Cys Gln Pro Leu His Asn
465                 470                 475                 480

Glu Leu

<210>  10
<211>  946
<212>  PRT

<213>  Artificial Sequence

<220>
<223>  Synthetic polypeptide

<400>  10

<210>  11
<211>  23
<212>  DNA
<213>  Artificial Sequence

<220>
<223>  Synthetic oligonucleotide

<400>  11
cacgtccccg gaacgcactg ctg                                               23


<210>  12
<211>  24
<212>  DNA
<213>  Artificial Sequence

<220>
<223>  Synthetic oligonucleotide

<400>  12
tcacagctca ttgtggaggg gctg                                              24


<210>  13
<211>  714
<212>  DNA
<213>  Artificial Sequence

<220>
<223>  Synthetic oligonucleotide

<400>  13
gccgccacca tggagacccc cgcccagctg ctgttcctgt tgctgctttg gcttccagat      60

actaccggcg acatccagat gacccagtct ccatcctcct tatctgcctc tctgggagaa     120

agagtcagtc tcacttgtcg ggcaagtcag gacattggtg gtaacttata ctggcttcag     180

cagggaccag atggaactat taaacgcctg atctacgcca catccagttt agattctggt     240

gtccccaaaa ggttcagtgg cagtaggtct gggtcagatt attctctcac catcagcagc     300

cttgagtctg aagattttgt agactattac tgtctacagt attctagttc tccgtggacg     360

ttcggtggag gcacaaagct ggaaataaaa cgaactgtgg ctgcaccatc tgtcttcatc     420

ttcccgccat ctgatgagca gttgaaatct ggaactgcct ctgttgtgtg cctgctgaat     480

aacttctatc ccagagaggc caaagtacag tggaaggtgg ataacgccct ccaatcgggt     540

aactcccagg agagtgtcac agagcaggac agcaaggaca gcacctacag cctcagcagc     600

accctgacgc tgagcaaagc agactacgag aaacacaaag tctacgcctg cgaagtcacc     660

catcagggcc tgagctcgcc cgtcacaaag agcttcaaca ggggagagtg ttag           714


<210>  14
<211>  2850
<212>  DNA
<213>  Artificial Sequence

<220>
<223>  Synthetic oligonucleotide

<400>  14
gccgccacca tggactggac ctggagggtg ttctgcctgc ttgcagtggc ccccggagcc      60

cacagccagg ttcagctgca gcagtctgga cctgagctgg tgaagcctgg ggctttagtg 120

aagatatcct gcaaggcttc tggttacacc ttcacaaact acgatataca ctgggtgaag 180

cagaggcctg gacagggact tgagtggatt ggatggattt atcctggaga tggtagtact 240

aagtacaatg agaaattcaa gggcaaggcc acactgactg cagacaaatc ctccagcaca 300

gcctacatgc acctcagcag cctgacttct gagaaatctg cagtctattt ctgtgcaaga 360

gagtgggctt actggggcca agggactctg gtcactgtct ctgcagctag caccaagggc 420

ccatcggtct tccccctggc accctcctcc aagagcacct ctgggggcac agcggccctg 480

ggctgcctgg tcaaggacta cttccccgaa ccggtgacgg tgtcgtggaa ctcaggcgcc 540

ctgaccagcg gcgtgcacac cttcccggct gtcctacagt cctcaggact ctactccctc 600

agcagcgtgg tgaccgtgcc ctccagcagc ttgggcaccc agacctacat ctgcaacgtg 660

aatcacaagc ccagcaacac caaggtggac aagaaagttg agcccaaatc ttgtgacaaa 720

actcacacat gcccaccgtg cccagcacct gaactcctgg ggggaccgtc agtcttcctc 780

ttccccccaa aacccaagga caccctcatg atctcccgga cccctgaggt cacatgcgtg 840

gtggtggacg tgagccacga agaccctgag gtcaagttca actggtacgt ggacggcgtg 900

gaggtgcata atgccaagac aaagccgcgg gaggagcagt acaacagcac gtaccgtgtg 960

gtcagcgtcc tcaccgtcct gcaccaggac tggctgaatg gcaaggagta caagtgcaag 1020

gtctccaaca aagccctccc agcccccatc gagaaaacca tctccaaagc caaagggcag 1080

ccccgagaac cacaggtgta caccctgccc ccatcccggg atgagctgac caagaaccag 1140

gtcagcctga cctgcctggt caaaggcttc tatcccagcg acatcgccgt ggagtgggag 1200

agcaatgggc agccggagaa caactacaag accacgcctc ccgtgctgga ctccgacggc 1260

tccttcttcc tctacagcaa gctcaccgtg gacaagagca ggtggcagca ggggaacgtc 1320

ttctcatgct ccgtgatgca tgaggctctg cacaaccact acacgcagaa gagcctctcc 1380

ctgtctcctg gtagtagttc acgtccccgg aacgcactgc tgctcctcgc ggatgacgga 1440

ggctttgaga gtggcgcgta caacaacagc gccatcgcca ccccgcacct ggacgccttg 1500

gcccgccgca gcctcctctt tcgcaatgcc ttcacctcgg tcagcagctg ctctcccagc 1560

cgcgccagcc tcctcactgg cctgccccag catcagaatg ggatgtacgg gctgcaccag 1620

gacgtgcacc acttcaactc cttcgacaag gtgcggagcc tgccgctgct gctcagccaa 1680

gctggtgtgc gcacaggcat catcgggaag aagcacgtgg ggccggagac cgtgtacccg 1740

tttgactttg cgtacacgga ggagaatggc tccgtcctcc aggtggggcg gaacatcact 1800

agaattaagc tgctcgtccg gaaattcctg cagactcagg atgaccggcc tttcttcctc 1860

tacgtcgcct tccacgaccc ccaccgctgt gggcactccc agccccagta cggaaccttc 1920

tgtgagaagt ttggcaacgg agagagcggc atgggtcgta tcccagactg gaccccccag 1980

gcctacgacc cactggacgt gctggtgcct tacttcgtcc ccaacacccc ggcagcccga 2040

gccgacctgg ccgctcagta caccaccgtc ggccgcatgg accaaggagt tggactggtg 2100

ctccaggagc tgcgtgacgc cggtgtcctg aacgacacac tggtgatctt cacgtccgac 2160

aacgggatcc ccttccccag cggcaggacc aacctgtact ggccgggcac tgctgaaccc 2220

ttactggtgt catccccgga gcacccaaaa cgctggggcc aagtcagcga ggcctacgtg 2280

agcctcctag acctcacgcc caccatcttg gattggttct cgatcccgta ccccagctac 2340

gccatctttg gctcgaagac catccacctc actggccggt ccctcctgcc ggcgctggag 2400

gccgagcccc tctgggccac cgtctttggc agccagagcc accacgaggt caccatgtcc 2460

taccccatgc gctccgtgca gcaccggcac ttccgcctcg tgcacaacct caacttcaag 2520

atgccctttc ccatcgacca ggacttctac gtctcaccca ccttccagga cctcctgaac 2580

cgcaccacag ctggtcagcc cacgggctgg tacaaggacc tccgtcatta ctactaccgg 2640

gcgcgctggg agctctacga ccggagccgg gacccccacg agacccagaa cctggccacc 2700

gacccgcact ttgctcagct tctggagatg cttcgggacc agctggccaa gtggcagtgg 2760

gagacccacg acccctgggt gtgcgccccc gacggcgtcc tggaggagaa gctctctccc 2820

cagtgccagc ccctccacaa tgagctgtga 2850

<210> 15
<211> 573
<212> DNA
<213> Artificial Sequence

<220>
<223> Synthetic oligonucleotide

<400> 15
gccgccacca tggttcgacc attgaactgc atcgtcgccg tgtcccaaaa tatggggatt 60

ggcaagaacg gagacctacc ctggcctccg ctcaggaacg agttcaagta cttccaaaga 120

atgaccacaa cctcttcagt ggaaggtaaa cagaatctgg tgattatggg taggaaaacc 180

tggttctcca ttcctgagaa gaatcgacct ttaaaggaca gaattaatat agttctcagt 240

agagaactca aagaaccacc acgaggagct cattttcttg ccaaaagttt ggatgatgcc 300

ttaagactta ttgaacaacc ggaattggca agtaaagtag acatggtttg gatagtcgga 360

ggcagttctg tttaccagga agccatgaat caaccaggcc acctcagact ctttgtgaca 420

aggatcatgc aggaatttga aagtgacacg tttttcccag aaattgattt ggggaaatat 480

aaacttctcc cagaataccc aggcgtcctc tctgaggtcc aggaggaaaa aggcatcaag 540

tataagtttg aagtctacga gaagaaagac taa 573

<210>  16
<211>  187
<212>  PRT
<213>  Artificial Sequence

<220>
<223>  Synthetic polypeptide

<400>  16

Met Val Arg Pro Leu Asn Cys Ile Val Ala Val Ser Gln Asn Met Gly
1               5                   10                  15

Ile Gly Lys Asn Gly Asp Leu Pro Trp Pro Pro Leu Arg Asn Glu Phe
            20                  25                  30

Lys Tyr Phe Gln Arg Met Thr Thr Thr Ser Ser Val Glu Gly Lys Gln
        35                  40                  45

Asn Leu Val Ile Met Gly Arg Lys Thr Trp Phe Ser Ile Pro Glu Lys
    50                  55                  60

Asn Arg Pro Leu Lys Asp Arg Ile Asn Ile Val Leu Ser Arg Glu Leu
65                  70                  75                  80

Lys Glu Pro Pro Arg Gly Ala His Phe Leu Ala Lys Ser Leu Asp Asp
                85                  90                  95

Ala Leu Arg Leu Ile Glu Gln Pro Glu Leu Ala Ser Lys Val Asp Met
            100                 105                 110

Val Trp Ile Val Gly Gly Ser Ser Val Tyr Gln Glu Ala Met Asn Gln
        115                 120                 125

Pro Gly His Leu Arg Leu Phe Val Thr Arg Ile Met Gln Glu Phe Glu
    130                 135                 140

Ser Asp Thr Phe Phe Pro Glu Ile Asp Leu Gly Lys Tyr Lys Leu Leu
145                 150                 155                 160

Pro Glu Tyr Pro Gly Val Leu Ser Glu Val Gln Glu Glu Lys Gly Ile
                165                 170                 175

Lys Tyr Lys Phe Glu Val Tyr Glu Lys Lys Asp
            180                 185


<210>  17
<211>  720
<212>  PRT
<213>  Artificial Sequence

<220>
<223>  Synthetic polypeptide

<400>  17

Asp Glu Ala Arg Glu Ala Ala Ala Val Arg Ala Leu Val Ala Arg Leu
1               5                   10                  15

Leu Gly Pro Gly Pro Ala Ala Asp Phe Ser Val Ser Val Glu Arg Ala
            20                  25                  30

Leu Ala Ala Lys Pro Gly Leu Asp Thr Tyr Ser Leu Gly Gly Gly Gly
        35                  40                  45

Ala Ala Arg Val Arg Val Arg Gly Ser Thr Gly Val Ala Ala Ala Ala
    50                  55                  60

Gly Leu His Arg Tyr Leu Arg Asp Phe Cys Gly Cys His Val Ala Trp
65                  70                  75                  80

Ser Gly Ser Gln Leu Arg Leu Pro Arg Pro Leu Pro Ala Val Pro Gly
                85                  90                  95

Glu Leu Thr Glu Ala Thr Pro Asn Arg Tyr Arg Tyr Tyr Gln Asn Val
            100                 105                 110

Cys Thr Gln Ser Tyr Ser Phe Val Trp Trp Asp Trp Ala Arg Trp Glu
        115                 120                 125

Arg Glu Ile Asp Trp Met Ala Leu Asn Gly Ile Asn Leu Ala Leu Ala
    130                 135                 140

Trp Ser Gly Gln Glu Ala Ile Trp Gln Arg Val Tyr Leu Ala Leu Gly
145                 150                 155                 160

Leu Thr Gln Ala Glu Ile Asn Glu Phe Phe Thr Gly Pro Ala Phe Leu
                165                 170                 175

Ala Trp Gly Arg Met Gly Asn Leu His Thr Trp Asp Gly Pro Leu Pro
            180                 185                 190

Pro Ser Trp His Ile Lys Gln Leu Tyr Leu Gln His Arg Val Leu Asp
        195                 200                 205

Gln Met Arg Ser Phe Gly Met Thr Pro Val Leu Pro Ala Phe Ala Gly
    210                 215                 220

His Val Pro Glu Ala Val Thr Arg Val Phe Pro Gln Val Asn Val Thr
225                 230                 235                 240

Lys Met Gly Ser Trp Gly His Phe Asn Cys Ser Tyr Ser Cys Ser Phe
                245                 250                 255

Leu Leu Ala Pro Glu Asp Pro Ile Phe Pro Ile Ile Gly Ser Leu Phe
            260                 265                 270

Leu Arg Glu Leu Ile Lys Glu Phe Gly Thr Asp His Ile Tyr Gly Ala
        275                 280                 285

Asp Thr Phe Asn Glu Met Gln Pro Pro Ser Ser Glu Pro Ser Tyr Leu
    290                 295                 300

Ala Ala Ala Thr Thr Ala Val Tyr Glu Ala Met Thr Ala Val Asp Thr
305                 310                 315                 320

Glu Ala Val Trp Leu Leu Gln Gly Trp Leu Phe Gln His Gln Pro Gln
                325                 330                 335

Phe Trp Gly Pro Ala Gln Ile Arg Ala Val Leu Gly Ala Val Pro Arg
            340                 345                 350

Gly Arg Leu Leu Val Leu Asp Leu Phe Ala Glu Ser Gln Pro Val Tyr
        355                 360                 365

Thr Arg Thr Ala Ser Phe Gln Gly Gln Pro Phe Ile Trp Cys Met Leu
    370                 375                 380

His Asn Phe Gly Gly Asn His Gly Leu Phe Gly Ala Leu Glu Ala Val
385                 390                 395                 400

Asn Gly Gly Pro Glu Ala Ala Arg Leu Phe Pro Asn Ser Thr Met Val
                405                 410                 415

Gly Thr Gly Met Ala Pro Glu Gly Ile Ser Gln Asn Glu Val Val Tyr
            420                 425                 430

Ser Leu Met Ala Glu Leu Gly Trp Arg Lys Asp Pro Val Pro Asp Leu
        435                 440                 445

Ala Ala Trp Val Thr Ser Phe Ala Ala Arg Arg Tyr Gly Val Ser His
    450                 455                 460

Pro Asp Ala Gly Ala Ala Trp Arg Leu Leu Leu Arg Ser Val Tyr Asn
465                 470                 475                 480

Cys Ser Gly Glu Ala Cys Arg Gly His Asn Arg Ser Pro Leu Val Arg

                485                 490                 495

Arg Pro Ser Leu Gln Met Asn Thr Ser Ile Trp Tyr Asn Arg Ser Asp
            500                 505                 510

Val Phe Glu Ala Trp Arg Leu Leu Leu Thr Ser Ala Pro Ser Leu Ala
        515                 520                 525

Thr Ser Pro Ala Phe Arg Tyr Asp Leu Leu Asp Leu Thr Arg Gln Ala
    530                 535                 540

Val Gln Glu Leu Val Ser Leu Tyr Tyr Glu Glu Ala Arg Ser Ala Tyr
545                 550                 555                 560

Leu Ser Lys Glu Leu Ala Ser Leu Leu Arg Ala Gly Gly Val Leu Ala
                565                 570                 575

Tyr Glu Leu Leu Pro Ala Leu Asp Glu Val Leu Ala Ser Asp Ser Arg
            580                 585                 590

Phe Leu Leu Gly Ser Trp Leu Glu Gln Ala Arg Ala Ala Ala Val Ser
        595                 600                 605

Glu Ala Glu Ala Asp Phe Tyr Glu Gln Asn Ser Arg Tyr Gln Leu Thr
    610                 615                 620

Leu Trp Gly Pro Glu Gly Asn Ile Leu Asp Tyr Ala Asn Lys Gln Leu
625                 630                 635                 640

Ala Gly Leu Val Ala Asn Tyr Tyr Thr Pro Arg Trp Arg Leu Phe Leu
                645                 650                 655

Glu Ala Leu Val Asp Ser Val Ala Gln Gly Ile Pro Phe Gln Gln His
            660                 665                 670

Gln Phe Asp Lys Asn Val Phe Gln Leu Glu Gln Ala Phe Val Leu Ser
        675                 680                 685

Lys Gln Arg Tyr Pro Ser Gln Pro Arg Gly Asp Thr Val Asp Leu Ala
    690                 695                 700

Lys Lys Ile Phe Leu Lys Tyr Tyr Pro Arg Trp Val Ala Gly Ser Trp
705                 710                 715                 720

<210> 18
<211> 1184
<212> PRT
<213> Artificial Sequence

<220>
<223>  Synthetic polypeptide

<400>  18

<210> 19
<211> 616
<212> PRT
<213> Artificial Sequence

<220>
<223> Synthetic polypeptide

<400> 19

Ala Ala Leu Leu Ala Pro Gly Gly Ser Ser Gly Arg Asp Ala Gln Ala
1               5                   10                  15

Ala Pro Pro Arg Asp Leu Asp Lys Lys Arg His Ala Glu Leu Lys Met
            20                  25                  30

Asp Gln Ala Leu Leu Leu Ile His Asn Glu Leu Leu Trp Thr Asn Leu
        35                  40                  45

Thr Val Tyr Trp Lys Ser Glu Cys Cys Tyr His Cys Leu Phe Gln Val
    50                  55                  60

Leu Val Asn Val Pro Gln Ser Pro Lys Ala Gly Lys Pro Ser Ala Ala
65                  70                  75                  80

Ala Ala Ser Val Ser Thr Gln His Gly Ser Ile Leu Gln Leu Asn Asp
                85                  90                  95

Thr Leu Glu Glu Lys Glu Val Cys Arg Leu Glu Tyr Arg Phe Gly Glu
            100                 105                 110

Phe Gly Asn Tyr Ser Leu Leu Val Lys Asn Ile His Asn Gly Val Ser
        115                 120                 125

Glu Ile Ala Cys Asp Leu Ala Val Asn Glu Asp Pro Val Asp Ser Asn
    130                 135                 140

Leu Pro Val Ser Ile Ala Phe Leu Ile Gly Leu Ala Val Ile Ile Val
145                 150                 155                 160

Ile Ser Phe Leu Arg Leu Leu Leu Ser Leu Asp Asp Phe Asn Asn Trp
                165                 170                 175

Ile Ser Lys Ala Ile Ser Ser Arg Glu Thr Asp Arg Leu Ile Asn Ser
            180                 185                 190

Glu Leu Gly Ser Pro Ser Arg Thr Asp Pro Leu Asp Gly Asp Val Gln
        195                 200                 205

Pro Ala Thr Trp Arg Leu Ser Ala Leu Pro Pro Arg Leu Arg Ser Val
    210                 215                 220

Asp Thr Phe Arg Gly Ile Ala Leu Ile Leu Met Val Phe Val Asn Tyr
225                 230                 235                 240

Gly Gly Gly Lys Tyr Trp Tyr Phe Lys His Ala Ser Trp Asn Gly Leu
                245                 250                 255

Thr Val Ala Asp Leu Val Phe Pro Trp Phe Val Phe Ile Met Gly Ser
            260                 265                 270

Ser Ile Phe Leu Ser Met Thr Ser Ile Leu Gln Arg Gly Cys Ser Lys
        275                 280                 285

Phe Arg Leu Leu Gly Lys Ile Ala Trp Arg Ser Phe Leu Leu Ile Cys
    290                 295                 300

Ile Gly Ile Ile Ile Val Asn Pro Asn Tyr Cys Leu Gly Pro Leu Ser
305                 310                 315                 320

Trp Asp Lys Val Arg Ile Pro Gly Val Leu Gln Arg Leu Gly Val Thr
                325                 330                 335

Tyr Phe Val Val Ala Val Leu Glu Leu Leu Phe Ala Lys Pro Val Pro
            340                 345                 350

Glu His Cys Ala Ser Glu Arg Ser Cys Leu Ser Leu Arg Asp Ile Thr
        355                 360                 365

Ser Ser Trp Pro Gln Trp Leu Leu Ile Leu Val Leu Glu Gly Leu Trp
    370                 375                 380

Leu Gly Leu Thr Phe Leu Leu Pro Val Pro Gly Cys Pro Thr Gly Tyr
385                 390                 395                 400

Leu Gly Pro Gly Gly Ile Gly Asp Phe Gly Lys Tyr Pro Asn Cys Thr
                405                 410                 415

Gly Gly Ala Ala Gly Tyr Ile Asp Arg Leu Leu Leu Gly Asp Asp His
            420                 425                 430

Leu Tyr Gln His Pro Ser Ser Ala Val Leu Tyr His Thr Glu Val Ala
        435                 440                 445

Tyr Asp Pro Glu Gly Ile Leu Gly Thr Ile Asn Ser Ile Val Met Ala
    450                 455                 460

Phe Leu Gly Val Gln Ala Gly Lys Ile Leu Leu Tyr Tyr Lys Ala Arg
465                 470                 475                 480

Thr Lys Asp Ile Leu Ile Arg Phe Thr Ala Trp Cys Cys Ile Leu Gly
                485                 490                 495

Leu Ile Ser Val Ala Leu Thr Lys Val Ser Glu Asn Glu Gly Phe Ile

500                 505                 510

Pro Val Asn Lys Asn Leu Trp Ser Leu Ser Tyr Val Thr Thr Leu Ser
        515                 520                 525

Ser Phe Ala Phe Phe Ile Leu Leu Val Leu Tyr Pro Val Val Asp Val
    530                 535                 540

Lys Gly Leu Trp Thr Gly Thr Pro Phe Phe Tyr Pro Gly Met Asn Ser
545                 550                 555                 560

Ile Leu Val Tyr Val Gly His Glu Val Phe Glu Asn Tyr Phe Pro Phe
                565                 570                 575

Gln Trp Lys Leu Lys Asp Asn Gln Ser His Lys Glu His Leu Thr Gln
            580                 585                 590

Asn Ile Val Ala Thr Ala Leu Trp Val Leu Ile Ala Tyr Ile Leu Tyr
        595                 600                 605

Arg Lys Lys Ile Phe Trp Lys Ile
    610                 615

<210>  20
<211>  1080
<212>  PRT
<213>  Artificial Sequence

<220>
<223>  Synthetic polypeptide

<400>  20

<210> 21
<211> 516

<212>   PRT
<213>   Artificial Sequence

<220>
<223>   Synthetic polypeptide

<400>   21

Val Phe Gly Val Ala Ala Gly Thr Arg Arg Pro Asn Val Val Leu Leu
1               5                   10                  15

Leu Thr Asp Asp Gln Asp Glu Val Leu Gly Gly Met Thr Pro Leu Lys
            20                  25                  30

Lys Thr Lys Ala Leu Ile Gly Glu Met Gly Met Thr Phe Ser Ser Ala
        35                  40                  45

Tyr Val Pro Ser Ala Leu Cys Cys Pro Ser Arg Ala Ser Ile Leu Thr
    50                  55                  60

Gly Lys Tyr Pro His Asn His His Val Val Asn Asn Thr Leu Glu Gly
65                  70                  75                  80

Asn Cys Ser Ser Lys Ser Trp Gln Lys Ile Gln Glu Pro Asn Thr Phe
                85                  90                  95

Pro Ala Ile Leu Arg Ser Met Cys Gly Tyr Gln Thr Phe Phe Ala Gly
            100                 105                 110

Lys Tyr Leu Asn Glu Tyr Gly Ala Pro Asp Ala Gly Gly Leu Glu His
        115                 120                 125

Val Pro Leu Gly Trp Ser Tyr Trp Tyr Ala Leu Glu Lys Asn Ser Lys
    130                 135                 140

Tyr Tyr Asn Tyr Thr Leu Ser Ile Asn Gly Lys Ala Arg Lys His Gly
145                 150                 155                 160

Glu Asn Tyr Ser Val Asp Tyr Leu Thr Asp Val Leu Ala Asn Val Ser
                165                 170                 175

Leu Asp Phe Leu Asp Tyr Lys Ser Asn Phe Glu Pro Phe Phe Met Met
            180                 185                 190

Ile Ala Thr Pro Ala Pro His Ser Pro Trp Thr Ala Ala Pro Gln Tyr
        195                 200                 205

Gln Lys Ala Phe Gln Asn Val Phe Ala Pro Arg Asn Lys Asn Phe Asn
    210                 215                 220

Ile His Gly Thr Asn Lys His Trp Leu Ile Arg Gln Ala Lys Thr Pro
225                 230                 235                 240

Met Thr Asn Ser Ser Ile Gln Phe Leu Asp Asn Ala Phe Arg Lys Arg
                245                 250                 255

Trp Gln Thr Leu Leu Ser Val Asp Asp Leu Val Glu Lys Leu Val Lys
            260                 265                 270

Arg Leu Glu Phe Thr Gly Glu Leu Asn Asn Thr Tyr Ile Phe Tyr Thr
        275                 280                 285

Ser Asp Asn Gly Tyr His Thr Gly Gln Phe Ser Leu Pro Ile Asp Lys
    290                 295                 300

Arg Gln Leu Tyr Glu Phe Asp Ile Lys Val Pro Leu Leu Val Arg Gly
305                 310                 315                 320

Pro Gly Ile Lys Pro Asn Gln Thr Ser Lys Met Leu Val Ala Asn Ile
                325                 330                 335

Asp Leu Gly Pro Thr Ile Leu Asp Ile Ala Gly Tyr Asp Leu Asn Lys
            340                 345                 350

Thr Gln Met Asp Gly Met Ser Leu Leu Pro Ile Leu Arg Gly Ala Ser
        355                 360                 365

Asn Leu Thr Trp Arg Ser Asp Val Leu Val Glu Tyr Gln Gly Glu Gly
    370                 375                 380

Arg Asn Val Thr Asp Pro Thr Cys Pro Ser Leu Ser Pro Gly Val Ser
385                 390                 395                 400

Gln Cys Phe Pro Asp Cys Val Cys Glu Asp Ala Tyr Asn Asn Thr Tyr
                405                 410                 415

Ala Cys Val Arg Thr Met Ser Ala Leu Trp Asn Leu Gln Tyr Cys Glu
            420                 425                 430

Phe Asp Asp Gln Glu Val Phe Val Glu Val Tyr Asn Leu Thr Ala Asp
        435                 440                 445

Pro Asp Gln Ile Thr Asn Ile Ala Lys Thr Ile Asp Pro Glu Leu Leu
    450                 455                 460

Gly Lys Met Asn Tyr Arg Leu Met Met Leu Gln Ser Cys Ser Gly Pro
465                 470                 475                 480

Thr Cys Arg Thr Pro Gly Val Phe Asp Pro Gly Tyr Arg Phe Asp Pro
                485                 490                 495

Arg Leu Met Phe Ser Asn Arg Gly Ser Val Arg Thr Arg Arg Phe Ser
            500                 505                 510

Lys His Leu Leu
        515

<210>  22
<211>  980
<212>  PRT
<213>  Artificial Sequence

<220>
<223>  Synthetic polypeptide

<400>  22

<210>  23
<211>  3564
<212>  DNA
<213>  Artificial Sequence

<220>
<223>  Synthetic oligonucleotide

<400>  23
gccgccacca tggactggac ctggagggtg ttctgcctgc ttgcagtggc ccccggagcc      60

cacagccagg ttcagctgca gcagtctgga cctgagctgg tgaagcctgg ggctttagtg     120

aagatatcct gcaaggcttc tggttacacc ttcacaaact acgatataca ctgggtgaag     180

cagaggcctg gacagggact tgagtggatt ggatggattt atcctggaga tggtagtact     240

aagtacaatg agaaattcaa gggcaaggcc acactgactg cagacaaatc ctccagcaca     300

gcctacatgc acctcagcag cctgacttct gagaaatctg cagtctattt ctgtgcaaga     360

gagtgggctt actggggcca agggactctg gtcactgtct ctgcagctag caccaagggc     420

ccatcggtct tccccctggc accctcctcc aagagcacct ctgggggcac agcggccctg     480

ggctgcctgg tcaaggacta cttccccgaa ccggtgacgg tgtcgtggaa ctcaggcgcc     540

ctgaccagcg gcgtgcacac cttcccggct gtcctacagt cctcaggact ctactccctc     600

agcagcgtgg tgaccgtgcc ctccagcagc ttgggcaccc agacctacat ctgcaacgtg     660

aatcacaagc ccagcaacac caaggtggac aagaaagttg agcccaaatc ttgtgacaaa     720

actcacacat gcccaccgtg cccagcacct gaactcctgg ggggaccgtc agtcttcctc     780

ttccccccaa aacccaagga caccctcatg atctcccgga cccctgaggt cacatgcgtg     840

gtggtggacg tgagccacga agaccctgag gtcaagttca actggtacgt ggacggcgtg     900

gaggtgcata atgccaagac aaagccgcgg gaggagcagt acaacagcac gtaccgtgtg     960

gtcagcgtcc tcaccgtcct gcaccaggac tggctgaatg gcaaggagta caagtgcaag    1020

gtctccaaca aagccctccc agcccccatc gagaaaacca tctccaaagc caaagggcag 1080

ccccgagaac cacaggtgta caccctgccc ccatcccggg atgagctgac caagaaccag 1140

gtcagcctga cctgcctggt caaaggcttc tatcccagcg acatcgccgt ggagtgggag 1200

agcaatgggc agccggagaa caactacaag accacgcctc ccgtgctgga ctccgacggc 1260

tccttcttcc tctacagcaa gctcaccgtg gacaagagca ggtggcagca ggggaacgtc 1320

ttctcatgct ccgtgatgca tgaggctctg cacaaccact acacgcagaa gagcctctcc 1380

ctgtctcctg gtagtagttc agacgaggcc cgggaggcgg cggccgtgcg ggcgctcgtg 1440

gcccggctgc tggggccagg ccccgcggcc gacttctccg tgtcggtgga gcgcgctctg 1500

gctgccaagc cgggcttgga cacctacagc ctgggcggcg gcggcgcggc gcgcgtgcgg 1560

gtgcgcggct ccacgggcgt ggcggccgcc gcggggctgc accgctacct gcgcgacttc 1620

tgtggctgcc acgtggcctg gtccggctct cagctgcgcc tgccgcggcc actgccagcc 1680

gtgccggggg agctgaccga ggccacgccc aacaggtacc gctattacca gaatgtgtgc 1740

acgcaaagct actctttcgt gtggtgggac tgggcccgct gggagcgaga gatagactgg 1800

atggcgctga atggcatcaa cctggcactg gcctggagcg gccaggaggc catctggcag 1860

cgggtgtacc tggccttggg cctgacccag gcagagatca atgagttctt tactggtcct 1920

gccttcctgg cctgggggcg aatgggcaac ctgcacacct gggatggccc cctgcccccc 1980

tcctggcaca tcaagcagct ttacctgcag caccgggtcc tggaccagat gcgctccttc 2040

ggcatgaccc cagtgctgcc tgcattcgcg gggcatgttc ccgaggctgt caccagggtg 2100

ttccctcagg tcaatgtcac gaagatgggc agttggggcc actttaactg ttcctactcc 2160

tgctccttcc ttctggctcc ggaagacccc atattcccca tcatcgggag cctcttcctg 2220

cgagagctga tcaaagagtt tggcacagac cacatctatg gggccgacac tttcaatgag 2280

atgcagccac cttcctcaga gccctcctac cttgccgcag ccaccactgc cgtctatgag 2340

gccatgactg cagtggatac tgaggctgtg tggctgctcc aaggctggct cttccagcac 2400

cagccgcagt tctgggggcc cgcccagatc agggctgtgc tgggagctgt gccccgtggc 2460

cgcctcctgg ttctggacct gtttgctgag agccagcctg tgtatacccg cactgcctcc 2520

ttccagggcc agcccttcat ctggtgcatg ctgcacaact ttgggggaaa ccatggtctt 2580

tttggagccc tagaggctgt gaacggaggc ccagaagctg cccgcctctt ccccaactcc 2640

accatggtag gcacgggcat ggcccccgag ggcatcagcc agaacgaagt ggtctattcc 2700

ctcatggctg agctgggctg gcgaaaggac ccagtgccag atttggcagc ctgggtgacc 2760

agctttgccg cccggcggta tggggtctcc cacccggacg caggggcagc gtggaggcta 2820

ctgctccgga gtgtgtacaa ctgctccggg gaggcctgca ggggccacaa tcgtagcccg 2880

ctggtcaggc ggccgtccct acagatgaat accagcatct ggtacaaccg atctgatgtg 2940

tttgaggcct ggcggctgct gctcacatct gctccctccc tggccaccag ccccgccttc 3000

cgctacgacc tgctggacct cactcggcag gcagtgcagg agctggtcag cttgtactat 3060

gaggaggcaa gaagcgccta cctgagcaag gagctggcct ccctgttgag ggctggaggc 3120

gtcctggcct atgagctgct gccggcactg gacgaggtgc tggctagtga cagccgcttc 3180

ttgctgggca gctggctaga gcaggcccga gcagcggcag tcagtgaggc cgaggccgat 3240

ttctacgagc agaacagccg ctaccagctg accttgtggg ggccagaagg caacatcctg 3300

gactatgcca acaagcagct ggcggggttg gtggccaact actacacccc tcgctggcgg 3360

cttttcctgg aggcgctggt tgacagtgtg gcccagggca tccctttcca acagcaccag 3420

tttgacaaaa atgtcttcca actggagcag gccttcgttc tcagcaagca gaggtacccc 3480

agccagccgc gaggagacac tgtggacctg gccaagaaga tcttcctcaa atattacccc 3540

cgctgggtgg ccggctcttg gtga 3564

<210> 24
<211> 3252
<212> DNA
<213> Artificial Sequence

<220>
<223> Synthetic oligonucleotide

<400> 24
gccgccacca tggactggac ctggagggtg ttctgcctgc ttgcagtggc ccccggagcc 60

cacagccagg ttcagctgca gcagtctgga cctgagctgg tgaagcctgg ggctttagtg 120

aagatatcct gcaaggcttc tggttacacc ttcacaaact acgatataca ctgggtgaag 180

cagaggcctg gacagggact tgagtggatt ggatggattt atcctggaga tggtagtact 240

aagtacaatg agaaattcaa gggcaaggcc acactgactg cagacaaatc ctccagcaca 300

gcctacatgc acctcagcag cctgacttct gagaaatctg cagtctattt ctgtgcaaga 360

gagtgggctt actggggcca agggactctg gtcactgtct ctgcagctag caccaagggc 420

ccatcggtct tccccctggc accctcctcc aagagcacct ctgggggcac agcggccctg 480

ggctgcctgg tcaaggacta cttccccgaa ccggtgacgg tgtcgtggaa ctcaggcgcc 540

ctgaccagcg gcgtgcacac cttcccggct gtcctacagt cctcaggact ctactccctc 600

agcagcgtgg tgaccgtgcc ctccagcagc ttgggcaccc agacctacat ctgcaacgtg 660

aatcacaagc ccagcaacac caaggtggac aagaaagttg agcccaaatc ttgtgacaaa 720

actcacacat gcccaccgtg cccagcacct gaactcctgg ggggaccgtc agtcttcctc 780

ttccccccaa aacccaagga caccctcatg atctcccgga cccctgaggt cacatgcgtg 840

gtggtggacg tgagccacga agaccctgag gtcaagttca actggtacgt ggacggcgtg 900

gaggtgcata atgccaagac aaagccgcgg gaggagcagt acaacagcac gtaccgtgtg 960

gtcagcgtcc tcaccgtcct gcaccaggac tggctgaatg gcaaggagta caagtgcaag 1020

gtctccaaca aagccctccc agcccccatc gagaaaacca tctccaaagc caaagggcag 1080

ccccgagaac cacaggtgta caccctgccc ccatcccggg atgagctgac caagaaccag 1140

gtcagcctga cctgcctggt caaaggcttc tatcccagcg acatcgccgt ggagtgggag 1200

agcaatgggc agccggagaa caactacaag accacgcctc ccgtgctgga ctccgacggc 1260

tccttcttcc tctacagcaa gctcaccgtg gacaagagca ggtggcagca ggggaacgtc 1320

ttctcatgct ccgtgatgca tgaggctctg cacaaccact acacgcagaa gagcctctcc 1380

ctgtctcctg gtagtagttc agccgcgctg ctggcccccg gcggctcttc ggggcgcgat 1440

gcccaggccg cgccgccacg agacttagac aaaaaaagac atgcagagct gaagatggat 1500

caggctttgc tactcatcca taatgaactt ctctggacca acttgaccgt ctactggaaa 1560

tctgaatgct gttatcactg cttgtttcag gttctggtaa acgttcctca gagtccaaaa 1620

gcagggaagc ctagtgctgc agctgcctct gtcagcaccc agcacggatc tatcctgcag 1680

ctgaacgaca ccttggaaga gaaagaagtt tgtaggttgg aatacagatt tggagaattt 1740

ggaaactatt ctctcttggt aaagaacatc cataatggag ttagtgaaat tgcctgtgac 1800

ctggctgtga acgaggatcc agttgatagt aaccttcctg tgagcattgc attccttatt 1860

ggtcttgctg tcatcattgt gatatccttt ctgaggctct tgttgagttt ggatgacttt 1920

aacaattgga tttctaaagc cataagttct cgagaaactg atcgcctcat caattctgag 1980

ctgggatctc ccagcaggac agaccctctc gatggtgatg ttcagccagc aacgtggcgt 2040

ctatctgccc tgccgccccg cctccgcagc gtggacacct tcagggggat tgctcttata 2100

ctcatggtct ttgtcaatta tggaggagga aaatattggt acttcaaaca tgcaagttgg 2160

aatgggctga cagtggctga cctcgtgttc ccgtggtttg tatttattat gggatcttcc 2220

atttttctat cgatgacttc tatactgcaa cgggggtgtt caaaattcag attgctgggg 2280

aagattgcat ggaggagttt cctgttaatc tgcataggaa ttatcattgt gaatcccaat 2340

tattgccttg gtccattgtc ttgggacaag gtgcgcattc ctggtgtgct gcagcgattg 2400

ggagtgacat actttgtggt tgctgtgttg gagctcctct ttgctaaacc tgtgcctgaa 2460

cattgtgcct cggagaggag ctgcctttct cttcgagaca tcacgtccag ctggccccag 2520

tggctgctca tcctggtgct ggaaggcctg tggctgggct tgacattcct cctgccagtc 2580

cctgggtgcc ctactggtta tcttggtcct gggggcattg gagattttgg caagtatcca 2640

aattgcactg gaggagctgc aggctacatc gaccgcctgc tgctgggaga cgatcacctt 2700

taccagcacc catcttctgc tgtactttac cacaccgagg tggcctatga ccccgagggc 2760

atcctgggca ccatcaactc catcgtgatg gcctttttag gagttcaggc aggaaaaata 2820

ctattgtatt acaaggctcg gaccaaagac atcctgattc gattcactgc ttggtgttgt 2880

attcttgggc tcatttctgt tgctctgacg aaggtttctg aaaatgaagg ctttattcca 2940

gtaaacaaaa atctctggtc cctttcgtat gtcactacgc tcagttcttt tgccttcttc 3000

atcctgctgg tcctgtaccc agttgtggat gtgaaggggc tgtggacagg aaccccattc 3060

ttttatccag gaatgaattc cattctggta tatgtcggcc acgaggtgtt tgagaactac 3120

ttcccctttc agtggaagct gaaggacaac cagtcccaca aggagcacct gactcagaac 3180

atcgtcgcca ctgccctctg ggtgctcatt gcctacatcc tctatagaaa gaagattttt 3240

tggaaaatct ga 3252

<210> 25
<211> 2952
<212> DNA
<213> Artificial Sequence

<220>
<223> Synthetic oligonucleotide

<400> 25
gccgccacca tggactggac ctggagggtg ttctgcctgc ttgcagtggc ccccggagcc 60

cacagccagg ttcagctgca gcagtctgga cctgagctgg tgaagcctgg ggctttagtg 120

aagatatcct gcaaggcttc tggttacacc ttcacaaact acgatataca ctgggtgaag 180

cagaggcctg gacagggact tgagtggatt ggatggattt atcctggaga tggtagtact 240

aagtacaatg agaaattcaa gggcaaggcc acactgactg cagacaaatc ctccagcaca 300

gcctacatgc acctcagcag cctgacttct gagaaatctg cagtctattt ctgtgcaaga 360

gagtgggctt actggggcca agggactctg gtcactgtct ctgcagctag caccaagggc 420

ccatcggtct tccccctggc accctcctcc aagagcacct ctgggggcac agcggccctg 480

ggctgcctgg tcaaggacta cttccccgaa ccggtgacgg tgtcgtggaa ctcaggcgcc 540

ctgaccagcg gcgtgcacac cttcccggct gtcctacagt cctcaggact ctactccctc 600

agcagcgtgg tgaccgtgcc ctccagcagc ttgggcaccc agacctacat ctgcaacgtg 660

aatcacaagc ccagcaacac caaggtggac aagaaagttg agcccaaatc ttgtgacaaa 720

actcacacat gcccaccgtg cccagcacct gaactcctgg ggggaccgtc agtcttcctc 780

ttccccccaa aacccaagga caccctcatg atctcccgga cccctgaggt cacatgcgtg 840

gtggtggacg tgagccacga agaccctgag gtcaagttca actggtacgt ggacggcgtg 900

gaggtgcata atgccaagac aaagccgcgg gaggagcagt acaacagcac gtaccgtgtg 960

gtcagcgtcc tcaccgtcct gcaccaggac tggctgaatg gcaaggagta caagtgcaag 1020

gtctccaaca aagccctccc agcccccatc gagaaaacca tctccaaagc caaagggcag 1080

ccccgagaac cacaggtgta caccctgccc ccatcccggg atgagctgac caagaaccag 1140

gtcagcctga cctgcctggt caaaggcttc tatcccagcg acatcgccgt ggagtgggag 1200

agcaatgggc agccggagaa caactacaag accacgcctc ccgtgctgga ctccgacggc 1260

tccttcttcc tctacagcaa gctcaccgtg gacaagagca ggtggcagca ggggaacgtc 1320

ttctcatgct ccgtgatgca tgaggctctg cacaaccact acacgcagaa gagcctctcc 1380

ctgtctcctg gtagtagttc agtcttcggg gtggctgcgg gaacccggag gcccaacgtg 1440

gtgctgctcc tcacggacga ccaggacgaa gtgctcggcg gcatgacacc gctaaagaaa 1500

accaaagctc tcatcggaga gatggggatg actttttcca gtgcttatgt gccaagtgct 1560

ctctgctgcc ccagcagagc cagtatcctg acaggaaagt acccacataa tcatcacgtt 1620

gtgaacaaca ctctggaggg gaactgcagt agtaagtcct ggcagaagat ccaagaacca 1680

aatactttcc cagcaattct cagatcaatg tgtggttatc agaccttttt tgcagggaaa 1740

tatttaaatg agtacggagc cccagatgca ggtggactag aacacgttcc tctgggttgg 1800

agttactggt atgccttgga aaagaattct aagtattata attacaccct gtctatcaat 1860

gggaaggcac ggaagcatgg tgaaaactat agtgtggact acctgacaga tgttttggct 1920

aatgtctcct tggactttct ggactacaag tccaactttg agcccttctt catgatgatc 1980

gccactccag cgcctcattc gccttggaca gctgcacctc agtaccagaa ggctttccag 2040

aatgtctttg caccaagaaa caagaacttc aacatccatg gaacgaacaa gcactggtta 2100

attaggcaag ccaagactcc aatgactaat tcttcaatac agtttttaga taatgcattt 2160

aggaaaaggt ggcaaactct cctctcagtt gatgaccttg tggagaaact ggtcaagagg 2220

ctggagttca ctggggagct caacaacact tacatcttct atacctcaga caatggctat 2280

cacacaggac agttttcctt gccaatagac aagagacagc tgtatgagtt tgatatcaaa 2340

gttccactgt tggttcgagg acctgggatc aaaccaaatc agacaagcaa gatgctggtt 2400

gccaacattg acttgggtcc tactattttg gacattgctg gctacgacct aaataagaca 2460

cagatggatg ggatgtcctt attgcccatt ttgagaggtg ccagtaactt gacctggcga 2520

tcagatgtcc tggtggaata ccaaggagaa ggccgtaacg tcactgaccc aacatgccct 2580

tccctgagtc ctggcgtatc tcaatgcttc ccagactgtg tatgtgaaga tgcttataac 2640

aatacctatg cctgtgtgag gacaatgtca gcattgtgga atttgcagta ttgcgagttt 2700

gatgaccagg aggtgtttgt agaagtctat aatctgactg cagacccaga ccagatcact 2760

aacattgcta aaaccataga cccagagctt ttaggaaaga tgaactatcg gttaatgatg 2820

ttacagtcct gttctgggcc aacctgtcgc actccagggg tttttgaccc cggatacagg 2880

tttgaccccc gtctcatgtt cagcaatcgc ggcagtgtca ggactcgaag attttccaaa 2940

catcttctgt ag 2952